(19) Europäisches Patentamt
European Patent Office
Office européen des brevets

(11) EP 3 778 571 A1

(12) **EUROPEAN PATENT APPLICATION**
published in accordance with Art. 153(4) EPC

(43) Date of publication:
**17.02.2021 Bulletin 2021/07**

(21) Application number: **19774270.3**

(22) Date of filing: **26.03.2019**

(51) Int Cl.:
***C07D 211/22*** (2006.01) ***A61K 31/4453*** (2006.01)
***A61K 31/496*** (2006.01) ***A61K 31/5375*** (2006.01)
***C07D 241/04*** (2006.01) ***C07D 265/30*** (2006.01)
***A23L 33/10*** (2016.01) ***A61P 3/04*** (2006.01)
***A61P 3/10*** (2006.01)

(86) International application number:
**PCT/KR2019/003525**

(87) International publication number:
**WO 2019/190177 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.03.2018 KR 20180034825**

(71) Applicant: **Bioway, Inc.**
**Gangnam-gu**
**Seoul 06211 (KR)**

(72) Inventors:
- **KIM, Jong Woo**
  **Gyeonggi-do 16016 (KR)**
- **LEE, Chi Woo**
  **Gyeonggi-do 15885 (KR)**
- **KIM, Hak Sung**
  **Jeonju-si Jeollabuk-do 54955 (KR)**
- **JOSHI, Dirgha Raj**
  **Incheon 21983 (KR)**
- **KWON, Sang Hoon**
  **Seoul 06668 (KR)**

(74) Representative: **D Young & Co LLP**
**120 Holborn**
**London EC1N 2DY (GB)**

(54) **(2E,4E)-5-PHENYL-PENTA-2,4-DIEN-1-ONE DERIVATIVE**

(57) The present application relates to a novel pentadienoyl compound and a pharmaceutical composition including the same. The pentadienoyl compound of the present application may be used to prevent or treat fatty liver and fatty liver-related disease by inhibiting lipogenesis and lipid accumulation in cells and activating lipid metabolism. In addition, the pentadienoyl compound of the present application may increase a SIRT1 expression level in cells or SIRT1 activity, and thus may be used to prevent or treat a SIRT1-mediated disease. In addition, the pentadienoyl compound of the present application may reduce a CK2 expression level in cells or CK2 activity, and thus may be used to prevent or treat a CK2-mediated disease.

Fig. 1

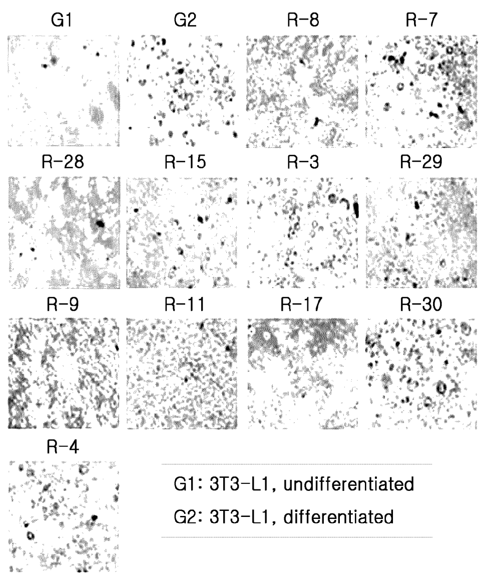


EP 3 778 571 A1

<110> Bioway, Inc.

<120> (2E,4E)-5-Phenyl-penta-2,4-dien-1-one derivatives

<130> OPP19-004-PCT

<150> KR 18/0034825
<151> 2018-03-27

<160> 14

<170> KoPatentIn 3.0

<210> 1
<211> 18
<212> DNA
<213> Artificial Sequence

<220>
<223> sense primer

<400> 1
aaggctgtgg tgctgatg 18

<210> 2
<211> 20
<212> DNA
<213> Artificial Sequence

<220>
<223> sense primer

<400> 2
acgctttcac cagcttcgag 20

<210> 3
<211> 19
<212> DNA
<213> Artificial Sequence

<220>
<223> sense primer

<400> 3
accagagccc cgaaagtct 19

<210> 4
<211> 26
<212> DNA
<213> Artificial Sequence

<220>
<223> sense primer

<400> 4
tgcagaagat gtagattgtg tgatga 26

<210> 5
<211> 20
<212> DNA
<213> Artificial Sequence

<220>
<223> sense primer

<400> 5
gcaaggccat cgactacatt 20

<210> 6
<211> 23
<212> DNA
<213> Artificial Sequence

<220>
<223> sense primer

<400> 6
ttgactgtga agctgtacga gga 23

<210> 7
<211> 21
<212> DNA
<213> Artificial Sequence

<220>
<223> sense primer

<400> 7
tgtcagggat ttcttcagtg c 21

<210> 8
<211> 18
<212> DNA
<213> Artificial Sequence

<220>
<223> anti-sense primer

<400> 8
ggtcctcctt ggtgaaca 18

<210> 9
<211> 20
<212> DNA
<213> Artificial Sequence

<220>
<223> anti-sense primer

<400> 9
gaaagaagcc cttgcagcct 20

<210> 10
<211> 26
<212> DNA
<213> Artificial Sequence

<220>
<223> anti-sense primer

<400> 10
cttgactccc aagatttgaa taactc 26

<210> 11
<211> 20
<212> DNA
<213> Artificial Sequence

<220>
<223> anti-sense primer

<400> 11
gggtccgggt gcagtttatt 20

<210> 12
<211> 20
<212> DNA
<213> Artificial Sequence

<220>
<223> anti-sense primer

<400> 12
ggtcagtgtg tcctccacct 20

<210> 13
<211> 19
<212> DNA
<213> Artificial Sequence

<220>
<223> anti-sense primer

<400> 13
caagcggttc atcagctgg 19

<210> 14
<211> 20
<212> DNA
<213> Artificial Sequence

<220>
<223> anti-sense primer

<400> 14
atcaaggaga gggtggactc 20

## Description

[Technical Field]

The present application relates to a novel pentadienoyl compound and a pharmaceutical composition including the same. More preferably, the present application relates to a pentadienoyl compound or a pharmaceutical composition including the same, which can be used to prevent or treat fatty liver and fatty liver-related disease.

[Background Art]

Obesity increases the risk of metabolic syndrome accompanied by high blood pressure, lipid metabolism abnormality, insulin resistance, etc. In addition, obesity causes chronic diseases such as diabetes, fatty liver and cardiovascular diseases. While there are drugs developed and approved as anti-obesity drugs, they are often accompanied by side effects.

Non-alcoholic fatty liver disease (NAFLD) is closely associated with obesity, type II diabetes, dyslipidemia, and metabolic syndrome. In fact, the prevalence of NAFLD is observed to be relatively high in patients with metabolic syndrome or type II diabetes. At least one of the parameters of metabolic syndrome is found in 90% of NAFLD patients, 1/3 of which are accompanied by metabolic syndrome. Globally, the prevalence of NAFLD is estimated to be 10 to 24%, and increases up to 57 to 74% in individuals with obesity. In Asia, the prevalence of NAFLD is estimated to be 12 to 24%, and the prevalence of individuals with obesity and NAFLD is rapidly increasing in China, Japan, and Korea.

Therapeutic methods for NAFLD include lifestyle improvement and drug treatment. Lifestyle improvement methods include weight loss, diet therapy, and concurrent exercise therapy. For drug treatment, thiazolidinedione and metformin, which are used as therapeutic agents for diabetes, and a therapeutic agent for hyperlipidemia have been used, but development of a drug which is more effective and able to be used safely for a long time is required.

Piperine is a basic plant ingredient, which is found in pepper, chili pepper and the like and has the molecular formula $C_{17}H_{19}NO_3$. As well-known physiological responses to piperine, there are anti-inflammatory, antipyretic, antifungal, antioxidant, antidiarrheal and anticancer effects. Piperine inhibits the physiological activity of mycotoxin, thereby reducing aflatoxin in mouse liver cancer cells and inhibiting micronucleus formation caused by benzo(a)pyrene, which is a carcinogen causing lung cancer in mice. In addition, various functions of piperine such as inhibition of the peroxidation of lipids and regulation of oxidative change for improving the synthesis of glutathione are known.

Recently, it has been revealed that piperine plays an important role in the prevention and treatment of obesity (Korean Patent No. 1,078,376). It has been also revealed that piperine derivatives also have preventive or therapeutic activity for obesity and metabolic diseases such as fatty liver, dyslipidemia, diabetes and insulin resistance syndrome (Korean Patent Nos. 1,186,500 and 1,498,218). The structural formula of LJ-2501 (hereinafter, YU2014), which is one of the piperine derivatives, is shown below (Korean Patent No. 1,498,218).

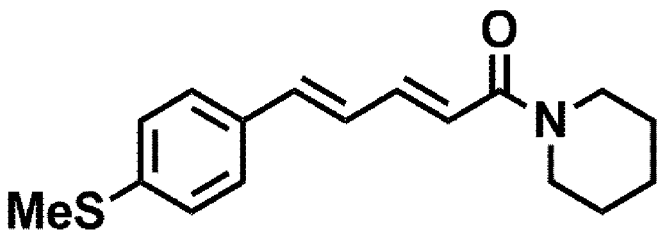

YU2014

[Disclosure]

[Technical Problem]

The present application is directed to providing a pentadienoyl compound or a pharmaceutical composition including the same, which can be used to prevent or treat fatty liver and fatty liver-related disease.

The present application is also directed to providing a pentadienoyl compound, which can be used as a SIRT1-activating compound.

The present application is also directed to providing a pentadienoyl compound or a pharmaceutical composition including the same, which can be used to prevent or treat a SIRT1-mediated disease.

The present application is also directed to providing a pentadienoyl compound, which can be used as a CK2-inhibiting compound.

The present application is also directed to providing a pentadienoyl compound or a pharmaceutical composition including the same, which can be used to prevent or treat a CK2-mediated disease.

[Technical Solution]

To achieve the objects of the present application, a compound represented by Formula 1 or a pharmaceutically acceptable salt thereof is provided.

[Formula 1]

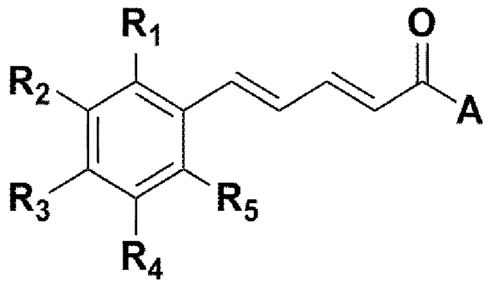

In Formula 1,

$R_1$, $R_2$, $R_3$, $R_4$, and $R_5$ are independently selected from H, $-OR_{11}$, and $-SR_{11}$;
A is selected from

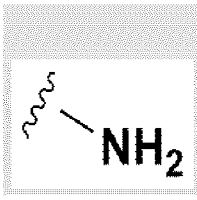, 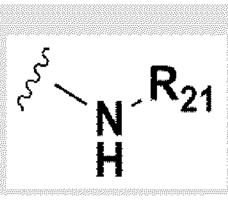, 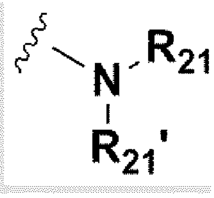

and

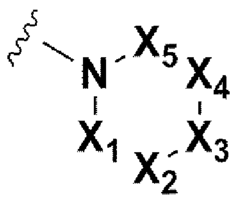;

each $R_{11}$ is independently unsubstituted or substituted $C_1$-$C_6$ alkyl, wherein the alkyl is linear alkyl, branched alkyl, or cycloalkyl;
$R_{21}$ and $R_{21}'$ are independently unsubstituted or substituted $C_1$-$C_6$ alkyl, wherein the alkyl is linear alkyl, branched alkyl, or cycloalkyl;
$X_1$, $X_2$, $X_3$, $X_4$, and $X_5$ are independently selected from $CR_6R_6'$, O, and $NR_7$;
each $R_6$ and $R_6'$ are independently selected from H, a halogen, $R_{31}$, -OH, $-OR_{31}$, -SH, $-SR_{31}$, -CN, $-N_3$, $-NH_2$, $-NHR_{31}$, $-N(R_{31})_2$, -C(=O)H, $-C(=O)R_{31}$, -C(=O)OH, $-C(=O)OR_{31}$, $-C(=O)NH_2$, $-C(=O)NHR_{31}$, $-C(=O)N(R_{31})_2$, -NHC(=O)H, -NHC(=O)OH, and $-NHC(=O)R_{31}$;
$R_7$ is independently selected from H, a halogen, $R_{31}$, -OH, $-OR_{31}$, -SH, $-SR_{31}$, -C(=O)H, $-C(=O)R_{31}$, -C(=O)OH, $-C(=O)OR_{31}$, $-C(=O)NH_2$, $-C(=O)NHR_{31}$, and $-C(=O)N(R_{31})_2$; and
each $R_{31}$ is independently unsubstituted or substituted $C_1$-$C_6$ alkyl, wherein the alkyl is linear alkyl, branched alkyl, or cycloalkyl.

The present application provides a pharmaceutical composition for preventing or treating fatty liver and fatty liver-related disease, which contains the compound represented by Formula 1 or a pharmaceutically acceptable salt thereof as an active ingredient. Here, the fatty liver and fatty liver-related diseases may include fatty liver, obesity, NAFLD, NASH, hepatic fibrosis, cirrhosis, diabetes, dyslipidemia, hyperinsulinemia, insulin resistance, insulin resistance syndrome, and metabolic syndrome.

The present application provides a pharmaceutical composition for preventing or treating a SIRT1-mediated disease, which contains the compound represented by Formula 1 or a pharmaceutically acceptable salt thereof as an active ingredient.

The present application provides a pharmaceutical composition for preventing or treating a CK2-mediated disease, which contains the compound represented by Formula 1 or a pharmaceutically acceptable salt thereof as an active ingredient.

[Advantageous Effects]

The present application provides a pentadienoyl compound or a pharmaceutical composition including the same, which can be used to inhibit lipogenesis in cells.

The present application provides a pentadienoyl compound or a pharmaceutical composition including the same, which can be used to inhibit lipid accumulation in cells.

The present application provides a pentadienoyl compound or a pharmaceutical composition including the same, which can be used to activate the mechanism of fatty acid β oxidation in cells.

The present application provides a pentadienoyl compound or a pharmaceutical composition including the same, which can be used to inhibit the mechanism of fat biosynthesis mechanism in cells.

The present application provides a pentadienoyl compound or a pharmaceutical composition including the same, which can be used to activate the mechanism of regulating lipid metabolism in cells.

The present application provides a pentadienoyl compound or a pharmaceutical composition including the same, which can be used to prevent or treat fatty liver and fatty liver-related disease.

The present application provides a pentadienoyl compound which can be used as a SIRT1-activating compound.

The present application provides a pentadienoyl compound or a pharmaceutical composition including the same, which can be used to prevent or treat a SIRT1-mediated disease.

The present application provides a pentadienoyl compound which can be used as a CK2-inhibiting compound.

The present application provides a pentadienoyl compound or a pharmaceutical composition including the same, which can be used to prevent or treat a CK2-mediated disease.

[Brief Description of Drawings]

FIGS. 1 and 2 show the results showing inhibitory effects of compounds of the present application on lipid droplet formation. After differentiation of 3T3-L1 preadipocytes is induced according to Experimental Example 1, the lipid droplets were identified through Oil-Red O (ORO) staining.

FIG. 3 shows the results of confirming the effects of compounds of the present application on reduction of triglyceride contents in 3T3-L1 cells according to Experimental Example 2.

FIG. 4 shows the results of confirming the effects of compounds of the present application on reduction of glycerol contents in 3T3-L1 cells according to Experimental Example 3.

FIGS. 5 to 9 show the results of confirming the inhibitory effects of compounds of the present application on lipid accumulation in Huh-7 cells according to Experimental Example 4.

FIG. 10 shows the results of confirming the Pparα mRNA level-increasing effects of compounds of the present application in steatosis-induced Huh-7 cells. The effects were confirmed by qRT-PCR according to Experimental Example 5.

FIG. 11 shows the results of confirming the PPARα protein level-increasing effects of the compounds of the present application in steatosis-induced Huh-7 cells. The effects were confirmed by western blotting according to Experimental Example 6.

FIG. 12 shows the results of confirming the Fgf21 mRNA level-increasing effects of compounds of the present application in steatosis-induced Huh-7 cells. The effects were confirmed by qRT-PCR according to Experimental Example 5.

FIG. 13 shows the results of confirming the Fas mRNA level-decreasing effects of compounds of the present application in steatosis-induced Huh-7 cells. The effects were confirmed by qRT-PCR according to Experimental Example 5.

FIG. 14 shows the results of confirming the Srebplc mRNA level-decreasing effects of compounds of the present application in steatosis-induced Huh-7 cells. The effects were confirmed by qRT-PCR according to Experimental Example 5.

FIG. 15 shows the results of confirming the SREBP1 protein level-decreasing effects of compounds of the present application in steatosis-induced Huh-7 cells. The effects were confirmed by western blotting according to Experimental Example 6.

FIG. 16 shows the results of confirming the Sirt1 mRNA level-increasing effects of compounds of the present application in steatosis-induced Huh-7 cells. The effects were confirmed by qRT-PCR according to Experimental Example 5.

FIG. 17 shows the results of confirming the SIRT1 protein level-increasing effects of compounds of the present application in steatosis-induced Huh-7 cells. The effects were confirmed by western blotting according to Experimental Example 6.

FIG. 18 shows the results of confirming the SIRT1 activity-increasing effects of compounds of the present application. The effects were confirmed using a Sirt1 direct fluorescent screening assay kit according to Experimental Example 7.

FIG. 19 shows the results of confirming the Ck2α mRNA level-decreasing effects of compounds of the present application in steatosis-induced Huh-7 cells. The effects were confirmed by qRT-PCR according to Experimental Example 5.

FIG. 20 shows the results of confirming the Ck2α protein level-decreasing effects of compounds of the present application in steatosis-induced Huh-7 cells. The effects were confirmed by western blotting according to Experimental Example 6.

FIG. 21 shows the results of confirming the CK2 activity-decreasing effects of compounds of the present application. The effects were confirmed using a CycLex CK2 kinase assay/inhibitor screening kit according to Experimental Example 8.

[Modes of the Invention]

The term "alkyl" used herein includes linear or branched hydrocarbyl represented by the general formula $C_nH_{2n+1}$ (n is a positive integer). For example, $C_1$ - $C_6$ alkyls may include linear alkyls such as methyl, ethyl, n-propyl, n-butyl, n-pentyl, and n-hexyl. In addition, the $C_1$ - $C_6$ alkyls may include branched alkyls such as isopropyl, sec-butyl, isobutyl, tert-butyl, 1-methylbutyl, 2-methylbutyl, 1-ethylpropyl, 3-methylbutyl, 1,1-dimethylpropyl, 1,2-dimethylpropyl, 2,2-dimethylpropyl, and 2-methylpentyl.

The term "alkyl" used herein includes cyclic hydrocarbyls represented by the general formula $C_nH_{2n-1}$ (n is a positive integer). For example, $C_1$ - $C_6$ alkyls may include cyclopropyl, cyclobutyl, cyclopentyl, and cyclohexyl.

The term "halogen" or "halo" used herein includes fluorine (F), chlorine (Cl), bromine (Br), and iodine (I).

The term "heteroatom" used herein refers to an atom other than carbon or hydrogen. For example, the heteroatoms may include nitrogen (N), oxygen (O), sulfur (S), phosphorus (P), fluorine (F), chlorine (Cl), bromine (Br), and iodine (I).

The "heterocycle" used herein refers to a ring structure in which at least two different elements are included on a ring.

The heterocycle may include at least one carbon atom and at least one nitrogen atom. For example, the heterocycle may include piperidine. For example, the heterocycle may include piperazine.

The heterocycle may include at least one carbon atom, at least one nitrogen atom, and at least one oxygen atom. For example, the heterocycle may include morpholine.

The term "substitution" used herein refers to replacement of a hydrogen atom binding to a specific atom with another atom or functional group.

Sirtuin 1 (hereinafter, SIRT1) is known to regulate the deacetylation and activities of gene-regulatory proteins such as ChREBP, SREBP-1, PPARα, PGC-1α, and NF-κB as well as histone, which is an NAD-dependent deacetylase.

SIRT1 is known to be involved in fatty acid oxidation and hepatic lipid metabolism. Steatosis, which is the initial stage of fatty liver disease, is characterized by accumulating excessive triglycerides in the liver in the form of lipid droplets. One cause of steatosis is the introduction of triglyceride-rich chylomicrons and free fatty acids into the liver through transmembrane proteins. Another cause of steatosis is the synthesis of triglycerides and free fatty acids in the liver by promoting new lipogenesis by high levels of glucose and insulin, activating transcription factors such as ChREBP and SREBP1c, and activating lipogenic enzymes such as FAS, ACC1, SCD1, and ELOVL6. As one of the pathways for eliminating the triglycerides and fatty acids, there is fatty acid β oxidation via a PPARα/PGC-1α signaling pathway in mitochondria, and as another one of the pathways, there is the release of triglycerides in the form of very low-density lipoprotein (VLDL). When SIRT1 is activated, deacetylation of ChREBP and SREBPlc may lead to blocking of the expression of a lipogenic gene and inhibition of new lipogenesis, and deacetylation of PPARα/PGC-1α may lead to an increase in fatty acid β oxidation (Ding RB, Bao J, Deng CX. Int J Biol Sci. (2017) 13(7):852-867).

According to Purushotham A, et al. Cell Metab. (2009) 9(4):327-38, liver cell-specific SIRT1 deletion damages PPARα signaling, and reduces fatty acid β oxidation. In addition, when liver-specific SIRT1 knockout mice are fed a high fat diet, hepatic steatosis and hepatic inflammation were observed.

According to Choi SE, Kwon S, Seok S, et al. Mol Cell Biol. (2017) 37(15):e00006-17, when SIRT1 activity is degraded by CK2-mediated phosphorylation of SIRT1, symptoms of fatty liver and fatty liver-related disease such as a decrease in *in vivo* lipid metabolism, an increase in lipogenesis and an increase in inflammation responses worsen.

In consideration of the study results, a SIRT1-activating compound is expected to be used in prevention or treatment of fatty liver and fatty liver-related disease.

SIRT1-activating compounds are known to have an effect of preventing obesity, NAFLD or diabetes, or improving their symptoms. Among materials known as SIRT1-activating compounds, there is resveratrol. Resveratrol (3,4',5-trihydroxystilbene) is a polyphenol compound with the molecular formula $C_{14}H_{12}O_3$ found in a plant such as grapes. The effect of resveratrol on prevention or treatment of heart diseases, cancer, diabetes, or NAFLD has been being studied.

Casein kinase 2 (hereinafter, CK2) is serine/threonine protein kinase. CK2 is a tetramer consisting of two α catalytic subunits and two β regulatory subunits. The α catalytic subunits may exhibit catalytic activity without β regulatory subunits.

There is the study result of reducing liver triglycerides and improving glucose control in CK2 function-decreased mouse models.

It has been confirmed in mouse models that CK2 is associated with activation of FASN, which is an enzyme playing a pivotal role in a process of promoting lipogenesis by a high level of insulin, which is one of the causes of steatosis (Viscarra JA, Wang Y, Hong IH, Sul HS. Sci Signal. (2017) 10(467):eaai8596). In addition, it is revealed the fact that CK2 knockdown or CK2 inhibition may inhibit hepatic lipogenesis and lower a triglyceride content.

A recent study showed the fact that CK2 lowers SIRT1 activity by S164 phosphorylation of SIRT1 (Choi SE, Kwon S, Seok S, et al. Mol Cell Biol. (2017) 37(15):e00006-17). In the same study, it was confirmed that a high CK2 level is shown in the liver of patients with fatty liver and fatty liver-related disease.

In consideration of the study results, a CK2-inhibiting compound is expected to be used in prevention or treatment of fatty liver and fatty liver-related disease. The CK2-inhibiting compound may include a CK2α inhibitory material. The CK2-inhibiting compound may include a CK2β inhibitory material.

The CK2-inhibiting compounds may be used in prevention or treatment of cancer, blood cancer, malignant lymphoma, etc. For example, the CK2-inhibiting compounds may be used in prevention or treatment of CLL, ALL, CML, AML, MM, etc.

The CK2-inhibiting compounds may be used in prevention or treatment of glioblastoma, Parkinson's disease, Huntington's disease, Lou Gehrig's disease, Alzheimer's disease, etc.

The term "protein activating material" refers to a compound that increases protein expression or increases at least one function of a protein *in vivo* or *in vitro.*

A protein activating material may increase a transcription level or mRNA level of a protein present in cells. For example, a SIRT1-activating compound may increase the Sirt1 mRNA level in cells.

The protein activating material may increase a translation level of a protein present in cells or an amount of the protein. For example, the SIRT1-activating compound may increase the amount of SIRT1 in cells.

The protein activating material may increase at least one function of the protein *in vivo* or *in vitro.* For example, the SIRT1-activating compound may increase the *in vitro* deacetylating activity of SIRT1.

The term "protein inhibitory material" refers to a compound that reduces protein expression, or reduces at least one function of the protein *in vivo* or *in vitro.*

The protein inhibitory material may reduce a transcription level or mRNA level of a protein present in cells. For example, a CK2-inhibiting compound may reduce the Ck2α mRNA level in cells.

The protein inhibitory material may reduce a translation level or amount of a protein present in cells. For example, the CK2-inhibiting compound may reduce the CK2 level in cells.

The protein inhibitory material may reduce at least one function of the protein *in vivo* or *in vitro.* For example, the CK2-inhibiting compound may reduce the *in vitro* phosphorylating activity of CK2.

The "fatty liver and fatty liver-related disease" refer to symptoms of fatty liver and fatty liver, symptoms or diseases which cause fatty liver, or symptoms or diseases occurring according to the progression of fatty liver.

For example, the "fatty liver and related diseases" may include fatty liver, obesity, NAFLD, NASH, hepatic fibrosis, cirrhosis, diabetes, dyslipidemia, hyperinsulinemia, insulin resistance, insulin resistance syndrome, and metabolic syndrome. As another example, the "fatty liver and related diseases" may include steatosis, NASH, and hepatic fibrosis.

The term "obesity" used herein refers to a condition in which adipose tissues are over-accumulated in the body, and when, in diagnosis, a body obesity index (Body Mass Index: a value obtained by dividing a body weight (kg) by the square of height (m)) is 25 or more (30 or more for Westerners), it is defined as obesity.

Fatty acids and glucose introduced from plasma into adipocytes are esterified and mainly accumulated in the

form of triglycerides, and obesity is known to significantly increase the occurrence rate of hyperlipidemia, high blood pressure, arteriosclerosis, diabetes, fatty liver or dysarthrosis.

The term "steatosis" used herein refers to symptoms of excessively accumulating lipids or fats in liver cells or liver tissue. Steatosis is characterized by accumulating excessive triglycerides in the liver in the form of lipid droplets in the initial stage of fatty liver disease.

The term "fatty liver" used herein refers to a disease caused by excessive fat building up in the liver, especially when the weight of fat, particularly, triglycerides is 5% or more on the basis of the liver weight. Generally, fatty liver is classified into alcoholic fatty liver, which is fatty liver caused by alcohol, and nonalcohol fatty liver, which is fatty liver not caused by alcohol, and becomes the cause of various diseases including angina pectoris, myocardial infarction, stroke, arteriosclerosis and pancreatitis. NAFLD is a condition which is not caused by alcohol, and a term including all series of processes including simple fat deposition in the liver, steatohepatitis and cirrhosis. NAFLD is a large part of the causes of chronic liver diseases, except for those occurring due to apparent causes such as a virus, alcohol, etc.

As causes of non-alcoholic fatty liver, there are side effects of antiarrhythmics, antivirals, steroids and cytotoxic drugs, intake of excessive calories such as carbohydrates, obesity, diabetes, and some genetic causes. When fatty liver progresses, it becomes steatohepatitis, and when the non-alcoholic fatty liver is combined with hepatitis B or other health problems, it may develop into cirrhosis or liver cancer.

The term "hepatic fibrosis" used herein refers to a symptom mostly occurring in chronic liver diseases, and excessive accumulation of an extracellular matrix protein such as collagen. When hepatic fibrosis progresses, cirrhosis or liver failure may occur.

The term "cirrhosis" or "hepatocirrhosis" used herein refers to a condition in which fibrotic tissues are increased due to long-term damage of the liver, and thus regenerated nodules in the form of fibers surrounding hepatocytes are distributed in the liver tissue. It has been known that the typical causes of cirrhosis are hepatitis and NAFLD.

The term "diabetes" or "diabetes mellitus" used herein is a type of metabolic disease caused by a lack of insulin secretion or abnormal function of insulin, and refers to a disease characterized by a high blood glucose concentration. Diabetes is classified into type I and type II, and type I is insulin-dependent diabetes, typically caused by destruction of β-cells. Type II diabetes is insulin-independent diabetes, caused by insufficient insulin secretion after a meal or insulin resistance.

Chronic high blood sugar caused by diabetes leads to damage and dysfunction of each organ of the body, and particularly, causes microvascular complications occurring in the retina, kidney and nerves, and macrovascular complications such as atherosclerosis, cardiovascular diseases and cerebrovascular diseases, thereby increasing mortality caused thereby.

The term "dyslipidemia" used herein refers to a condition in which total cholesterol, LDL cholesterol, and triglycerides are increased or HDL cholesterol is decreased in blood, and a disease including hyperlipidemia, hypercholesterolemia and hypertriglyceridemia.

In most cases, dyslipidemia may be caused by obesity, diabetes or alcohol, or may also be caused by an increase in specific lipids in blood due to a genetic factor.

Hyperlipidemia refers to a condition in which lipids including cholesterols and triglycerides in blood are increased. Hyperlipidemia may increase the risk of coronary artery diseases such as arteriosclerosis or myocardial infarction.

Hypercholesterolemia refers to a disease in which total cholesterol and LDL cholesterol are elevated while cholesterol is increased in the blood.

Hypertriglyceridemia refers to a condition in which triglycerides are increased in the blood.

The term "hyperinsulinemia" used herein refers to a condition in which insulin is increased in the blood.

The term "insulin resistance" refers to a phenomenon in which cells do not effectively burn glucose due to degradation in the functions of insulin. When insulin resistance is high, high blood pressure, dyslipidemia, heart disease or diabetes may be caused.

The term "insulin resistance syndrome" is the generic term for diseases caused by insulin resistance. The symptoms of insulin resistance syndrome include hyperinsulinemia, an increase in very low density lipoprotein (VLDL), an increase in triglycerides, a decrease in high density lipoprotein (HDL), and high blood pressure.

The term "metabolic syndrome" or "metabolic disease" refers to a group of diseases associated with the risk of developing various cardiovascular diseases and type II diabetes. The metabolic syndrome includes all of insulin resistance and related metabolic abnormalities and clinical aspects.

The term "prevention" of a disease or illness refers to reduction or elimination of risk factors that can be generated before the occurrence of a disease or illness. In addition, the prevention means the delay of the start of at least one symptom of a disease. The prevention also means the reduction of the frequency of at least one symptom of a disease.

The term "treatment" of a disease or illness refers to alleviating or healing at least one symptom.

The present application provides a pentadienoyl compound represented by Formula 1 below or a pharmaceutically acceptable salt thereof.

[Formula 1]

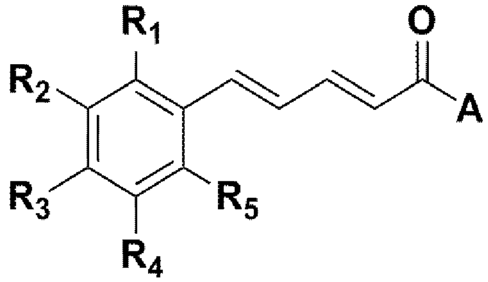

In Formula 1,

$R_1$, $R_2$, $R_3$, $R_4$, and $R_5$ are independently selected from H, $-OR_{11}$, and $-SR_{11}$;
A is selected from

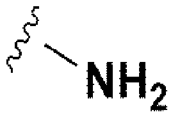,
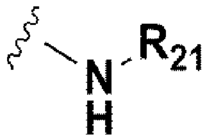,
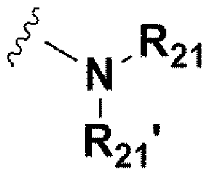

and

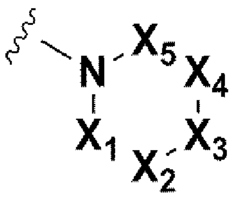;

each $R_{11}$ is independently unsubstituted or substituted $C_1$-$C_6$ alkyl, wherein the alkyl is linear alkyl, branched alkyl, or cycloalkyl;
$R_{21}$ and $R_{21}'$ are independently unsubstituted or substituted $C_1$-$C_6$ alkyl, wherein the alkyl is linear alkyl, branched alkyl, or cycloalkyl;
$X_1$, $X_2$, $X_3$, $X_4$, and $X_5$ are independently selected from $CR_6R_6'$, O, and $NR_7$;
each $R_6$ and $R_6'$ are independently selected from H, a halogen, $R_{31}$, -OH, - $OR_{31}$, -SH, $-SR_{31}$, -CN, $-N_3$, $-NH_2$, $-NHR_{31}$, $-N(R_{31})_2$, -C(=O)H, $-C(=O)R_{31}$, - C(=O)OH, $-C(=O)OR_{31}$, $-C(=O)NH_2$, $-C(=O)NHR_{31}$, $-C(=O)N(R_{31})_2$, -NHC(=O)H, - NHC(=O)OH, and $-NHC(=O)R_{31}$;
$R_7$ is independently selected from H, a halogen, $R_{31}$, -OH, $-OR_{31}$, -SH, $-SR_{31}$, -C(=O)H, $-C(=O)R_{31}$, -C(=O)OH, $-C(=O)OR_{31}$, $-C(=O)NH_2$, $-C(=O)NHR_{31}$, and - $C(=O)N(R_{31})_2$; and
each $R_{31}$ is independently unsubstituted or substituted $C_1$-$C_6$ alkyl, wherein the alkyl is linear alkyl, branched alkyl, or cycloalkyl.

In some exemplary embodiments of the present application, the pentadienoyl compound may include phenyl as a substituent at the fifth position. The phenyl may include at least one substituent. The at least one substituent may include alkoxy and alkylthio. The alkoxy may include methoxy. The alkylthio may include methylthio. The at least one substituent may be located at the third position of phenyl. The at least one substituent may be located at the fourth position of phenyl.

In some exemplary embodiments of the present application, a pentadienoyl compound represented by Formula 1 below or a pharmaceutically acceptable salt thereof is provided.

[Formula 1]

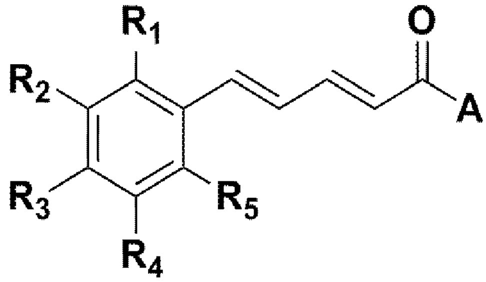

In Formula 1,

three of $R_1$, $R_2$, $R_3$, $R_4$, and $R_5$ are H, and the other two are independently selected from $OR_{11}$ and $-SR_{11}$;
A is selected from

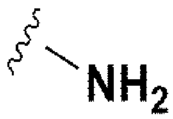 , 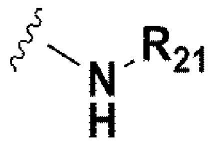 , 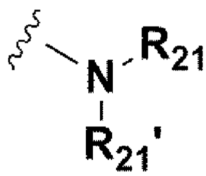

and

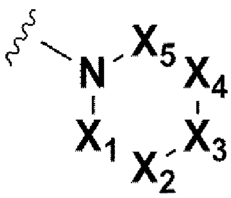 ;

each $R_{11}$ is independently unsubstituted or substituted $C_1$-$C_6$ alkyl, wherein the alkyl is linear alkyl, branched alkyl, or cycloalkyl;
$R_{21}$ and $R_{21}'$ are independently unsubstituted or substituted $C_1$-$C_6$ alkyl, wherein the alkyl is linear alkyl, branched alkyl, or cycloalkyl;
$X_1$, $X_2$, $X_3$, $X_4$, and $X_5$ are independently selected from $CR_6R_6'$, O, and $NR_7$;
each $R_6$ and $R_6'$ are independently selected from H, a halogen, $R_{31}$, -OH, - $OR_{31}$, -SH, $-SR_{31}$, -CN, $-N_3$, $-NH_2$, $-NHR_{31}$, $-N(R_{31})_2$, -C(=O)H, $-C(=O)R_{31}$, - C(=O)OH, $-C(=O)OR_{31}$, $-C(=O)NH_2$, $-C(=O)NHR_{31}$, $-C(=O)N(R_{31})_2$, -NHC(=O)H, - NHC(=O)OH, and $-NHC(=O)R_{31}$;
$R_7$ is independently selected from H, a halogen, $R_{31}$, -OH, $-OR_{31}$, -SH, $-SR_{31}$, -C(=O)H, $-C(=O)R_{31}$, -C(=O)OH, $-C(=O)OR_{31}$, $-C(=O)NH_2$, $-C(=O)NHR_{31}$, and - $C(=O)N(R_{31})_2$; and
each $R_{31}$ is independently unsubstituted or substituted $C_1$-$C_6$ alkyl, wherein the alkyl is linear alkyl, branched alkyl, or cycloalkyl.

In some exemplary embodiments of the present application, a pentadienoyl compound represented by Formula 1 below or a pharmaceutically acceptable salt thereof is provided.

[Formula 1]

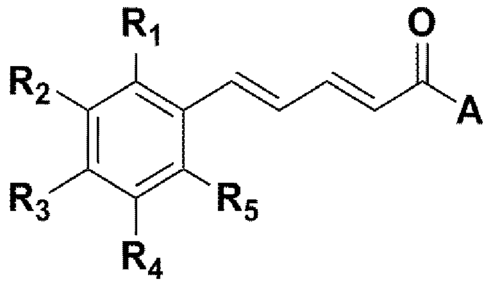

In Formula 1,

$R_1$, $R_4$, and $R_5$ are H;
$R_2$ and $R_3$ are independently selected from $-OR_{11}$ and $-SR_{11}$; and
A, $R_{11}$, $R_{21}$, $R_{21}'$, $X_1$, $X_2$, $X_3$, $X_4$, $X_5$, $R_6$, $R_6'$, $R_7$, and $R_{31}$ are as described above.

In some exemplary embodiments of the present application, a pentadienoyl compound represented by Formula 1 below or a pharmaceutically acceptable salt thereof is provided.

[Formula 1]

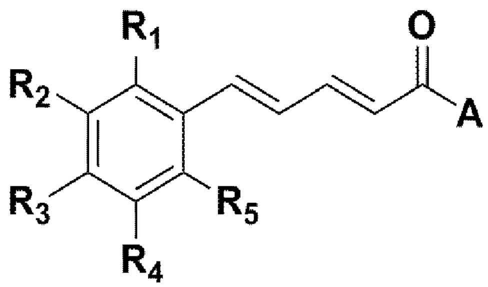

In Formula 1,

four of $R_1$, $R_2$, $R_3$, $R_4$, and $R_5$ are H, and the other is selected from $-OR_{11}$ and $-SR_{11}$; and
A, $R_{11}$, $R_{21}$, $R_{21}'$, $X_1$, $X_2$, $X_3$, $X_4$, $X_5$, $R_6$, $R_6'$, $R_7$, and $R_{31}$ are as described above.

In some exemplary embodiments of the present application, a pentadienoyl compound represented by Formula 1 below or a pharmaceutically acceptable salt thereof is provided.

[Formula 1]

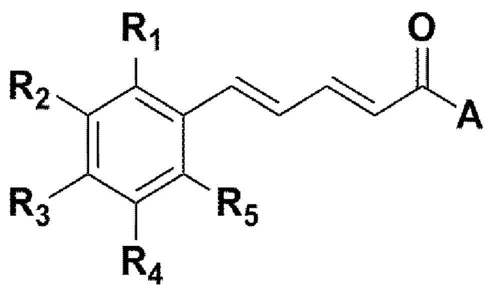

In Formula 1,

$R_1$, $R_3$, $R_4$, and $R_5$ are H;
$R_2$ is selected from $-OR_{11}$ and $-SR_{11}$; and
A, $R_{11}$, $R_{21}$, $R_{21}'$, $X_1$, $X_2$, $X_3$, $X_4$, $X_5$, $R_6$, $R_6'$, $R_7$, and $R_{31}$ are as described above.

In some exemplary embodiments of the present application, a pentadienoyl compound represented by Formula 1 below or a pharmaceutically acceptable salt thereof is provided.

[Formula 1]

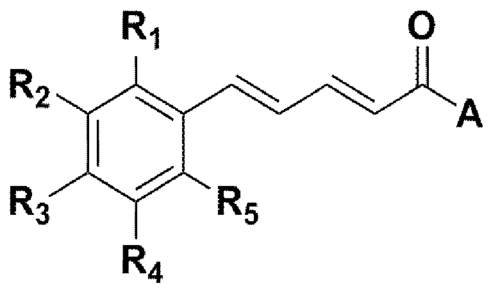

In Formula 1,

$R_1$, $R_2$, $R_4$, and $R_5$ are H;
$R_3$ is selected from $-OR_{11}$ and $-SR_{11}$; and
A, $R_{11}$, $R_{21}$, $R_{21}'$, $X_1$, $X_2$, $X_3$, $X_4$, $X_5$, $R_6$, $R_6'$, $R_7$, and $R_{31}$ are as described above.

In some exemplary embodiments of the present application, a pentadienoyl compound represented by Formula 1 below or a pharmaceutically acceptable salt thereof is provided.

[Formula 1]

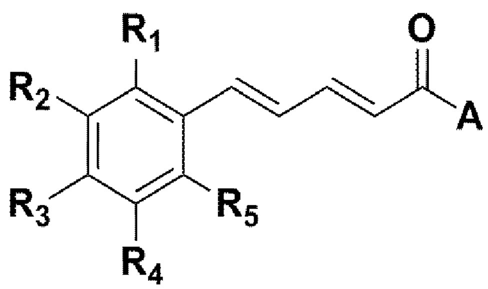

In Formula 1,

$R_1$, $R_2$, $R_3$, $R_4$, and $R_5$ are independently selected from H, $-OR_{11}$, and $-SR_{11}$;
A is

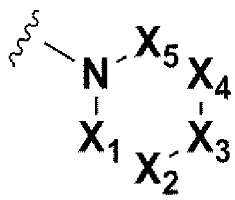

;

each $R_{11}$ is independently unsubstituted or substituted $C_1$-$C_6$ alkyl, wherein the alkyl is linear alkyl, branched alkyl, or cycloalkyl;
$X_1$, $X_2$, $X_3$, $X_4$, and $X_5$ are independently selected from $CR_6R_6'$, O, and $NR_7$;

each $R_6$ and $R_6$' are independently selected from H, a halogen, $R_{31}$, -OH, - $OR_{31}$, -SH, -$SR_{31}$, -CN, -$N_3$, -$NH_2$, -$NHR_{31}$, -$N(R_{31})_2$, -C(=O)H, -C(=O)$R_{31}$, - C(=O)OH, -C(=O)$OR_{31}$, -C(=O)$NH_2$, -C(=O)$NHR_{31}$, -C(=O)$N(R_{31})_2$, -NHC(=O)H, - NHC(=O)OH, and -NHC(=O)$R_{31}$;

$R_7$ is independently selected from H, a halogen, $R_{31}$, -OH, -$OR_{31}$, -SH, -$SR_{31}$, -C(=O)H, -C(=O)$R_{31}$, -C(=O)OH, -C(=O)$OR_{31}$, -C(=O)$NH_2$, -C(=O)$NHR_{31}$, and - C(=O)$N(R_{31})_2$; and

$R_{31}$ is independently unsubstituted or substituted $C_1$-$C_6$ alkyl, wherein the alkyl is linear alkyl, branched alkyl or cycloalkyl.

In some exemplary embodiments of the present application,
in Formula 1,
three of $R_1$, $R_2$, $R_3$, $R_4$, and $R_5$ are H, and the other two are independently selected from -$OR_{11}$ and -$SR_{11}$.

In another exemplary embodiment of the present application,
in Formula 1,

$R_1$, $R_4$, and $R_5$ are H; and
$R_2$ and $R_3$ are independently selected from -$OR_{11}$ and -$SR_{11}$.

In another exemplary embodiment of the present application,
in Formula 1,
four of $R_1$, $R_2$, $R_3$, $R_4$, and $R_5$ are H, and the other is selected from -$OR_{11}$ and -$SR_{11}$.

In another exemplary embodiment of the present application,
in Formula 1,

$R_1$, $R_3$, $R_4$, and $R_5$ are H; and
$R_2$ is selected from -$OR_{11}$ and -$SR_{11}$.

In another exemplary embodiment of the present application,
in Formula 1,

$R_1$, $R_2$, $R_4$, and $R_5$ are H; and
$R_3$ is selected from -$OR_{11}$ and -$SR_{11}$.

In some exemplary embodiments of the present application, a pentadienoyl compound represented by Formula 1a below or a pharmaceutically acceptable salt thereof is provided.

[Formula 1a]

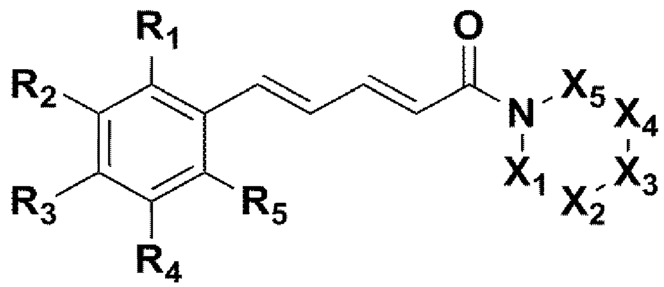

In Formula 1a,

three of $X_1$, $X_2$, $X_3$, $X_4$, and $X_5$ are independently selected from $CH_2$-, CHF, CHCl, CHBr, CHI, CHOH, and $CHNH_2$, and the other two are independently selected from $CR_6R_6$', O, and $NR_7$; and
$R_1$, $R_2$, $R_3$, $R_4$, $R_5$, $R_{11}$, $R_6$, $R_6$', $R_7$, and $R_{31}$ are as described above.

In some exemplary embodiments of the present application,
in Formula 1a,
three of $R_1$, $R_2$, $R_3$, $R_4$, and $R_5$ are H, and the other two are independently selected from -$OR_{11}$ and -$SR_{11}$.

In another exemplary embodiment of the present application,

in Formula 1a,

$R_1$, $R_4$, and $R_5$ are H; and
$R_2$ and $R_3$ are independently selected from $-OR_{11}$ and $-SR_{11}$.

In another exemplary embodiment of the present application,
in Formula 1a,
four of $R_1$, $R_2$, $R_3$, $R_4$, and $R_5$ are H, and the other is selected from $-OR_{11}$ and $-SR_{11}$.

In another exemplary embodiment of the present application,
in Formula 1a,

$R_1$, $R_3$, $R_4$, and $R_5$ are H; and
$R_2$ is selected from $-OR_{11}$ and $-SR_{11}$.

In another exemplary embodiment of the present application,
in Formula 1a,

$R_1$, $R_2$, $R_4$, and $R_5$ are H; and
$R_3$ is selected from $-OR_{11}$ and $-SR_{11}$.

In some exemplary embodiments of the present application, a pentadienoyl compound represented by Formula 1a below or a pharmaceutically acceptable salt thereof is provided.

[Formula 1a]

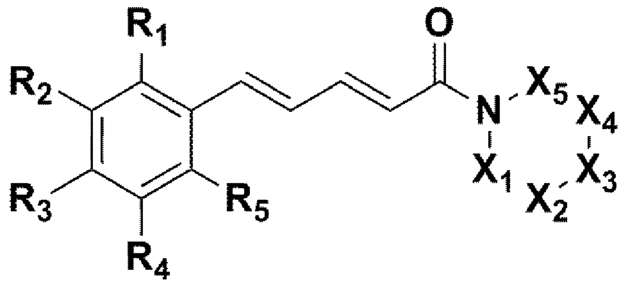

In Formula 1a,

$X_1$, $X_2$, and $X_5$ are independently selected from $CH_2$-, CHF, CHCl, CHBr, CHI, CHOH, and $CHNH_2$;
$X_3$ and $X_4$ are independently selected from $CR_6R_6'$, O, and $NR_7$; and
$R_1$, $R_2$, $R_3$, $R_4$, $R_5$, $R_{11}$, $R_6$, $R_6'$, $R_7$, and $R_{31}$ are as described above.

In some exemplary embodiments of the present application,
in Formula 1a,
three of $R_1$, $R_2$, $R_3$, $R_4$, and $R_5$ are H, and the other two are selected independently from $-OR_{11}$ and $-SR_{11}$.

In another exemplary embodiment of the present application,
in Formula 1a,

$R_1$, $R_4$, and $R_5$ are H; and
$R_2$ and $R_3$ are independently selected from $-OR_{11}$ and $-SR_{11}$.

In another exemplary embodiment of the present application,
in Formula 1a,
four of $R_1$, $R_2$, $R_3$, $R_4$, and $R_5$ are H, and the other is selected from $-OR_{11}$ and $-SR_{11}$.

In another exemplary embodiment of the present application,
in Formula 1a,

$R_1$, $R_3$, $R_4$, and $R_5$ are H; and

$R_2$ is selected from $-OR_{11}$ and $-SR_{11}$.

In another exemplary embodiment of the present application, in Formula 1a,

$R_1$, $R_2$, $R_4$, and $R_5$ are H; and
$R_3$ is selected from $-OR_{11}$ and $-SR_{11}$.

In some exemplary embodiments of the present application, a pentadienoyl compound represented by Formula 1a below or a pharmaceutically acceptable salt thereof is provided.

[Formula 1a]

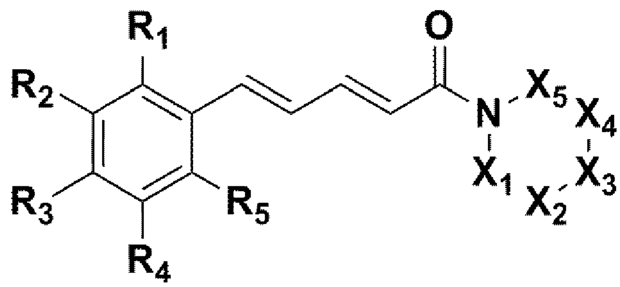

In Formula 1a,

four of $X_1$, $X_2$, $X_3$, $X_4$, and $X_5$ are selected from $CH_2$-, CHF, CHCl, CHBr, CHI, CHOH, and $CHNH_2$, and the other is selected from $CR_6R_6$', O, and $NR_7$; and
$R_1$, $R_2$, $R_3$, $R_4$, $R_5$, $R_{11}$, $R_6$, $R_6$', $R_7$, and $R_{31}$ are as described above.

In some exemplary embodiments of the present application, in Formula 1a, three of $R_1$, $R_2$, $R_3$, $R_4$, and $R_5$ are H, and the other two are independently selected from $-OR_{11}$ and $-SR_{11}$.

In another exemplary embodiment of the present application, in Formula 1a,

$R_1$, $R_4$, and $R_5$ are H; and
$R_2$ and $R_3$ are independently selected from $-OR_{11}$ and $-SR_{11}$.

In another exemplary embodiment of the present application, in Formula 1a, four of $R_1$, $R_2$, $R_3$, $R_4$, and $R_5$ are H, and the other is selected from $-OR_{11}$ and $-SR_{11}$.

In another exemplary embodiment of the present application, in Formula 1a,

$R_1$, $R_3$, $R_4$, and $R_5$ are H; and
$R_2$ is selected from $-OR_{11}$ and $-SR_{11}$.

In another exemplary embodiment of the present application, in Formula 1a,

$R_1$, $R_2$, $R_4$, and $R_5$ are H; and
$R_3$ is selected from $-OR_{11}$ and $-SR_{11}$.

In some exemplary embodiments of the present application, a pentadienoyl compound represented by Formula 1a below or a pharmaceutically acceptable salt thereof is provided.

[Formula 1a]

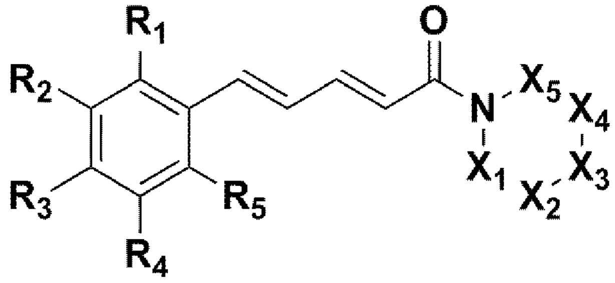

In Formula 1a,

$X_1$, $X_2$, $X_3$, and $X_5$ are selected from $CH_2$-, CHF, CHCl, CHBr, CHI, CHOH, and $CHNH_2$;
$X_4$ is selected from $CR_6R_6$', O, and $NR_7$; and
$R_1$, $R_2$, $R_3$, $R_4$, $R_5$, $R_{11}$, $R_6$, $R_6$', $R_7$, and $R_{31}$ are as described above.

In some exemplary embodiments of the present application,
in Formula 1a,
three of $R_1$, $R_2$, $R_3$, $R_4$, and $R_5$ are H, and the other two are independently selected from $-OR_{11}$ and $-SR_{11}$.

In another exemplary embodiment of the present application,
in Formula 1a,

$R_1$, $R_4$, and $R_5$ are H; and
$R_2$ and $R_3$ are independently selected from $-OR_{11}$ and $-SR_{11}$.

In another exemplary embodiment of the present application,
in Formula 1a,
four of $R_1$, $R_2$, $R_3$, $R_4$, and $R_5$ are H, and the other is selected from $-OR_{11}$ and $-SR_{11}$.

In another exemplary embodiment of the present application,
in Formula 1a,

$R_1$, $R_3$, $R_4$, and $R_5$ are H; and
$R_2$ is selected from $-OR_{11}$ and $-SR_{11}$.

In another exemplary embodiment of the present application,
in Formula 1a,

$R_1$, $R_2$, $R_4$, and $R_5$ are H; and
$R_3$ is selected from $-OR_{11}$ and $-SR_{11}$.

In some exemplary embodiments of the present application, a pentadienoyl compound represented by Formula 1a below or a pharmaceutically acceptable salt thereof is provided.

[Formula 1a]

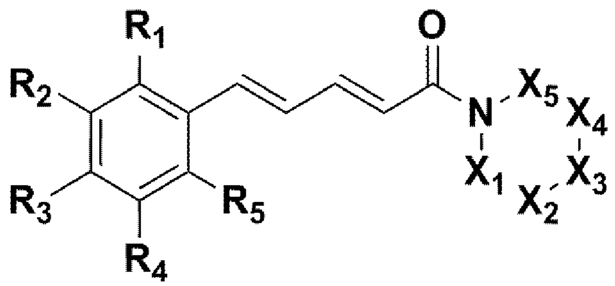

in Formula 1a,

$X_1$, $X_2$, $X_4$, and $X_5$ are selected from $CH_2$-, CHF, CHCl, CHBr, CHI, CHOH, and $CHNH_2$;
$X_3$ is selected from $CR_6R_6$', O, and $NR_7$; and
$R_1$, $R_2$, $R_3$, $R_4$, $R_5$, $R_{11}$, $R_6$, $R_6$', $R_7$, and $R_{31}$ are as described above.

In some exemplary embodiments of the present application,
in Formula 1a,
three of $R_1$, $R_2$, $R_3$, $R_4$, and $R_5$ are H, and the other two is independently selected from -$OR_{11}$ and -$SR_{11}$.

In another exemplary embodiment of the present application,
in Formula 1a,

$R_1$, $R_4$, and $R_5$ are H; and
$R_2$ and $R_3$ is independently selected from -$OR_{11}$ and -$SR_{11}$.

In another exemplary embodiment of the present application,
in Formula 1a,
four of $R_1$, $R_2$, $R_3$, $R_4$, and $R_5$ are H, and the other is selected from -$OR_{11}$ and -$SR_{11}$.

In another exemplary embodiment of the present application,
in Formula 1a,

$R_1$, $R_3$, $R_4$, and $R_5$ are H; and
$R_2$ is selected from -$OR_{11}$ and -$SR_{11}$.

In another exemplary embodiment of the present application,
in Formula 1a,

$R_1$, $R_2$, $R_4$, and $R_5$ are H; and
$R_3$ is selected from -$OR_{11}$ and -$SR_{11}$.

In some exemplary embodiments of the present application, a pentadienoyl compound may include a ring structure as a substituent at the first position. The ring structure may include a heterocycle. The heterocycle may include at least one nitrogen atom (N). The heterocycle may include a 6-membered ring. The heterocycle may include piperidinyl, piperazinyl, and morpholinyl. The heterocycle may include at least one substituent. The pentadienoyl compound in which the ring structure is located at the first position may include a compound represented by Formula 2 below.

In some exemplary embodiments of the present application, a pentadienoyl compound represented by Formula 2 below or a pharmaceutically acceptable salt thereof is provided.

[Formula 2]

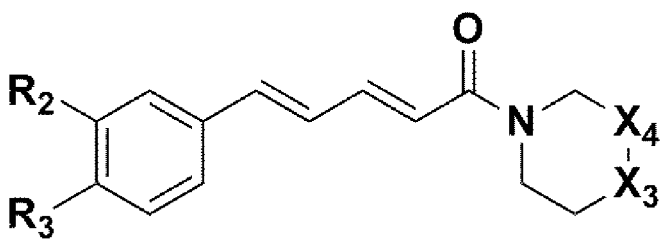

In Formula 2,

$R_2$ and $R_3$ are independently selected from H, -$OR_{11}$, and -$SR_{11}$;
each $R_{11}$ is independently unsubstituted or substituted $C_1$-$C_6$ alkyl, wherein the alkyl is linear alkyl, branched alkyl, or cycloalkyl;
$X_3$ and $X_4$ are independently selected from $CR_6R_6$', O, and $NR_7$;
$R_6$ and $R_6$' are independently selected from H, a halogen, $R_{31}$, -OH, -$OR_{31}$, - SH, -$SR_{31}$, -CN, -$N_3$, -$NH_2$, -$NHR_{31}$,

-$N(R_{31})_2$, -C(=O)H, -C(=O)$R_{31}$, -C(=O)OH, - C(=O)O$R_{31}$, -C(=O)$NH_2$, -C(=O)NH$R_{31}$, -C(=O)$N(R_{31})_2$, -NHC(=O)H, - NHC(=O)OH, and -NHC(=O)$R_{31}$;

$R_7$ is selected from H, a halogen, $R_{31}$, -OH, -O$R_{31}$, -SH, -S$R_{31}$, -C(=O)H, - C(=O)$R_3$, -C(=O)OH, -C(=O)O$R_3$, -C(=O)$NH_2$, -C(=O)NH$R_3$ , and -C(=O)$N(R_{31})_2$; and

each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, the pentadienoyl compound may include piperidinyl as a substituent at the first position. The piperidinyl may include poiperidine-1-yl. The piperidinyl may include at least one substituent. The at least one substituent may include at least one functional group. The at least one substituent may include a functional group capable of regulating hydrophilicity of the piperidinyl and the pentadienoyl compound. The functional group may include at least one of halo, alkyl, hydroxyl, alkoxy, sulfhydryl (thiol), alkylthio, cyanide, azide, amine, aldehyde, acyl, carboxylic acid, ester, and amide. The functional group may include at least one of alkyl, hydroxyl, alkoxy, azide, amine, acyl, ester and amide. The functional group may include at least one of hydroxyl, alkoxy, azide and amine. The at least one substituent may be located at the third position of the piperidinyl. The at least one substituent may be located at the fourth position of the piperidinyl. The pentadienoyl compound in which the piperidinyl including at least one substituent is located at the first position may include a compound represented by any one of Formulas 2 to 10.

In some exemplary embodiments of the present application, a pentadienoyl compound represented by Formula 2 below or a pharmaceutically acceptable salt thereof is provided.

[Formula 2]

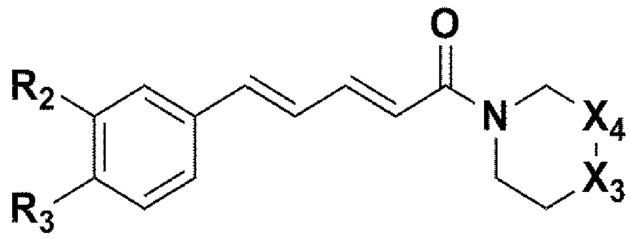

in Formula 2,

$R_2$ and $R_3$ are independently selected from H, -O$R_{11}$, and -S$R_{11}$;

each $R_{11}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl);

$X_3$ and $X_4$ are $CR_6R_6$';

both $X_3$ and $X_4$ are not $CH_2$ at once;

each $R_6$ and $R_6$' are independently selected from H, a halogen, $R_{31}$, -OH, - O$R_{31}$, -SH, -S$R_{31}$, -CN, -$N_3$, -$NH_2$, -NH$R_{31}$, -$N(R_{31})_2$, -C(=O)H, -C(=O)$R_{31}$, - C(=O)OH, -C(=O)O$R_{31}$, -C(=O)$NH_2$, -C(=O)NH$R_{31}$, -C(=O)$N(R_{31})_2$, -NHC(=O)H, - NHC(=O)OH, and -NHC(=O)$R_{31}$ (or $R_6$ is selected from -OH, -O$R_{31}$, -$NH_2$, and -$N_3$, and $R_6$' is H); and

each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 3 below or a pharmaceutically acceptable salt thereof is provided.

[Formula 3]

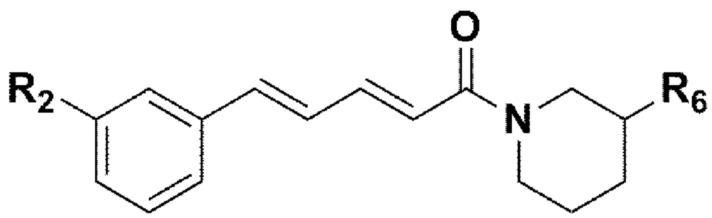

In Formula 3,

$R_2$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$R_6$ is selected from halo, $R_{31}$, -OH, $-OR_{31}$, -SH, $-SR_{31}$, -CN, $-N_3$, $-NH_2$, - $NHR_{31}$, $-N(R_{31})_2$, -C(=O)H, $-C(=O)R_{31}$, -C(=O)OH, $-C(=O)OR_{31}$, $-C(=O)NH_2$, - $C(=O)NHR_{31}$, $-C(=O)N(R_{31})_2$, -NHC(=O)H, -NHC(=O)OH, and $-NHC(=O)R_{31}$ (or, $R_6$ is selected from -OH, $-OR_{31}$, $-NH_2$, and $-N_3$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 4 below or a pharmaceutically acceptable salt thereof is provided.

[Formula 4]

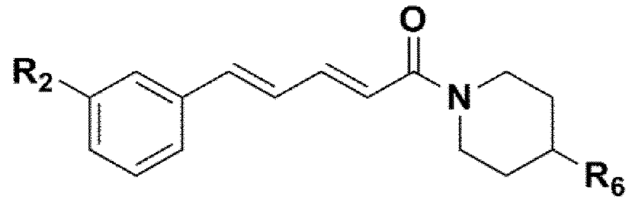

In Formula 4,

$R_2$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$R_6$ is selected from halo, $R_{31}$, -OH, $-OR_{31}$, -SH, $-SR_{31}$, -CN, $-N_3$, $-NH_2$, - $NHR_{31}$, $-N(R_{31})_2$, -C(=O)H, $-C(=O)R_{31}$, -C(=O)OH, $-C(=O)OR_{31}$, $-C(=O)NH_2$, - $C(=O)NHR_{31}$, $-C(=O)N(R_{31})_2$, -NHC(=O)H, -NHC(=O)OH, and $-NHC(=O)R_{31}$ (or, $R_6$ is selected from -OH, $-OR_{31}$, $-NH_2$, and $-N_3$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application a pentadienoyl compound represented by Formula 5 below or a pharmaceutically acceptable salt thereof is provided.

[Formula 5]

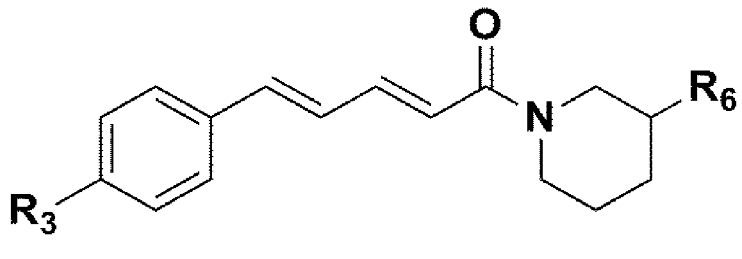

In some exemplary embodiments of the present application,
in Formula 5,

$R_3$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$R_6$ is selected from halo, $R_{31}$, -OH, $-OR_{31}$, -SH, $-SR_{31}$, -CN, $-N_3$, $-NH_2$, - $NHR_{31}$, $-N(R_{31})_2$, -C(=O)H, $-C(=O)R_{31}$, -C(=O)OH, $-C(=O)OR_{31}$, $-C(=O)NH_2$, - $C(=O)NHR_{31}$, $-C(=O)N(R_{31})_2$, -NHC(=O)H, -NHC(=O)OH, and $-NHC(=O)R_{31}$ (or, $R_6$ is selected from -OH, $-OR_{31}$, $-NH_2$, and $-N_3$); and

each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 6 below or a pharmaceutically acceptable salt thereof is provided.

[Formula 6]

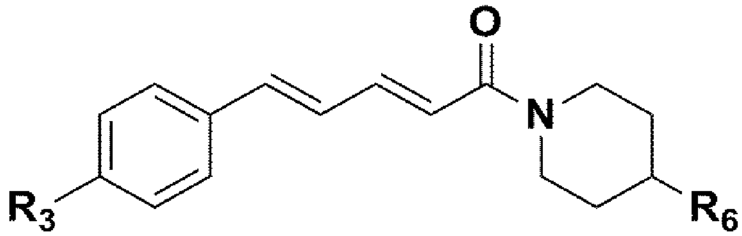

In Formula 6,

$R_3$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$X_3$ is $NR_7$;
$R_6$ is selected from halo, $R_{31}$, -OH, $-OR_{31}$, -SH, $-SR_{31}$, -CN, $-N_3$, $-NH_2$, - $NHR_{31}$, $-N(R_{31})_2$, -C(=O)H, $-C(=O)R_{31}$, -C(=O)OH, $-C(=O)OR_{31}$, $-C(=O)NH_2$, - $C(=O)NHR_{31}$, $-C(=O)N(R_{31})_2$, -NHC(=O)H, -NHC(=O)OH, and $-NHC(=O)R_{31}$ (or, $R_6$ is selected from -OH, $-OR_{31}$, $-NH_2$, and $-N_3$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 3 or 4 below or a pharmaceutically acceptable salt thereof is provided.

[Formula 3]

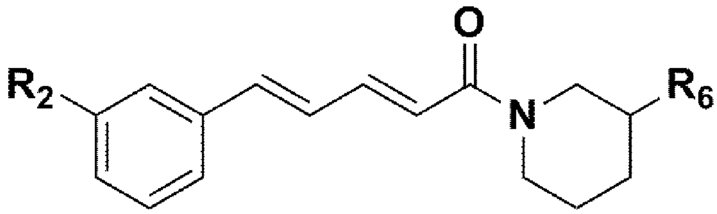

[Formula 4]

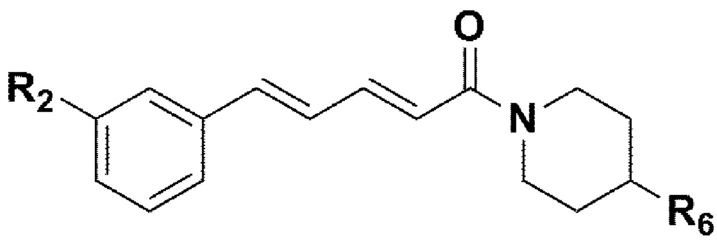

In Formula 3 or 4,

$R_2$ is selected from $-OCH_3$ and $-SCH_3$;
$R_6$ is selected from -OH, $-OR_{31}$, $-NH_2$, and $-N_3$; and

$R_{31}$ is selected from methyl, ethyl, n-propyl, isopropyl, n-butyl, sec-butyl, isobutyl, and tert-butyl.

In some exemplary embodiments of the present application, a pentadienoyl compound represented by Formula 5 or 6 below or a pharmaceutically acceptable salt thereof is provided.

[Formula 5]

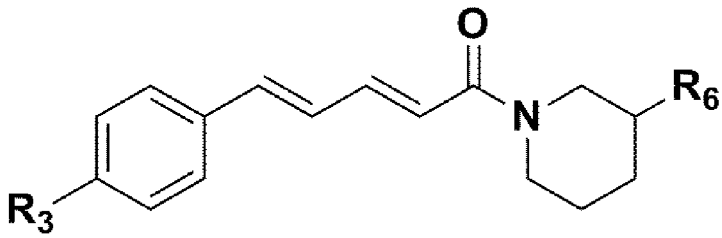

[Formula 6]

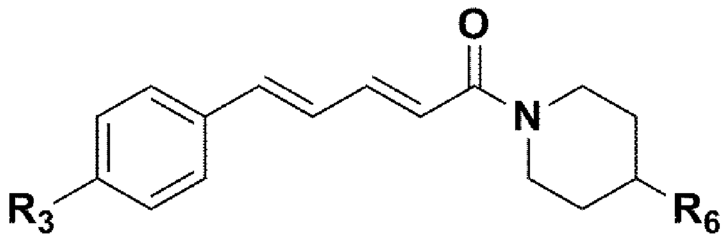

In Formula 5 or 6,

$R_3$ is selected from $-OCH_3$ and $-SCH_3$;
$R_6$ is selected from -OH, $-OR_{31}$, $-NH_2$, and $-N_3$; and
$R_{31}$ is selected from methyl, ethyl, n-propyl, isopropyl, n-butyl, sec-butyl, isobutyl, and tert-butyl.

In some exemplary embodiments of the present application, the pentadienoyl compound may include piperazinyl as a substituent at the first position. The piperazinyl may include piperazine-1-yl. The piperazinyl may include at least one substituent. The at least one substituent may include at least one functional group. The at least one substituent may include a functional group capable of regulating hydrophilicity of the piperazinyl and the pentadienoyl compound. The functional group may include halo, alkyl, hydroxyl, alkoxy, sulfhydryl (thiol), alkylthio, aldehyde, acyl, carboxylic acid, ester, and amide. The functional group may include at least one of acyl, ester, and amide. The functional group may include at least one of acyl and ester. The at least one substituent may be located at the fourth position of the piperazinyl. As the substituent at the first position of the pentadienoyl compound, piperazine-1-yl shown below may be included, but the present application is not limited thereto.

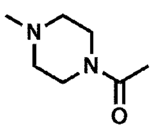 , 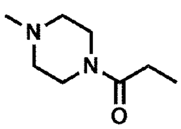 , 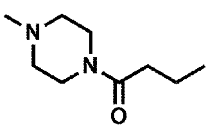 , 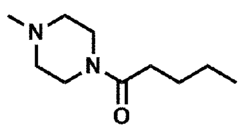 ,

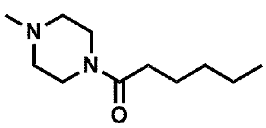 , 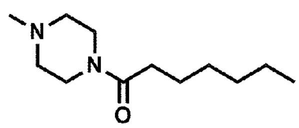 , 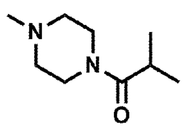 , 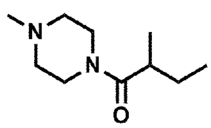 ,

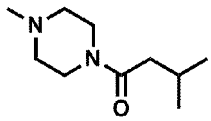, 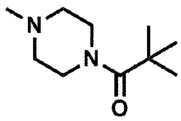, 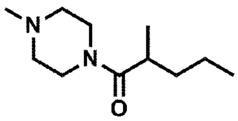, 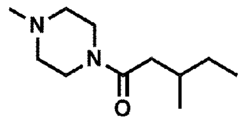,

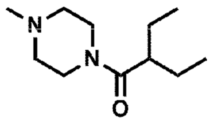, 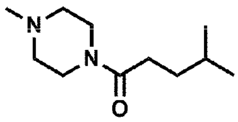, 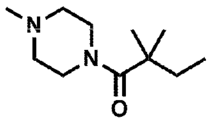, 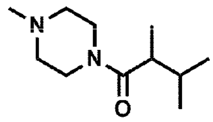,

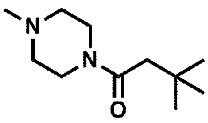, 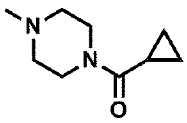, 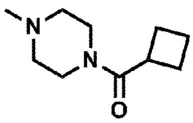, 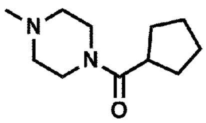, 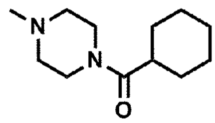,

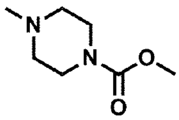, 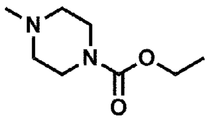, 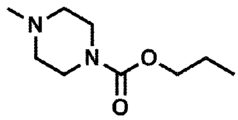, 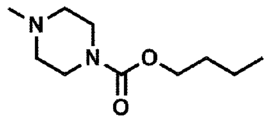,

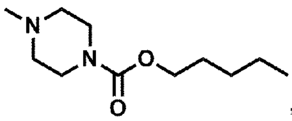, 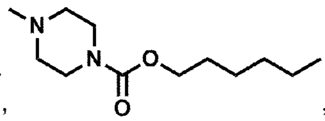, 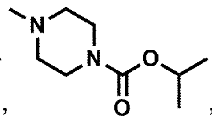, 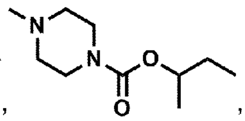,

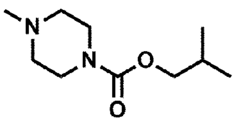, 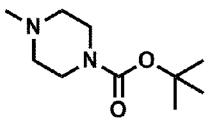, 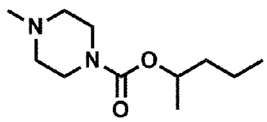, 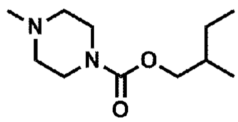,

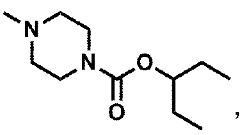, 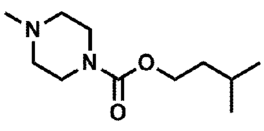, 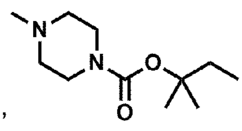, 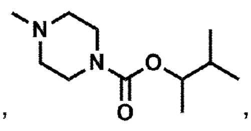,

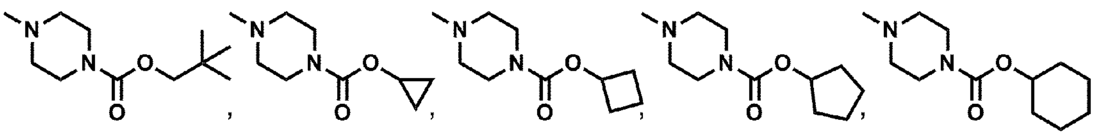

The pentadienoyl compound may include a compound represented by any one of Formulas 2 and 7 to 14 below. The pentadienoyl compound in which piperazinyl with at least one substituent is located at the first position may include a compound represented by any one of Formulas 2, 8, and 10 to 14 below.

In some exemplary embodiments of the present application, a pentadienoyl compound represented by Formula 2 below or a pharmaceutically acceptable salt thereof is provided.

[Formula 2]

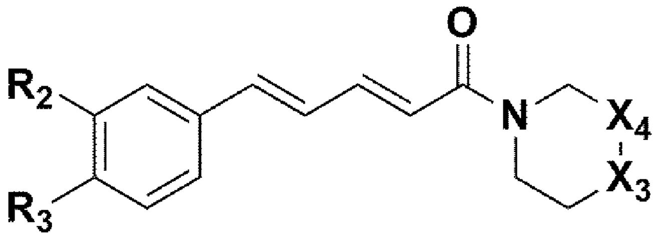

In Formula 2,

$R_2$ and $R_3$ are independently selected from H, $-OR_{11}$, and $-SR_{11}$;
each $R_{11}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl);
$X_3$ and $X_4$ are independently selected from $CH_2$ and $NR_7$;
both $X_3$ and $X_4$ are not $CH_2$ at once;
$R_7$ is selected from H, a halogen, $R_{31}$, -OH, $-OR_{31}$, -SH, $-SR_{31}$, -C(=O)H, - C(=O)$R_{31}$, -C(=O)OH, -C(=O)$OR_{31}$, -C(=O)$NH_2$, -C(=O)$NHR_{31}$, and -C(=O)N$(R_{31})_2$ (or, $R_7$ is selected from -C(=O)$OR_3$ and -C(=O)$R_{31}$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 7 below or a pharmaceutically acceptable salt thereof is provided.

[Formula 7]

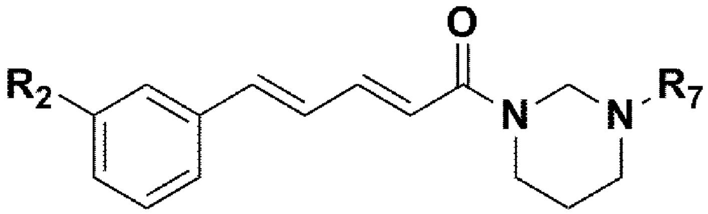

In Formula 7,

$R_2$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$X_1$, $X_2$, $X_3$, and $X_5$ is $CH_2$;
$X_4$ is $NR_7$;
$R_7$ is selected from H, a halogen, $R_{31}$, -OH, $-OR_{31}$, -SH, $-SR_{31}$, -C(=O)H, - C(=O)$R_{31}$, -C(=O)OH, -C(=O)$OR_{31}$, -C(=O)$NH_2$, -C(=O)$NHR_{31}$, and -C(=O)N$(R_{31})_2$ (or, $R_7$ is selected from -C(=O)$OR_3$ and -C(=O)$R_3$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 8 below or a pharmaceutically acceptable salt thereof is provided.

[Formula 8]

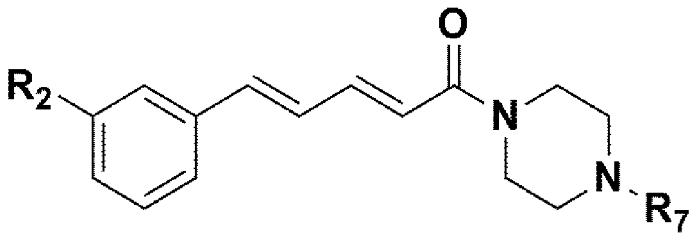

In Formula 8,

$R_2$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$X_3$ is $NR_7$;
$R_7$ is selected from H, a halogen, $R_{31}$, -OH, $-OR_{31}$, -SH, $-SR_{31}$, -C(=O)H, - C(=O)$R_{31}$, -C(=O)OH, -C(=O)O$R_{31}$, -C(=O)$NH_2$, -C(=O)NH$R_{31}$, and -C(=O)N$(R_{31})_2$ (or, $R_7$ is selected from -C(=O)O$R_{31}$ and -C(=O)$R_{31}$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 9 below or a pharmaceutically acceptable salt thereof is provided.

[Formula 9]

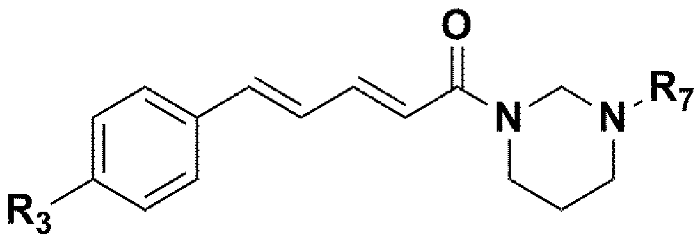

In Formula 9,

$R_3$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$X_4$ is $NR_7$;
$R_7$ is selected from H, a halogen, $R_{31}$, -OH, $-OR_{31}$, -SH, $-SR_{31}$, -C(=O)H, - C(=O)$R_{31}$, -C(=O)OH, -C(=O)O$R_{31}$, -C(=O)$NH_2$, -C(=O)NH$R_{31}$, and -C(=O)N$(R_{31})_2$ (or, $R_7$ is selected from -C(=O)O$R_{31}$ and -C(=O)$R_{31}$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 10 below or a pharmaceutically acceptable salt thereof is provided.

[Formula 10]

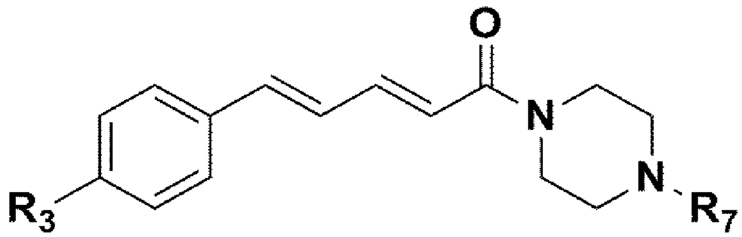

In Formula 10,

$R_3$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$X_3$ is $NR_7$;
$R_7$ is selected from H, a halogen, $R_{31}$, -OH, $-OR_{31}$, -SH, $-SR_{31}$, -C(=O)H, - C(=O)$R_{31}$, -C(=O)OH, -C(=O)O$R_{31}$, -C(=O)$NH_2$, -C(=O)NH$R_{31}$, and -C(=O)N$(R_{31})_2$ (or, $R_7$ is selected from -C(=O)O$R_{31}$ and -C(=O)$R_{31}$); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 11 or 12 below or a pharmaceutically acceptable salt thereof is provided.

[Formula 11]

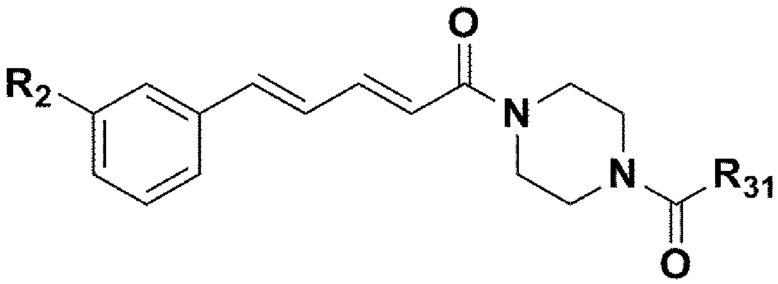

[Formula 12]

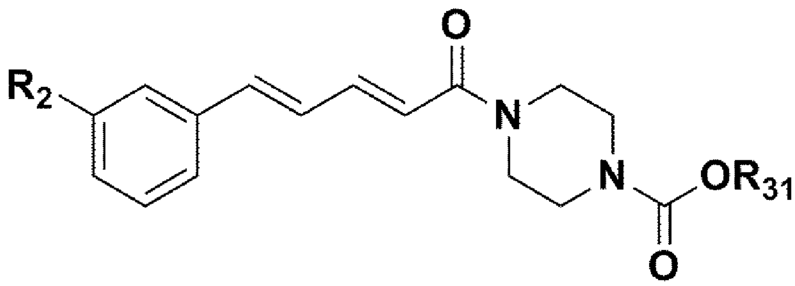

In Formula 11 or 12,

$R_2$ is selected from $-OCH_3$ and $-SCH_3$; and
$R_{31}$ is selected from methyl, ethyl, n-propyl, isopropyl, n-butyl, sec-butyl, isobutyl, and tert-butyl.

In some exemplary embodiments of the present application, a pentadienoyl compound represented by Formula 13 or 14 below or a pharmaceutically acceptable salt thereof is provided.

[Formula 13]

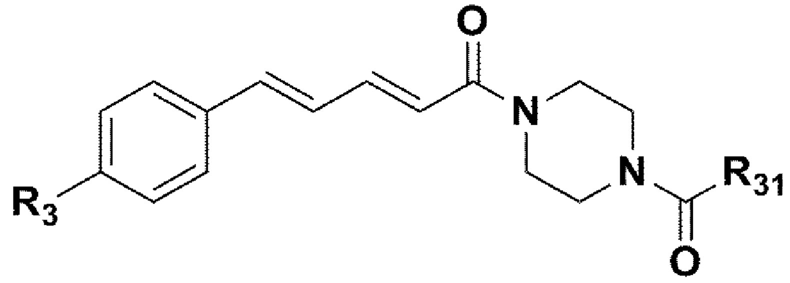

[Formula 14]

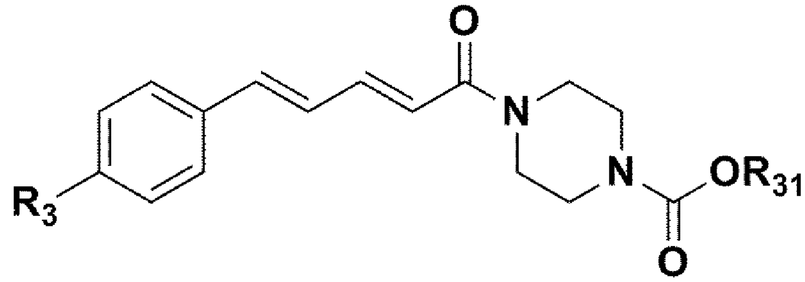

In Formula 13 or 14,

$R_3$ is selected from $-OCH_3$ and $-SCH_3$;
$R_{31}$ is selected from methyl, ethyl, n-propyl, isopropyl, n-butyl, sec-butyl, isobutyl, and tert-butyl.

In some exemplary embodiments of the present application, the pentadienoyl compound may include morpholinyl as a substituent at the first position. The morpholinyl may include morpholin-4-yl. The pentadienoyl compound may include a compound represented by any one of Formulas 2, and 15 to 20. The pentadienoyl compound in which the morpholinyl is located at the first position may include a compound represented by any one of Formulas 2, 16, and 18 to 20 below.

In some exemplary embodiments of the present application, a pentadienoyl compound represented by Formula 2 below or a pharmaceutically acceptable salt thereof is provided.

[Formula 2]

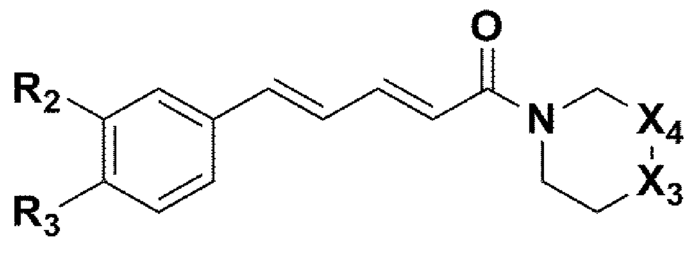

In Formula 2,

$R_2$ and $R_3$ are independently selected from H, $-OR_{11}$, and $-SR_{11}$;
each $R_{11}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl);
$X_3$ and $X_4$ are independently selected from $CR_6R_6$'and O;
both $X_3$ and $X_4$ are not $CH_2$ at once;
each $R_6$ and $R_6$' are independently selected from H, a halogen, $R_{31}$, -OH, - $OR_{31}$, -SH, $-SR_{31}$, -CN, $-N_3$, $-NH_2$,

$-NHR_{31}$, $-N(R_{31})_2$, $-C(=O)H$, $-C(=O)R_{31}$, - $C(=O)OH$, $-C(=O)OR_{31}$, $-C(=O)NH_2$, $-C(=O)NHR_{31}$, $-C(=O)N(R_{31})_2$, $-NHC(=O)H$, - $NHC(=O)OH$, and $-NHC(=O)R_{31}$ (or, $R_6$ and $R_6$' are H); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 15 below or a pharmaceutically acceptable salt thereof is provided.

[Formula 15]

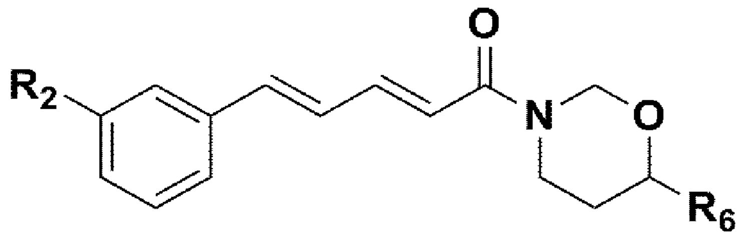

In Formula 15,

$R_2$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$R_6$ is selected from H, a halogen, $R_{31}$, -OH, $-OR_{31}$, -SH, $-SR_{31}$, -CN, $-N_3$, - $NH_2$, $-NHR_{31}$, $-N(R_{31})_2$, $-C(=O)H$, $-C(=O)R_{31}$, $-C(=O)OH$, $-C(=O)OR_{31}$, $-C(=O)NH_2$, $-C(=O)NHR_{31}$, $-C(=O)N(R_{31})_2$, $-NHC(=O)H$, $-NHC(=O)OH$, and $-NHC(=O)R_{31}$ (or, $R_6$ is H); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 16 below or a pharmaceutically acceptable salt thereof is provided.

[Formula 16]

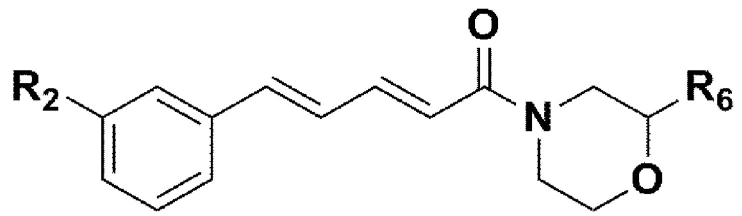

In Formula 16,

$R_2$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$R_6$ is selected from H, a halogen, $R_{31}$, -OH, $-OR_{31}$, -SH, $-SR_{31}$, -CN, $-N_3$, - $NH_2$, - $-N(R_{31})_2$, $-C(=O)H$, $-C(=O)R_{31}$, $-C(=O)OH$, $-C(=O)OR_{31}$, $-C(=O)NH_2$, $-C(=O)NHR_{31}$, $-C(=O)N(R_{31})_2$, $-NHC(=O)H$, $-NHC(=O)OH$, and $-NHC(=O)R_{31}$ (or, $R_6$ is H); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 17 below or a pharmaceutically acceptable salt thereof is provided.

[Formula 17]

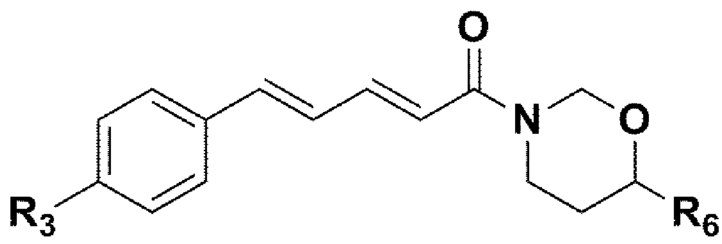

In Formula 17,

$R_3$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$R_6$ is selected from H, a halogen, $R_{31}$, -OH, $-OR_{31}$, -SH, $-SR_{31}$, -CN, $-N_3$, - $NH_2$, $-NHR_{31}$, $-N(R_{31})_2$, -C(=O)H, $-C(=O)R_{31}$, -C(=O)OH, $-C(=O)OR_{31}$, $-C(=O)NH_2$, $-C(=O)NHR_{31}$, $-C(=O)N(R_{31})_2$, -NHC(=O)H, -NHC(=O)OH, and $-NHC(=O)R_{31}$ (or, $R_6$ is H); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 18 below or a pharmaceutically acceptable salt thereof is provided.

[Formula 18]

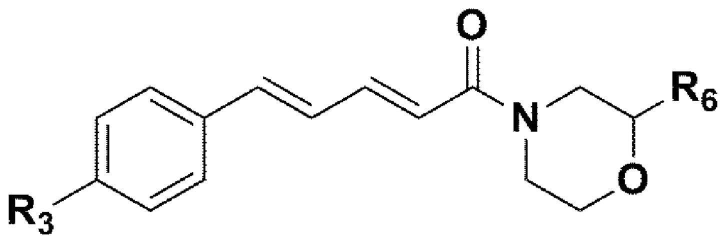

In Formula 18,

$R_3$ is selected from $-OR_{11}$ and $-SR_{11}$;
each $R_{11}$ is independently unsubstituted or substituted $C_1$-$C_6$ alkyl, wherein the alkyl is linear alkyl, branched alkyl, or cycloalkyl;
$R_6$ is selected from H, a halogen, $R_{31}$, -OH, $-OR_{31}$, -SH, $-SR_{31}$, -CN, $-N_3$, - $NH_2$, $-NHR_{31}$, $-N(R_{31})_2$, -C(=O)H, $-C(=O)R_{31}$, -C(=O)OH, $-C(=O)OR_{31}$, $-C(=O)NH_2$, $-C(=O)NHR_{31}$, $-C(=O)N(R_{31})_2$, -NHC(=O)H, -NHC(=O)OH, and $-NHC(=O)R_{31}$ (or, $R_6$ is H); and
each $R_{31}$ is independently unsubstituted or substituted $C_1$-$C_6$ alkyl, wherein the alkyl is linear alkyl, branched alkyl, or cycloalkyl (the alkyl may include methyl, ethyl, n-propyl, isopropyl, n-butyl, sec-butyl, isobutyl, and tert-butyl).

In some exemplary embodiments of the present application, a pentadienoyl compound represented by Formula 19 below or a pharmaceutically acceptable salt thereof is provided.

[Formula 19]

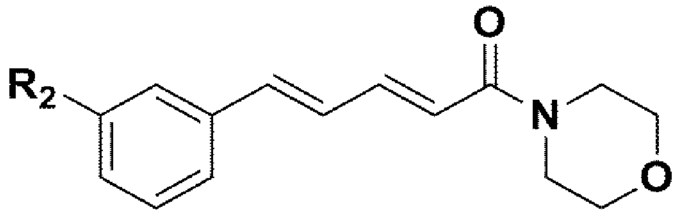

In Formula 19,
$R_2$ is selected from $-OCH_3$ and $-SCH_3$.

In some exemplary embodiments of the present application, a pentadienoyl compound represented by Formula 20 below or a pharmaceutically acceptable salt thereof is provided.

[Formula 20]

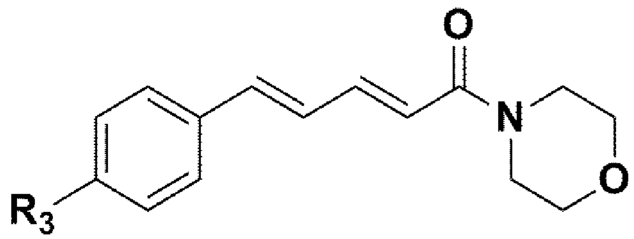

In Formula 20,
$R_3$ is selected from $-OCH_3$ and $-SCH_3$.

The compound represented by Formula 1 specifically includes compounds having the structures R-1 to R-30 of Table 1 below, but the present application is not limited thereto.

[Table 1]

| No. | Structural Formula |
|---|---|
| R-1 | 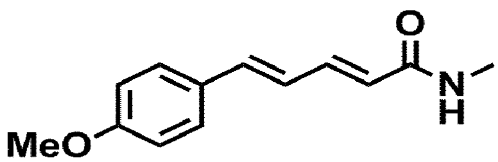 |
| R-2 | 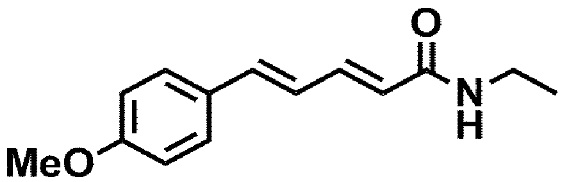 |
| R-3 | 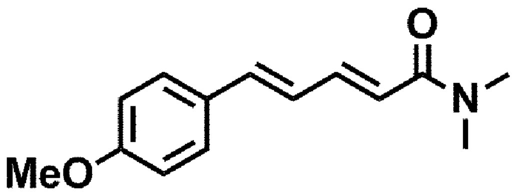 |

(continued)

| No. | Structural Formula |
|---|---|
| R-4 | 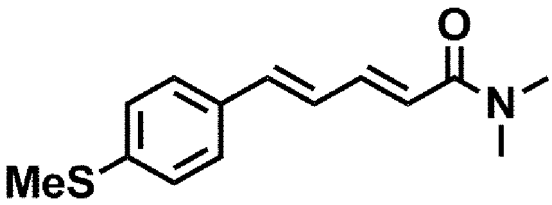 |
| R-5 | 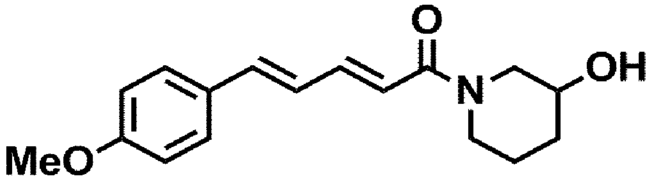 |
| R-6 | 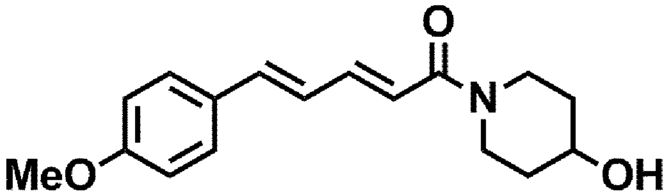 |
| R-7 | 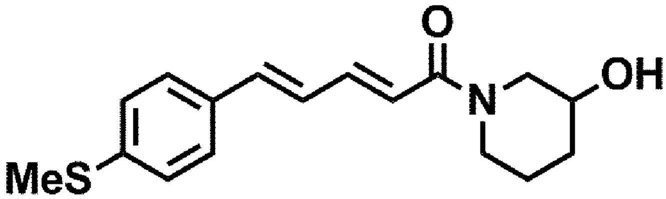 |
| R-8 | 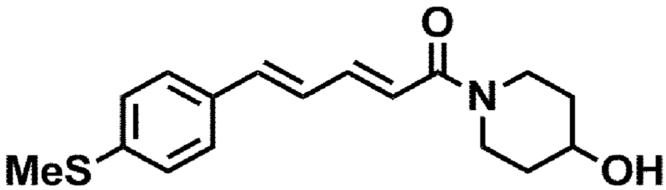 |
| R-9 | 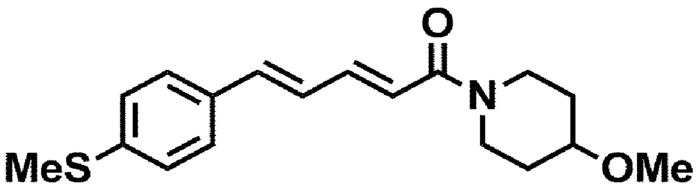 |
| R-10 | 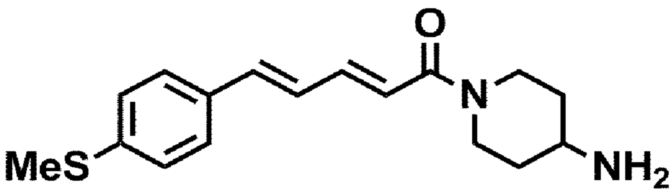 |

(continued)

| No. | Structural Formula |
|---|---|
| R-11 | 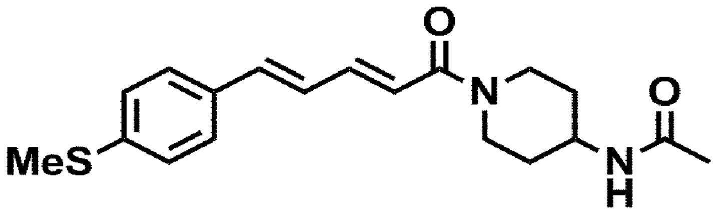 |
| R-12 | 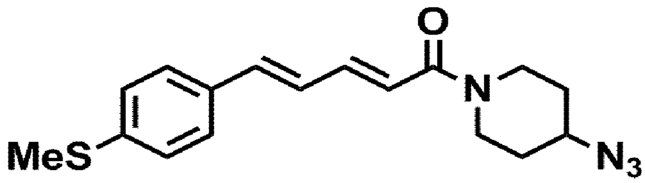 |
| R-13 | 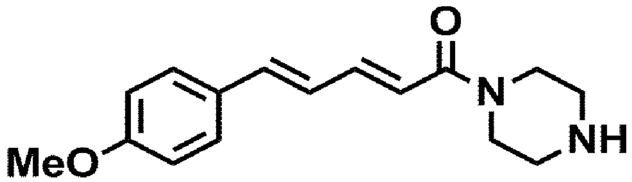 |
| R-14 | 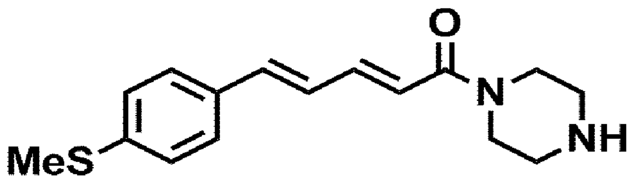 |
| R-15 | 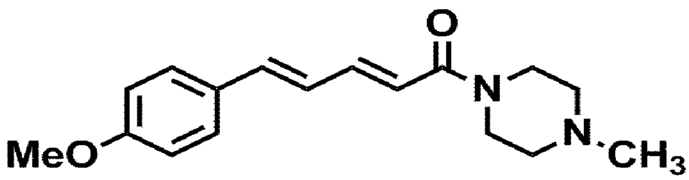 |
| R-16 | 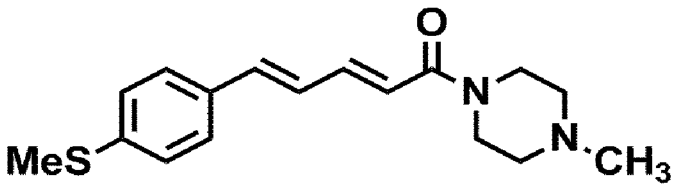 |
| R-17 | 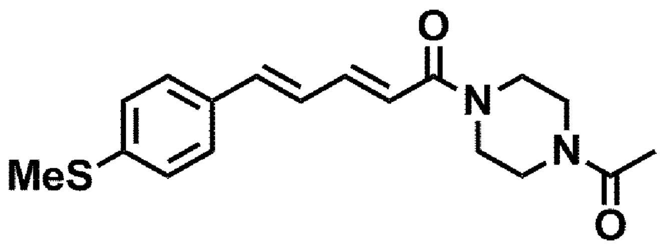 |

(continued)

| No. | Structural Formula |
|---|---|
| R-18 BWL-233 | 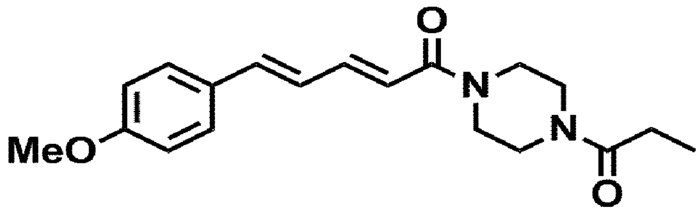 |
| R-19 BWL-258 | 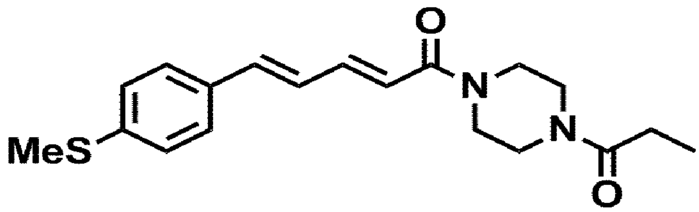 |
| R-20 BWL-215 | 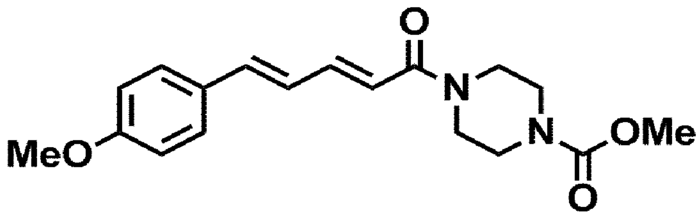 |
| R-21 BWL-216 | 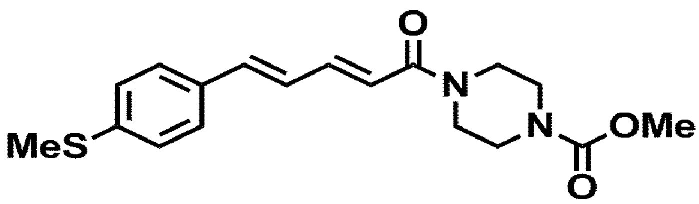 |
| R-22 BWL-231 | 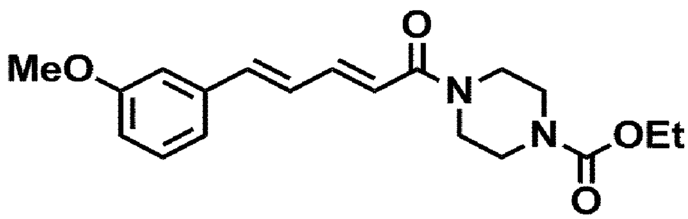 |
| R-23 BWL-211 | 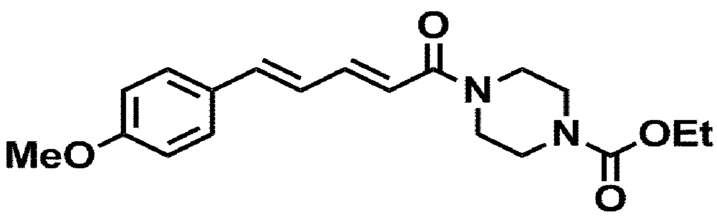 |
| R-24 BWL-212 | 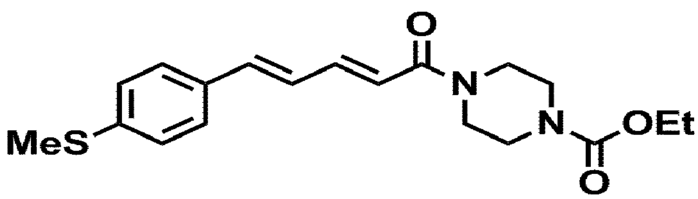 |

(continued)

| No. | Structural Formula |
|---|---|
| R-25 BWL-232 | 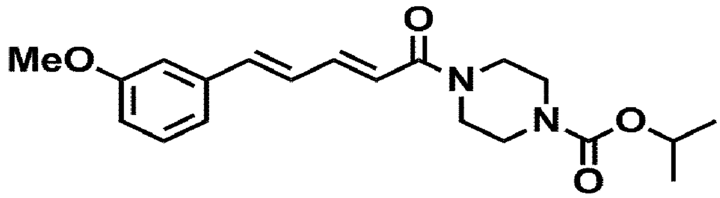 |
| R-26 BWL-213 | 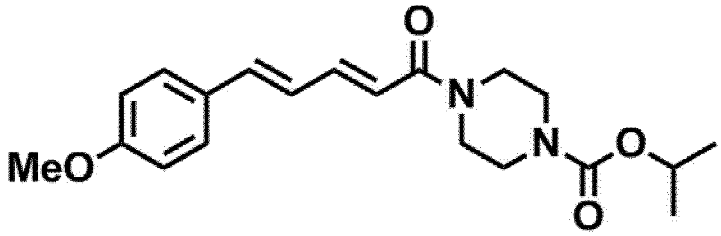 |
| R-27 BWL-214 | 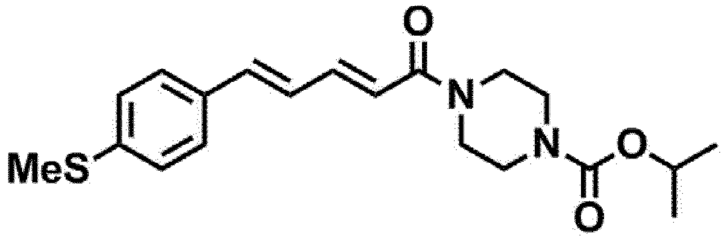 |
| R-28 BWL-102 | 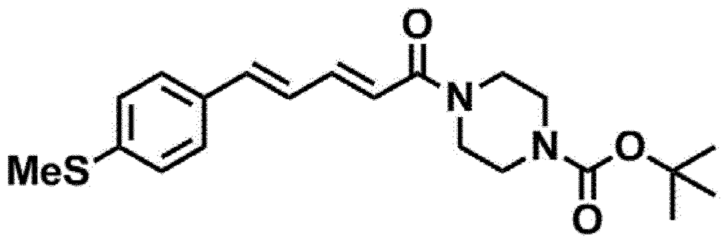 |
| R-29 | 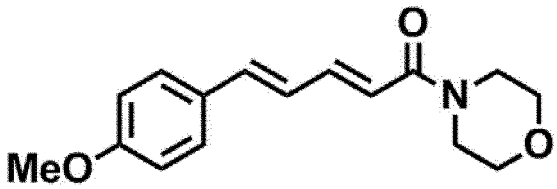 |
| R-30 | 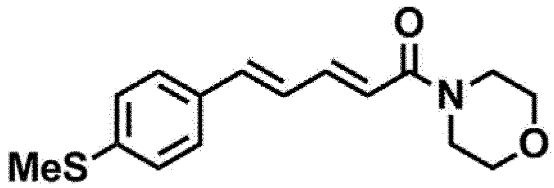 |

The compound R-1 is a compound represented by Formula 1, in which $R_1$, $R_2$, $R_4$, and $R_5$ are H;

$R_3$ is $-OCH_3$; and

A is

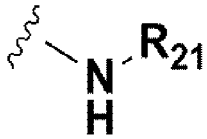

,

wherein $R_{21}$ is methyl.

The compound R-2 is a compound represented by Formula 1,

in which $R_1$, $R_2$, $R_4$, and $R_5$ are H;
$R_3$ is $-OCH_3$; and
A is

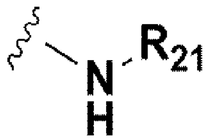

,

wherein $R_{21}$ is ethyl.

The compound R-3 is a compound represented by Formula 1,

in which $R_1$, $R_2$, $R_4$, and $R_5$ are H;
$R_3$ is $-OCH_3$; and
A is

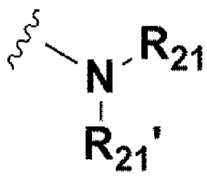

,

wherein $R_{21}$ is methyl, and $R_{21}$' is methyl.

The compound R-4 is a compound represented by Formula 1,

in which $R_1$, $R_2$, $R_4$, and $R_5$ are H;
$R_3$ is $-SCH_3$; and
A is

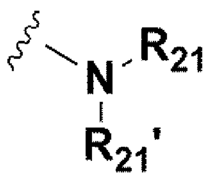

,

wherein $R_{21}$ is methyl, and $R_{21}$' is methyl.

The compound R-5 is a compound represented by Formula 5,

in which $R_3$ is $-OCH_3$; and

$R_6$ is -OH.

The compound R-6 is a compound represented by Formula 6,

in which $R_3$ is $-OCH_3$; and
$R_6$ is -OH.

The compound R-7 is a compound represented by Formula 5,

in which $R_3$ is $-SCH_3$; and
$R_6$ is -OH.

The compound R-8 is a compound represented by Formula 6,

in which $R_3$ is $-SCH_3$; and
$R_6$ is -OH.

The compound R-9 is a compound represented by Formula 6,

in which $R_2$ is $-SCH_3$; and
$R_6$ is $-OR_{31}$,
wherein $R_{31}$ is methyl.

The compound R-10 is a compound represented by Formula 6,

in which $R_3$ is $-SCH_3$; and
$R_6$ is $-NH_2$.

The compound R-11 is a compound represented by Formula 6,

in which $R_3$ is $-SCH_3$; and
$R_6$ is $-NHC(=O)R_{31}$,
wherein $R_{31}$ is methyl.

The compound R-12 is a compound represented by Formula 6,

in which $R_3$ is $-SCH_3$; and
$X_6$ is $-N_3$.

The compound R-13 is a compound represented by Formula 10,

in which $R_3$ is - $OCH_3$; and
$R_7$ is H.

The compound R-14 is a compound represented by Formula 10,

in which $R_3$ is $-SCH_3$; and
$R_7$ is H.

The compound R-15 is a compound represented by Formula 10,

in which $R_3$ is $-OCH_3$; and
$R_7$ is methyl.

The compound R-16 is a compound represented by Formula 10,

in which $R_3$ is $-SCH_3$; and
$R_7$ is methyl.

The compound R-17 is a compound represented by Formula 13,

in which $R_3$ is $-SCH_3$; and
$R_{31}$ is methyl.

The compound R-18 (BWL-233) is a compound represented by Formula 13,

in which $R_3$ is $-OCH_3$; and
$R_{31}$ is ethyl.

The compound R-19 (BWL-258) is a compound represented by Formula 13,

in which $R_3$ is $-SCH_3$; and
$R_{31}$ is ethyl.

The compound R-20 (BWL-215) is a compound represented by Formula 14,

in which $R_3$ is $-OCH_3$; and
$R_{31}$ is methyl.

The compound R-21 (BWL-216) is a compound represented by Formula 14,

in which $R_3$ is $-SCH_3$; and
$R_{31}$ is methyl.

The compound R-22 (BWL-231) is a compound represented by Formula 12,

in which $R_2$ is $-OCH_3$; and
$R_{31}$ is ethyl.

The compound R-23 (BWL-211) is a compound represented by Formula 14,

in which $R_3$ is $-OCH_3$; and
$R_{31}$ is ethyl.

The compound R-24 (BWL-212) is a compound represented by Formula 14,

in which $R_3$ is $-SCH_3$; and
$R_{31}$ is ethyl.

The compound R-25 (BWL-232) is a compound represented by Formula 12,

in which $R_2$ is $-OCH_3$; and
$R_{31}$ is isopropyl.

The compound R-26 (BWL-213) is a compound represented by Formula 14,

in which $R_3$ is $-OCH_3$; and
$R_{31}$ is isopropyl.

The compound R-27 (BWL-214) is a compound represented by Formula 14,

in which $R_3$ is $-SCH_3$; and
$R_{31}$ is isopropyl.

The compound R-28 (BWL-102) is a compound represented by Formula 14,

in which $R_3$ is $-SCH_3$; and

$R_{31}$ is tert-butyl.

The compound R-29 is a compound represented by Formula 20,
in which $R_3$ is $-OCH_3$.

The compound R-30 is a compound represented by Formula 20,
in which $R_3$ is $-SCH_3$.

The compound of the present application may be used in the form of a pharmaceutically acceptable salt, and in this case, an acid addition salt formed by a pharmaceutically acceptable free acid. Acid addition salts are obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono- and dicarboxylates, phenyl-substituted alkanoates, hydroxy alkanoates and alkanedioates, aromatic acids, aliphatic and aromatic sulfonic acids; and organic acids such as acetic acid, benzoic acid, citric acid, lactic acid, maleic acid, gluconic acid, methanesulfonic acid, 4-toluenesulfonic acid, tartaric acid and fumaric acid. Examples of the pharmaceutically non-toxic salts include sulfates, pyrosulfates, bisulfates, sulfites, bisulfites, nitrates, phosphates, monohydrogen phosphates, dihydrogen phosphates, metaphosphates, pyrophosphate chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butyne-1,4-dioates, hexane-1,6-dioates, benzoates, chlorobenzoates, methylbenzoates, dinitrobenzoates, hydroxybenzoates, methoxybenzoates, phthalates, terephthalates, benzenesulfonates, toluene sulfonates, chlorobenzene sulfonates, xylene sulfonates, phenylacetates, phenylpropionates, phenylbutyrates, citrates, lactates, β-hydroxybutyrates, glycolates, malates, tartrates, methanesulfonates, propanesulfonates, naphthalene-1-sulfonates, naphthalene-2-sulfonates, and mandelates.

The acid addition salt according to the present application may be prepared by a conventional method, and for example, may be prepared by dissolving a derivative of the compound in an organic solvent such as methanol, ethanol, acetone, dichloromethane or acetonitrile, filtering and drying a precipitate produced by applying an organic acid or inorganic acid, or prepared by performing distillation under reduced pressure for a solvent and an excessive amount of acid, drying the resulting product, and performing crystallization in the presence of an organic solvent.

In addition, a pharmaceutically acceptable metal salt may be prepared by using a base. For example, an alkali metal or alkali earth metal salt is obtained by dissolving a compound in a solution of an excessive alkali metal hydroxide or alkali earth metal hydroxide, filtering a non-soluble compound salt, evaporating and drying the filtrate. Here, as a metal salt, a sodium, potassium or calcium salt is pharmaceutically suitable. In addition, a salt corresponding thereto is obtained by reacting an alkali metal or alkali earth metal salt with a suitable silver salt (e.g., silver nitrate).

Further, the present application includes all of solvates, stereoisomers and hydroxides as well as compounds and pharmaceutically acceptable salts thereof.

The compound or pharmaceutically acceptable salt thereof according to the present application may be used to inhibit lipogenesis in cells. The compound or pharmaceutically acceptable salt thereof according to the present application may be used to inhibit lipogenesis in adipocytes. The compound or pharmaceutically acceptable salt thereof according to the present application may be used to inhibit lipogenesis in liver cells. The compound or pharmaceutically acceptable salt thereof according to the present application may be used to inhibit lipogenesis in bio tissue. The compound or pharmaceutically acceptable salt thereof according to the present application may be used to inhibit lipogenesis in adipose tissue. The compound or pharmaceutically acceptable salt thereof according to the present application may be used to inhibit lipogenesis in liver tissue. The compound or pharmaceutically acceptable salt thereof according to the present application may be used to inhibit lipogenesis in the living body.

The compound or pharmaceutically acceptable salt thereof according to the present application may be used to inhibit lipid accumulation in cells. The compound or pharmaceutically acceptable salt thereof according to the present application may be used to inhibit lipid accumulation in adipocytes. The compound or pharmaceutically acceptable salt thereof according to the present application may be used to inhibit lipid accumulation in liver cells. The compound or pharmaceutically acceptable salt thereof according to the present application may be used to inhibit lipid accumulation in bio tissue. The compound or pharmaceutically acceptable salt thereof according to the present application may be used to inhibit lipid accumulation in adipose tissue. The compound or pharmaceutically acceptable salt thereof according to the present application may be used to inhibit lipid accumulation in liver tissue. The compound or pharmaceutically acceptable salt thereof according to the present application may be used to inhibit lipid accumulation in the living body.

The compound or pharmaceutically acceptable salt thereof according to the present application may be used to activate the mechanism of fatty acid β oxidation in cells. The compound or pharmaceutically acceptable salt thereof according to the present application may be used to activate the mechanism of fatty acid β oxidation in adipocytes. The compound or pharmaceutically acceptable salt thereof according to the present application may be used to activate the mechanism of fatty acid β oxidation in liver cells. The compound or pharmaceutically acceptable salt thereof according to the present application may be used to activate the mechanism of fatty acid β oxidation in adipose tissue. The compound or pharmaceutically acceptable salt thereof according to the present application may be used to activate the

mechanism of fatty acid β oxidation in liver tissue. The compound or pharmaceutically acceptable salt thereof according to the present application may be used to activate the mechanism of fatty acid β oxidation in a bio tissue. The compound or pharmaceutically acceptable salt thereof according to the present application may be used to activate the mechanism of fatty acid β oxidation in the living body.

The compound or pharmaceutically acceptable salt thereof according to the present application may be used to inhibit the mechanism of fat biosynthesis in cells. The compound or pharmaceutically acceptable salt thereof according to the present application may be used to inhibit the mechanism of fat biosynthesis in adipocytes. The compound or pharmaceutically acceptable salt thereof according to the present application may be used to inhibit the mechanism of fat biosynthesis in liver cells. The compound or pharmaceutically acceptable salt thereof according to the present application may be used to inhibit the mechanism of fat biosynthesis in adipose tissue. The compound or pharmaceutically acceptable salt thereof according to the present application may be used to inhibit the mechanism of fat biosynthesis in liver tissue. The compound or pharmaceutically acceptable salt thereof according to the present application may be used to inhibit the mechanism of fat biosynthesis in bio tissue. The compound or pharmaceutically acceptable salt thereof according to the present application may be used to inhibit the mechanism of fat biosynthesis in the living body.

The compound or pharmaceutically acceptable salt thereof according to the present application may be used to activate the mechanism of regulating lipid metabolism in cells. The compound or pharmaceutically acceptable salt thereof according to the present application may be used to activate the mechanism of regulating lipid metabolism in adipocytes. The compound or pharmaceutically acceptable salt thereof according to the present application may be used to activate the mechanism of regulating lipid metabolism in liver cells. The compound or pharmaceutically acceptable salt thereof according to the present application may be used to activate the mechanism of regulating lipid metabolism in adipose tissue. The compound or pharmaceutically acceptable salt thereof according to the present application may be used to activate the mechanism of regulating lipid metabolism in liver tissue. The compound or pharmaceutically acceptable salt thereof according to the present application may be used to activate the mechanism of regulating lipid metabolism in bio tissue. The compound or pharmaceutically acceptable salt thereof according to the present application may be used to activate the mechanism of regulating lipid metabolism in the living body.

The compound or pharmaceutically acceptable salt thereof according to the present application may be used to prevent or treat at least one of fatty liver and fatty liver-related disease. The compound or pharmaceutically acceptable salt thereof according to the present application may be used to prevent or treat at least one of obesity, fatty liver, NAFLD, NASH, hepatic fibrosis, cirrhosis, liver cancer, diabetes, dyslipidemia, hyperinsulinemia, insulin resistance, insulin resistance syndrome, and metabolic syndrome. The compound or pharmaceutically acceptable salt thereof according to the present application may be used to prevent or treat at least one of steatosis, NASH, and hepatic fibrosis.

The compound or pharmaceutically acceptable salt thereof according to the present application may be used as a SIRT1-activating compound.

The compound or pharmaceutically acceptable salt thereof according to the present application may be used to prevent or treat a SIRT1-mediated disease.

Aging/stress

In one aspect, the present application may provide a method of extending the lifespan of cells, increasing the proliferation ability of cells, delaying the aging of cells, promoting the survival of cells, delaying cellular senescence in cells, mimicking a calorie-restricting effect, increasing stress-mediated cell resistance, or preventing cell apoptosis by bringing the cells into contact with the compound or pharmaceutically acceptable salt thereof according to the present application, which increases a SIRT1 protein level and/or activity. In an exemplary embodiment, the method or use may include bringing cells into contact with the compound or pharmaceutically acceptable salt thereof according to the present application.

The compound or pharmaceutically acceptable salt thereof according to the present application may be used to increase an amount of time for keeping cells, particularly, primary cells (i.e., cells obtained from an organism, e.g., a human) alive during cell culture. In addition, as embryonic stem (ES) cells and pluripotent cells, and cells differentiated therefrom are treated with the compound or pharmaceutically acceptable salt thereof according to the present application which increases the level and/or activity of SIRT1 protein, cells or descendants thereof may be further maintained in culture for a long time. These cells may also be used for implantation into a subject, for example, after *in vitro* modification.

In one aspect, cells intended to be retained for a long time may be treated with the compound or pharmaceutically acceptable salt thereof according to the present application which increases the level and/or activity of SIRT1 protein. The cells may be present in a suspension (e.g., blood cells, serum, a biological growth medium or the like), or tissue or an organ. For example, as blood collected from an individual for blood transfusion may be treated with the compound or pharmaceutically acceptable salt thereof according to the present application, which increases the level and/or activity of SIRT1 protein, blood cells may be stored for a longer period of time. In addition, blood to be used for Forensic Science may be preserved using the compound or pharmaceutically acceptable salt thereof according to the present application

which increases the level and/or activity of SIRT1 protein. Other cells that can be treated to extend a lifespan or be protected from apoptosis include cells for consumption, for example, cells derived from a non-human mammal (e.g., meat) or plant cells (e.g., vegetable).

The compound or pharmaceutically acceptable salt thereof according to the present application, which increases the level and/or activity of SIRT1 protein, may also be applied during the genesis and growth periods in mammals, plant, insects or microorganisms to change, delay or promote genesis and/or development procedures.

In another aspect, the compound or pharmaceutically acceptable salt thereof according to the present application, which increases the level and/or activity of SIRT1 protein, may be used to treat a solid tissue graft, an organ implant, a cell suspension, or cells useful for implantation or cell therapy, which include stem cells and bone marrow cells. Cells or tissue may be an autograft, an allograft, an isograft or a xenograft. Cells or tissue may be treated with the compound or pharmaceutically acceptable salt thereof according to the present application before administration/implantation into a subject, during administration/implantation, and/or after administration/implantation. Cells or tissue may be treated before the removal of cells from a donor individual, *in vitro* after the removal of cells or tissue from the donor individual, or after the implantation into a recipient. For example, the donor or recipient individual may be systemically treated with the compound or pharmaceutically acceptable salt thereof according to the present application, or may have a subset of cells/tissue locally treated with the compound or pharmaceutically acceptable salt thereof according to the present application, which increases the level and/or activity of SIRT1 protein. In a specific exemplary embodiment, the cells or tissue (or donor/recipient individual) may be additionally treated with another therapeutic agent useful for extending graft survival, for example, an immunosuppressant, a cytokine, or an angiogenic factor.

In another exemplary embodiment, cells may be treated with the compound or pharmaceutically acceptable salt thereof according to the present application, which increases the level and/or activity of SIRT1 protein in the living body, thereby, for example, increasing the lifespan or preventing apoptosis of the cells. For example, dermal or epithelial cells may be treated with the compound or pharmaceutically acceptable salt thereof according to the present application, which increases the level and/or activity of SIRT1 protein, thereby protecting the skin from aging (e.g., wrinkling, the loss of elasticity, etc.). In an exemplary embodiment, the skin is brought into contact with a pharmaceutical or cosmetic composition, which includes the compound or pharmaceutically acceptable salt thereof according to the present application, which increases the level and/or activity of SIRT1 protein. Exemplary dermal pain or conditions which can be treated according to the method or use described in the present application are associated with inflammation, sun damage or natural aging, or include a disorder or disease caused thereby. For example, the composition is useful in prevention or treatment of contact dermatitis (including irritant contact dermatitis and allergic contact dermatitis), atopic dermatitis (also known as allergic eczema), actinic keratosis, cornification diseases (including eczema), epidermolysis bullosa diseases (including pemphigus), exfoliative dermatitis, seborrheic dermatitis, erythema (including erythema multiforme and erythema nodosum), damage caused by sun light or a different light source, discoid lupus erythematosus, dermatomyositis, psoriasis, skin cancer and natural aging. In still another exemplary embodiment, the compound or pharmaceutically acceptable salt thereof according to the present application, which increases the level and/or activity of SIRT1 protein, may be used to promote curing by treating, for example, wounds and/or burns including first, second or third-degree burns and/or thermal, chemical or electrical burns. The formulation may be topically administered on dermal or mucosal tissue.

A topical formulation including one or more compounds or pharmaceutically acceptable salts thereof according to the present application, which increase the level and/or activity of SIRT1 protein, may also be used as a prophylactic, for example, a chemopreventive composition. When such formulation is used in a chemopreventive method, susceptible skin may be treated before any visible condition in a particular individual.

The compound or pharmaceutically acceptable salt thereof according to the present application may be locally or systemically delivered to a subject. In a specific exemplary embodiment, the compound or pharmaceutically acceptable salt thereof according to the present application may be locally delivered to tissue or an organ of a subject by injection or a topical formulation.

In a yet another exemplary embodiment, the compound or pharmaceutically acceptable salt thereof according to the present application, which increases the level and/or activity of SIRT1 protein, may be used to treat or prevent a disease or condition which is caused or aggravated by cellular senescence in a subject; for example, in a method of decreasing a senescence rate of a subject after the onset of senescence or for that purpose; in a method of extending the lifespan of a subject; as a method or condition of treating or preventing a lifespan-related disease or for that purpose; in a method of treating or preventing a disease or condition related to the proliferative ability of cells or for that purpose; and in a method of treating or preventing a disease or condition caused by cell damage or death or for that purpose. In a specific exemplary embodiment, the method does not work by reducing the incidence of a disease shortening the lifespan of the subject. In a specific exemplary embodiment, the method does not work by reducing the mortality caused by a disease, for example, cancer.

In yet another exemplary embodiment, the compound or pharmaceutically acceptable salt thereof according to the present application, which increases the level and/or activity of SIRT1 protein, may be administered to generally

increase the lifespan of cells of a subject, and to protect cells of a subject from stress and/or apoptosis. It is considered that the treatment of a subject with the compound described in the present application is similar to subjecting a subject to hormesis, that is, mild stress which is beneficial to an organism and may extend the lifespan thereof.

The compound or pharmaceutically acceptable salt thereof according to the present application, which increases the level and/or activity of SIRT1 protein, may be administered to a subject to prevent aging, and aging-related prognosis or diseases, for example, stroke, heart disease, heart failure, arthritis, high blood pressure and Alzheimer's disease. Other conditions that can be treated include, for example, ocular diseases associated with the aging of the eye, such as cataracts, glaucoma and macular degeneration. The compound or pharmaceutically acceptable salt thereof according to the present application, which increases the level and/or activity of SIRT1 protein, may also be administered to a subject to protect cells from cell death by treating a cell death-associated disease, for example, a chronic disease. Exemplary diseases include diseases associated with neuronal death or dysfunction, or muscle cell death or dysfunction, for example, Parkinson's diseases, Alzheimer's disease, multiple sclerosis, amyotrophic lateral sclerosis, and muscular dystrophy; AIDS; fulminant hepatitis; diseases associated with degeneration of the brain, such as Creutzfeldt-Jakob disease, pigmented retinitis and cerebellar degeneration; myelodysplasia such as aplastic anemia; ischemic diseases such as myocardial infarction and stroke; liver diseases such as alcoholic hepatitis, type B hepatitis and type C hepatitis; a joint disease such as osteoarthritis; atherosclerosis; alopecia; a damage to skin due to UV light; lichen planus; skin atrophy; cataracts; and graft rejection. Cell death may also be caused by surgery, drug therapy, chemical exposure or radiation exposure.

The compound or pharmaceutically acceptable salt thereof according to the present application, which increases the level and/or activity of SIRT1 protein, may also be administered to a subject suffering from an acute disease, such as damage to an organ or tissue, for example, a subject suffering from stroke or myocardial infarction or a subject suffering from spinal cord injury. The compound or pharmaceutically acceptable salt thereof according to the present application, which increases the level and/or activity of SIRT1 protein, may also be used to repair an alcoholic liver.

Cardiovascular disease

In yet another exemplary embodiment, the present application may provide a method of treating and/or preventing a cardiovascular disease by administering the compound or pharmaceutically acceptable salt thereof according to the present application, which increases the level and/or activity of SIRT1 protein, to a subject required to treat and/or prevent a cardiovascular disease.

Cardiovascular diseases that can be treated or prevented using the compound or pharmaceutically acceptable salt thereof according to the present application, which increases the level and/or activity of SIRT1 protein, include cardiomyopathy or myocarditis; for example, idiopathic cardiomyopathy, metabolic cardiomyopathy, alcoholic cardiomyopathy, drug-induced cardiomyopathy, ischemic cardiomyopathy, and hypertensive cardiomyopathy. Diseases to be treated or prevented using the compound, method or use described in the present application are atherosclerosis (macrovascular disease) of a major blood vessel, such as the aorta, the coronary artery, the carotid artery, the cerebrovascular artery, the renal artery, the iliac artery, the femoral artery, and the dorsal artery. Other vascular diseases to be treated or prevented include those related to platelet aggregation, the retinal arterioles, the glomerular arterioles, the vasa nervorum, cardiac arterioles, and associated capillary beds of the eye, the kidney, the heart, and the central and peripheral nervous systems. The compound or pharmaceutically acceptable salt thereof according to the present application, which increases the level and/or activity of SIRT1 protein, may also be used for increasing the HDL level in plasma of an individual.

Yet other diseases that can be treated with the compound or pharmaceutically acceptable salt thereof according to the present application, which increases the level and/or activity of SIRT1 protein, include restenosis, for example, after coronary intervention, and diseases relating to an abnormal level of high density and low density cholesterol.

In a specific exemplary embodiment, the compound or pharmaceutically acceptable salt thereof according to the present application, which increases the level and/or activity of SIRT1 protein, may be administered as a part of combination therapy with another cardiovascular agent. In a specific exemplary embodiment, the compound or pharmaceutically acceptable salt thereof according to the present application, which increases the level and/or activity of SIRT1 protein, may be administered as a part of combination therapy with an anti-arrhythmia agent. In yet another exemplary embodiment, the compound or pharmaceutically acceptable salt thereof according to the present application, which increases the level and/or activity of SIRT1 protein, may be administered as a part of combination therapy with another cardiovascular agent.

Cell death/cancer

The compound or pharmaceutically acceptable salt thereof according to the present application, which increases the level and/or activity of SIRT1 protein, may be administered to a subject who has recently received or is likely to

receive a predetermined dose of radiation or toxin. In a particular exemplary embodiment, the dose of radiation or toxin is received as a part of a work-related or medical procedure, and for example, administered as a prophylactic measure. In yet another exemplary embodiment, the radiation or toxin exposure is received unintentionally. In this case, the compound is preferably administered as soon as possible, after exposure to inhibit apoptosis and the subsequent development of an acute radiation syndrome.

The compound or pharmaceutically acceptable salt thereof according to the present application may be used to treat and/or prevent cancer. In a particularly exemplary embodiment, the compound or pharmaceutically acceptable salt thereof according to the present application, which increases the level and/or activity of SIRT1 protein, may be used to treat and/or prevent cancer. Calorie restriction has been associated with the reduction in the occurrence of age-related diseases including cancer. Accordingly, an increase in the SIRT1 protein level and/or activity may be useful for treating and/or preventing the occurrence of an age-related diseases, such as cancer. Exemplary cancers that can be treated using the compound or pharmaceutically acceptable salt thereof according to the present application are brain and kidney cancers; hormone-dependent cancers including breast cancer, prostatic cancer, testicular cancer and ovarian cancer; lymphoma, and leukemia. In cancers associated with solid tumors, a modulating compound may be administered directly to a tumor. Cancer of blood cells, for example, leukemia, may be treated by administering a modulating compound to the blood stream or the bone marrow. Benign cell growth, for example, warts, may also be treated. Other diseases which can be treated include autoimmune diseases in which autoimmune cells should be removed, for example, systemic lupus erythematosus, scleroderma, and arthritis. Viral infections such as herpes, HIV, adenoviruses, and HTLV-1 associated malignant and benign diseases may be treated by administration of the compound or pharmaceutically acceptable salt thereof according to the present application. Alternatively, cells may be obtained from a subject, treated *in vitro* to remove cancer cells, which are undesirable cells, or administered again to the same or different subj ect.

Chemotherapeutic agents may be co-administered with the modulating compounds described herein as having anti-cancer activity, e.g., compounds inducing apoptosis, compounds reducing a lifespan, or compounds rendering cells susceptible to stress. The chemotherapeutic agents themselves may induce cell death, reduce a lifespan or increase sensitivity to stress, and may be used in combination with the compound or pharmaceutically acceptable salt thereof according to the present application and/or other chemotherapeutic agents. In addition to conventional chemotherapeutic agents, the compound or pharmaceutically acceptable salt thereof according to the present application may also be used with antisense RNA, RNAi or other polynucleotides to inhibit the expression of cell components that contribute to undesired cell proliferation.

Combination therapies including the compound or pharmaceutically acceptable salt thereof according to the present application and a conventional chemotherapeutic agent may be advantageous, compared to combination therapies known in the art, which is because the combination allows conventional chemotherapeutic agents to exhibit a greater effect at a lower dose. In some exemplary embodiments, when being used in combination with the compound or pharmaceutically acceptable salt thereof according to the present application, the effective dose ($ED_{50}$) for a chemotherapeutic agent or combination with a conventional chemotherapeutic agent is at least two fold, and more preferably, 5 fold, 10 fold or even 25 fold less than the ED50 for the chemotherapeutic agent alone. On the other hand, when being used in combination with the compound or pharmaceutically acceptable salt thereof according to the present application, the therapeutic index (TI) for such chemotherapeutic agent or combination therewith may be at least 2 fold, and more preferably, 5, 10 or even 25 fold greater than the TI for conventional chemotherapy alone.

## Neuronal disease/disorder

In a particular aspect, the compound or pharmaceutically acceptable salt thereof according to the present application, which increases the level and/or activity of SIRT1 protein, may be used to treat patients suffering from neurodegenerative diseases, and traumatic or mechanical injuries to the central nervous system (CNS) or spinal cord or peripheral nervous system (PNS). Neurodegenerative diseases typically involve a reduction in the mass and volume of the human brain, which may be caused by the atrophy and/or death of brain cells, and such reduction is much more severe than an aging-induced reduction of healthy persons. Neurodegenerative diseases may gradually occur after a long period of normal brain function due to progressive degeneration (e.g., nerve cell dysfunction and death) of a specific brain region. Alternatively, trauma or toxin-associated neurodegenerative diseases may rapidly occur. The actual onset of cerebral degeneration may precede clinical manifestation by several years. Examples of neurodegenerative diseases include, but are not limited to, Alzheimer's disease (AD), Parkinson's disease (PD), Huntington's disease (HD), amyotrophic lateral sclerosis (ALS; Lou Gehrig's disease), diffuse Lewy body disease, chorea-acanthocytosis, primary lateral sclerosis, ocular diseases (ocular neuritis), chemotherapy-induced neuropathies (e.g., from vincristine, paclitaxel and bortezomib), diabetes-induced neuropathies and Friedreich's ataxia. The compound or pharmaceutically acceptable salt thereof according to the present application, which increases the level and/or activity of SIRT1 protein, may be used to treat these diseases and other diseases to be described below.

AD is a CNS disorder that results in memory loss, unusual behavior, personality change, and a decline in

intellectual capacity. These losses are related to the death of specific types of brain cells, and the breakdown of connections and their supporting network (e.g., glial cells). The earliest symptoms include loss of recent memory, judgment error, and personality changes. PD is a CNS disease resulting in uncontrolled body movement, rigidity, tremors, and dyskinesia, and is associated with the death of brain cells in the brain region producing dopamine. ALS (motor neuron disease) is a CNS disease that attacks motor neurons, which are components of CNS connecting the brain with the skeletal muscles.

HD is another neurodegenerative disease that causes uncontrolled movement, loss of intellectual capacity, and emotional illnesses. Tay-Sachs disease and Sandhoff disease are glycolipid storage diseases where GM2 gangliosides and related glycolipid substrates for β-hexosaminidase accumulate in the nervous system and trigger acute neurodegeneration.

It is well known that apoptosis plays a role in AIDS pathogenesis in the immune system. However, HIV-1 also includes a neurological disease, which may be treated with the compound or pharmaceutically acceptable salt thereof according to the present application.

Neuronal loss is a prominent trait of prion diseases, such as Creutzfeldt-Jakob disease in human, BSE in cow, Scrapie disease in sheep and goats, and feline spongiform encephalopathy (FSE) in cats. The compound or pharmaceutically acceptable salt thereof according to the present application, which increases the level and/or activity of SIRT1 protein, may be useful for treating or preventing neuronal loss due to these previous diseases.

In yet another exemplary embodiment, the compound or pharmaceutically acceptable salt thereof according to the present application, which increases the level and/or activity of SIRT1 protein, may be used to treat or prevent any disorder or disease accompanied by axonopathy. Distal axonopathy is a type of peripheral neuropathy that results from some metabolic or toxic diseases of the peripheral nervous system (PNS). It is the most common response of a neuron to metabolic or toxic diseases, which may be caused by metabolic diseases such as diabetes, renal failure, deficiency syndrome such as malnutrition and alcoholism, or the effects of toxins or drugs. The disorders or diseases accompanied by distal axonopathy typically present a symmetrical glove-stocking sensory-motor disease. Deep tendon reflexes and autonomic nervous system (ANS) functions are also low or diminished in affected areas.

Diabetic neuropathy is a neuropathic disease associated with diabetes. Relatively common conditions which may be associated with diabetic neuropathy include third nerve palsy; mononeuropathy; mononeuritis multiplex; diabetic amyotrophy; painful polyneuropathy; autonomic neuropathy; and thoracoabdominal neuropathy.

Peripheral neuropathy is the medical term for damage to nerves of the peripheral nervous system, which may be caused by a nerve disease or from the side effects of systemic illness. While diabetes is the most probable cause, the major causes of peripheral neuropathy include seizures, nutritional deficiencies, and HIV.

In an exemplary embodiment, the compound or pharmaceutically acceptable salt thereof according to the present application, which increases the level and/or activity of SIRT1 protein, may be used to treat or prevent multiple sclerosis (MS) including recurrent MS and monosymptomatic MS, and other demyelinating conditions, such as, for example, chronic inflammatory demyelinating polyneuropathy (CIDP), or symptoms associated therewith.

In yet another exemplary embodiment, the compound or pharmaceutically acceptable salt thereof according to the present application, which increases the level and/or activity of SIRT1 protein, may be used to treat nerve trauma, including trauma due to a disease, injury (including surgical intervention), or environmental trauma (e.g., neurotoxins, alcoholism, etc.).

The compound or pharmaceutically acceptable salt thereof according to the present application, which increases the level and/or activity of SIRT1 protein, may also be useful for preventing, treating and alleviating symptoms of various PNS diseases. The term "peripheral neuropathy" encompasses a wide range of diseases in which nerves outside the brain and spinal cord-peripheral nerves are damaged. Peripheral neuropathy may also be referred to as peripheral neuritis, or when it involves many nerves, a term such as polyneuropathy or polyneuritis may be used.

PNS diseases treatable with the compound or pharmaceutically acceptable salt thereof according to the present application, which increases the level and/or activity of SIRT1 protein, include: diabetes, leprosy, Charcot-Marie Tooth disease, Guillain-Barre syndrome and Brachial Plexus Neuropathies (diseases of the cervical and first thoracic roots, nerve trunks, cords, and peripheral nerve components of the brachial plexus).

In yet another exemplary embodiment, the compound or pharmaceutically acceptable salt thereof according to the present application may be used to treat or prevent a polyglutamine disease. Exemplary polyglutamine diseases include Spinobulbar muscular atrophy (Kennedy disease), Huntington's Disease (HD), Dentatorubral-pallidoluysian atrophy (Haw River syndrome), Spinocerebellar ataxia type 1, Spinocerebellar ataxia type 2, Spinocerebellar ataxia type 3 (Machado-Joseph disease), Spinocerebellar ataxia type 6, Spinocerebellar ataxia type 7, and Spinocerebellar ataxia type 17.

In a particular exemplary embodiment, the present application provides a method of treating cells of the central nervous system to prevent damage in response to a decrease in blood flow to the cell. Typically, the severity of damage to be prevented may vary, generally, according to the degree of reduction in blood flow to cells and the duration of the decrease. In a particular exemplary embodiment, apoptotic or necrotic cell death may be prevented. In an additional

exemplary embodiment, ischemic-mediated damage, such as cytotoxic edema or central nervous system tissue anoxemia, may be prevented. In each exemplary embodiment, cells of the central nervous system may be spinal cells or brain cells.

Another aspect encompasses administrating the compound or pharmaceutically acceptable salt thereof according to the present application to a subject to treat a central nervous system ischemic condition. A number of ischemic conditions of the central nervous system may be treated by the compound or pharmaceutically acceptable salt thereof according to the present application. In a particular exemplary embodiment, the ischemic condition is a stroke that results in any type of ischemic central nervous system damage, such as apoptotic or necrotic cell death, cytotoxic edema or central nervous system tissue anoxia. Stroke may be caused by impact to any area of the brain or any etiological factor generally known to cause the onset of stroke. In another embodiment, the stroke is brain stem stroke. In still another embodiment, the stroke is cerebellar stroke. In yet another embodiment, the stroke is embolic stroke. In yet another embodiment, the stroke is hemorrhagic stroke. In an additional embodiment, the stroke is thrombotic stroke.

In yet another aspect, the compound or pharmaceutically acceptable salt thereof according to the present application may be administered to reduce an infarct size of the ischemic core, following the ischemic condition of the central nervous system. Moreover, the compound or pharmaceutically acceptable salt thereof according to the present application may also be beneficially administered to reduce a size of the ischemic penumbra or transitional zone, following the ischemic condition of the central nervous system.

In a particular exemplary embodiment, combination drug therapy may include a drug or compound for treating or preventing a neurodegenerative disease or secondary condition associated with the conditions thereof. Therefore, the combination drug therapy may include one or more types of SIRT1 activators, and one or more types of anti-neurodegenerative agents.

Blood coagulation disorder

In another aspect, the compound or pharmaceutically acceptable salt thereof according to the present application, which increases the level and/or activity of SIRT1 protein, may be used to treat or prevent a blood coagulation disorder (or hemostatic disorder). The terms "hemostasis," "blood coagulation" and "blood clotting", which are interchangeably used herein, refer to the control of bleeding, including the physiological properties of vasoconstriction and coagulation. The blood coagulation assists in maintaining the integrity of mammalian circulation after injury, inflammation, disease, a congenital defect, dysfunction or other disruptions. In addition, the blood clotting does not only limit bleeding in case of an injury (hemostasis), but may also lead to serious organ damage and death in the context of atherosclerotic diseases by occlusion of an important artery or vein. Therefore, thrombosis is blood clotting at the wrong time and the wrong place.

Accordingly, in some exemplary embodiments of the present application, the present application may provide anticoagulation and antithrombotic treatments which aim at inhibiting the formation of clots to prevent or treat a blood coagulation disorder, such as myocardial infarction, stroke, loss of a limb by peripheral artery disease or pulmonary embolism.

The term "modulating or modulation of hemostasis" and "regulating or regulation of hemostasis", which are interchangeably used herein, include the induction (e.g., stimulation or increase) of hemostasis, as well as the inhibition (e.g., reduction or decrease) of hemostasis.

In one aspect, the present application may provide a method of reducing or inhibiting hemostasis in a subject by administering the compound or pharmaceutically acceptable salt thereof according to the present application, which increases the level and/or activity of SIRT1 protein. The composition, and method or use described in the present application are useful for treating or preventing a hemostatic disorder. The term "hemostatic disorder" used herein includes any disorder or condition characterized by excessive or unwanted coagulation or hemostatic activity, or a hypercoagulable state. The thrombotic disorder includes a disorder or disease accompanied by platelet adhesion and thrombus formation, and may occur as an increased propensity to form thrombi, e.g., an increased number of blood clots, thrombosis at an early age, a familial tendency towards thrombosis, and thrombosis at unusual sites.

In yet another exemplary embodiment, the combination drug therapy may include a drug or compound for treating or preventing a blood coagulation disorder or secondary conditions associated with conditions thereof. Accordingly, the combination drug therapy may include the compound or pharmaceutically acceptable salt thereof according to the present application, which increases the level and/or activity of SIRT1 protein, and one or more types of anticoagulating or antithrombotic agents.

Weight control

In still another aspect, the compound or pharmaceutically acceptable salt thereof according to the present application, which increases the level and/or activity of SIRT1 protein, may be used to treat or prevent weight gain or

obesity in a subject. For example, the compound or pharmaceutically acceptable salt thereof according to the present application, which increases the level and/or activity of SIRT1 protein, may be used, for example, to treat or prevent hereditary obesity, dietary obesity, hormone related obesity, obesity related to the administration of medication, to reduce the weight of a subject, or to reduce or prevent weight gain in a subject. A subject in need of such a treatment may be a subject who is obese, likely to become obese, overweight, or likely to become overweight. Subjects who are likely to become obese or overweight can be identified, for example, based on family history, genetics, diet, activity level, medication intake, or various combinations thereof.

In yet another exemplary embodiment, the compound or pharmaceutically acceptable salt thereof according to the present application, which increases the level and/or activity of SIRT1 protein, may be administered to subjects suffering from a variety of other diseases and conditions that may be treated or prevented by promoting weight loss in the subjects. Such diseases include, for example, high blood pressure, hypertension, high blood cholesterol, dyslipidemia, type 2 diabetes, insulin resistance, glucose intolerance, hyperinsulinemia, coronary heart disease, angina pectoris, congestive heart failure, stroke, gallstones, cholecystitis and cholelithiasis, gout, osteoarthritis, obstructive sleep apnea and respiratory problems, some types of cancer (such as endometrial, breast, prostate, and colon), complications of pregnancy, poor female reproductive health (such as menstrual irregularities, infertility, irregular ovulation), bladder control problems (such as stress incontinence); uric acid nephrolithiasis; psychological disorders (such as depression, eating disorders, distorted body image, and low self-esteem). Finally, patients with AIDS may have lipodystrophy or insulin resistance in response to combination therapy for AIDS.

In yet another exemplary embodiment, the compound or pharmaceutically acceptable salt thereof according to the present application, which increases the level and/or activity of SIRT1 protein, may be used to inhibit lipogenesis or adipocyte differentiation either *in vitro* or *in vivo.* Such methods may be used to treat or prevent obesity.

In a different exemplary embodiment, the compound or pharmaceutically acceptable salt thereof according to the present application, which increases the level and/or activity of SIRT1 protein, may be used to reduce appetite and/or increase satiety, thereby causing weight loss or avoidance of weight gain. A subject in need of such a treatment may be a subject who is overweight, obese or a subject likely to become overweight or obese. The method may include administering a dose of the compound or pharmaceutically acceptable salt thereof to a subject, for example, in the form of a pill daily, every other day or once a week. The dose may be an "appetite reducing dose".

In an exemplary embodiment, the compound or pharmaceutically acceptable salt thereof according to the present application, which increases the level and/or activity of SIRT1 protein, may be administered as combination therapy for treating or preventing weight gain or obesity. For example, one or more types of the compounds or pharmaceutically acceptable salts thereof, which increase the level and/or activity of SIRT1 protein may be administered in combination with one or more anti-obesity agents.

In another exemplary embodiment, the compound or pharmaceutically acceptable salt thereof according to the present application, which increases the level and/or activity of SIRT1 protein, may be administered to reduce drug-induced weight gain. For example, the compound or pharmaceutically acceptable salt thereof according to the present application, which increases the level and/or activity of SIRT1 protein, may be administered as combination therapy with a medication that may stimulate appetite or cause a weight gain, particularly, a weight gain caused by a reason other than water retention.

Metabolic disease/diabetes

In still another aspect, the compound or pharmaceutically acceptable salt thereof according to the present application, which increases the level and/or activity of SIRT1 protein, may be used to treat or prevent a metabolic disease, such as insulin-resistance, a pre-diabetic state, type II diabetes, and/or complications thereof. Administration of the compound or pharmaceutically acceptable salt thereof according to the present application, which increases the level and/or activity of SIRT1 protein, may increase insulin sensitivity and/or lower an insulin level in a subject. A subject in need of such a treatment may be a subject who has insulin resistance or other precursor symptom of type II diabetes, who has type II diabetes, or who is likely to develop any of these conditions. For example, the subject may be a subject having insulin resistance, for example, having high circulation levels of insulin and/or associated conditions, such as hyperlipidemia, dyslipogenesis, hypercholesterolemia, impaired glucose tolerance, high blood glucose sugar level, other manifestations of syndrome X, hypertension, atherosclerosis and lipodystrophy.

In an exemplary embodiment, the compound or pharmaceutically acceptable salt thereof according to the present application, which increases the level and/or activity of SIRT1 protein, may be administered as combination therapy for treating or preventing a metabolic disease. For example, one or more types of the compounds or pharmaceutically acceptable salts thereof according to the present application, which increase the level and/or activity of SIRT1 protein, may be administered in combination with one or more types of antidiabetic agents.

Inflammatory disease

In another aspect, the compound or pharmaceutically acceptable salt thereof according to the present application, which increases the level and/or activity of SIRT1 protein, may be used to treat or prevent an inflammation-associated disorder or disease. The compound or pharmaceutically acceptable salt thereof according to the present application, which increases the level and/or activity of SIRT1 protein, may be administered before the onset of inflammation, at the initiation of inflammation, or after the initiation of inflammation. When used prophylactically, the compound is preferably provided in advance of any inflammatory response or symptom. Administration of the compound may prevent or attenuate an inflammatory response or symptom.

In yet another exemplary embodiment, the compound or pharmaceutically acceptable salt thereof according to the present application, which increases the level and/or activity of SIRT1 protein, may be used to treat or prevent allergic and respiratory conditions, including asthma, bronchitis, pulmonary fibrosis, allergic rhinitis, oxygen toxicity, emphysema, chronic bronchitis, acute respiratory distress syndrome, and any chronic obstructive pulmonary disease (COPD). The compound may be used to treat chronic hepatitis infection, including hepatitis B and hepatitis C.

In addition, the compound or pharmaceutically acceptable salt thereof according to the present application, which increases the level and/or activity of SIRT1 protein, may be used to treat an autoimmune disease, and/or inflammation associated with an autoimmune disease, for example, an organ-tissue autoimmune disease (e.g., Raynaud’s syndrome), ulcerative colitis, Crohn’s disease, oral mucositis, scleroderma, myasthenia gravis, transplant rejection, endotoxin shock, sepsis, psoriasis, eczema, dermatitis, multiple sclerosis, autoimmune thyroiditis, uveitis, systemic lupus erythematosis, Addison’s disease, an autoimmune polyglandular disease (also known as autoimmune polyglandular syndrome) and Grave’s disease, as well as arthritis including rheumatoid arthritis, psoriatic arthritis, and ankylosing spondylitis.

In a particular exemplary embodiment, the compound or pharmaceutically acceptable salt thereof according to the present application, which increases the level and/or activity of SIRT1 protein, may be used alone or in combination with other compounds useful for treating or preventing inflammation.

Flushing

In still another aspect, the compound or pharmaceutically acceptable salt thereof according to the present application, which increases the level and/or activity of SIRT1 protein, may be used to reduce the incidence or severity of flushing and/or hot flashes which are symptoms of a disease. For example, the method of the present application includes the use of the compound or pharmaceutically acceptable salt thereof according to the present application, which increases the level and/or activity of SIRT1 protein, independently or in combination with a different activator to reduce the incidence or severity of flushing and/or hot flashes in cancer patients. In a different exemplary embodiment, the method provides a use of the compound or pharmaceutically acceptable salt thereof according to the present application, which increases the level and/or activity of SIRT1 protein, to reduce the incidence or severity of flushing and/or hot flashes in menopausal and post-menopausal women.

In still another aspect, the compound or pharmaceutically acceptable salt thereof according to the present application, which increases the level and/or activity of SIRT1 protein, may be used as therapy for reducing the incidence or severity of flushing and/or hot flashes, for example, drug-induced flushing, which are side-effects of another drug therapy. In a particular exemplary embodiment, a method for treating and/or preventing drug-induced flushing includes administering a formulation including at least one type of flushing-inducing compound and at least one type of the compound or pharmaceutically acceptable salt thereof according to the present application to a patient in need thereof. In another exemplary embodiment, the method for treating and/or preventing drug-induced flushing includes individually administering one or more types of compounds and one or more types of the compounds or pharmaceutically acceptable salts thereof according to the present application, which induce flushing, and for example, the compound or pharmaceutically acceptable salt thereof according to the present application and a flushing-inducing agent is not formulated with the same composition. When individual formulations are used, the compound or pharmaceutically acceptable salt thereof according to the present application may be used (1) with simultaneous administration of the flushing-inducing agent, (2) with intermittent administration of the flushing-inducing agent, (3) with staggered administration of the flushing-inducing agent, (4) prior to administration of the flushing-inducing agent, (5) after administration of the flushing-inducing agent, and (6) in combination thereof. Examples of the flushing-inducing agents include, for example, niacin, raloxifene, antidepressants, anti-psychotics, chemotherapeutics, calcium channel blockers, and antibiotics.

In a particular exemplary embodiment, the compound or pharmaceutically acceptable salt thereof according to the present application, which increases the level and/or activity of SIRT1 protein, may be used to reduce flushing side effects of a vasodilator or an antilipemic agent (including anticholesteremic agents and lipotropic agents). In an exemplary embodiment, the compound or pharmaceutically acceptable salt thereof according to the present application, which increases the level and/or activity of SIRT1 protein, may be used to reduce flushing associated with the administration of niacin.

In yet another exemplary embodiment, the present application may provide a method of treating and/or preventing hyperlipidemia with reduced flushing side effects. In another representative embodiment, the method may involve the use of the compound or pharmaceutically acceptable salt thereof according to the present application, which increases the level and/or activity of SIRT1 protein, to reduce the flushing side effect of raloxifene. In still another representative embodiment, the method may involve the use of the compound or pharmaceutically acceptable salt thereof according to the present application, which increases the level and/or activity of SIRT1 protein, to reduce the flushing side effect of an antidepressant or antipsychotic agent. For example, the compound or pharmaceutically acceptable salt thereof according to the present application, which increases the level and/or activity of SIRT1 protein, may be used (administered separately or together with) in combination with a serotonin reuptake inhibitor, or a 5HT2 receptor antagonist.

In a particular exemplary embodiment, the compound or pharmaceutically acceptable salt thereof according to the present application, which increases the level and/or activity of SIRT1 protein, may be used as a part of a treatment with serotonin reuptake inhibitor (SRI) to reduce flushing. In yet another representative embodiment, the compound or pharmaceutically acceptable salt thereof according to the present application, which increases the level and/or activity of SIRT1 protein, may be used to reduce the flushing side effect of a chemotherapeutic agent, for example, cyclophosphamide and tamoxifen.

In yet another exemplary embodiment, the compound or pharmaceutically acceptable salt thereof according to the present application, which increases the level and/or activity of SIRT1 protein, may be used to reduce the flushing side effect of a calcium channel blocker, for example, amlodipine.

In yet another exemplary embodiment, the compound or pharmaceutically acceptable salt thereof according to the present application, which increases the level and/or activity of SIRT1 protein, may be used to reduce the flushing side effect of an antibiotic. For example, the compound or pharmaceutically acceptable salt thereof according to the present application, which increases the level and/or activity of SIRT1 protein, may be used in combination with levofloxacin.

Ocular disease

In some aspects of the present application, the present application may provide a method of inhibiting, reducing or otherwise treating visual impairment by administering, to a patient, a therapeutic dose of a SIRT1 activating material or inhibitory material selected from the compound disclosed in the present application or a pharmaceutically acceptable salt thereof, a prodrug and a metabolic derivative thereof.

In a particular aspect of the present application, the visual impairment is caused by damage to the optic nerve or the central nervous system. In a particular exemplary embodiment, the optic nerve damage is caused by high intraocular pressure, for example, by glaucoma. In another particular exemplary embodiment, the optic nerve damage is caused by swelling of the nerve, which is often associated with an infection or an immune (e.g., autoimmune) response such as in optic neuritis.

In a particular aspect of the present application, the visual impairment is caused by retinal damage. In a particular exemplary embodiment, the retinal damage is caused by a disease associated with the blood flow to an eye (e.g., arteriosclerosis or vasculitis). In a particular exemplary embodiment, the retinal damage is caused by macular disruption (e.g., exudative or non-exudative macular degeneration).

Exemplary retinal diseases include Exudative Age Related Macular Degeneration, Nonexudative Age Related Macular Degeneration, Retinal Electronic Prosthesis and RPE Transplantation Age Related Macular Degeneration, Acute Multifocal Placoid Pigment Epitheliopathy, Acute Retinal Necrosis, Best Disease, Branch Retinal Artery Occlusion, Branch Retinal Vein Occlusion, Cancer Associated and Related Autoimmune Retinopathies, Central Retinal Artery Occlusion, Central Retinal Vein Occlusion, Central Serous Chorioretinopathy, Eales Disease, Epimacular Membrane, Lattice Degeneration, Macroaneurysms, Diabetic Macular Edema, Irvine-Gass Macular Edema, Macular Holes, Subretinal Neovascular Membranes, Diffuse Unilateral Subacute Neuroretinitis, Nonpseudophakic Cystoid Macular Edema, Presumed Ocular Histoplasmosis Syndrome, Exudative Retinal Detachment, Postoperative Retinal Detachment, Proliferative Retinal Detachment, Rhegmatogenous Retinal Detachment, Tractional Retinal Detachment, Retinitis Pigmentosa, CMV Retinitis, Retinoblastoma, Retinopathy of Prematurity, Birdshot Retinopathy, Background Diabetic Retinopathy, Proliferative Diabetic Retinopathy, Hemoglobinopathies Retinopathy, Purtscher Retinopathy, Valsalva Retinopathy, Juvenile Retinoschisis, Senile Retinoschisis, Terson Syndrome and White Dot Syndromes.

Other exemplary diseases include ocular bacterial infections (e.g. conjunctivitis, keratitis, tuberculosis, syphilis, gonorrhea), viral infections (e.g. Ocular Herpes Simplex Virus, Varicella Zoster Virus, Cytomegalovirus retinitis, Human Immunodeficiency Virus (HIV)) as well as progressive outer retinal necrosis secondary to HIV or other HIV-associated and other immunodeficiency-associated ocular diseases. In addition, ocular diseases include fungal infections (e.g. Candida choroiditis, histoplasmosis), protozoal infections (e.g. toxoplasmosis) and others such as ocular toxocariasis and sarcoidosis.

In another aspect of the present application, the present application may provide a method of inhibiting, reducing or treating visual impairment in a subject undergoing treatment with a chemotherapeutic drug ((e.g., a neurotoxic drug, a drug that raises intraocular pressure such as a steroid), by administering, to a subject in need of treatment of visual impairment, a therapeutic dose of the SIRT1-activating or inhibiting compound disclosed in the present application.

In still another aspect of the present application, the present application may provide a method of inhibiting, reducing or treating visual impairment in a subject undergoing surgery, including ocular or other surgeries performed in the prone position such as spinal cord surgery, by administering, to the subject in need of the treatment of visual impairment, a therapeutic dose of the SIRT1-activating or inhibiting compound disclosed in the present application. Ocular surgeries include cataract surgery, iridotomy and lens replacements.

In yet another aspect of the present application, the present application may provide treatment including inhibitory and prophylactic treatment of age-related ocular diseases, including cataracts, dry eye, age-related macular degeneration (AMD) and retinal damage by administering, to a subject in need of the treatment of such diseases, a therapeutic dose of the SIRT1-activating or inhibiting compound disclosed in the present application.

In yet another aspect of the present application, the present application may provide prevention or treatment of ocular damage caused by stress, chemical damage or radiation by administering, to a subject in need of the treatment of such damage, a therapeutic dose of the SIRT1-activating or inhibiting compound disclosed in the present application. Radiation or electromagnetic damage to the eye can include that caused by CRT's or exposure to sunlight or UV.

In a particular exemplary embodiment, the combination drug therapy may include drugs or compounds for the treatment or prevention of ocular disorders or secondary conditions associated with these conditions. Accordingly, the combination drug therapy may include one or more types of the SIRT1 activators and one or more types of treatments for the treatment of an ocular disease.

In a particular exemplary embodiment, the SIRT1-activating or inhibiting compound may be administered in combination with therapy for reducing intraocular pressure. In yet another exemplary embodiment, the SIRT1-activating or inhibiting compound may be administered in combination with therapy for treating and/or preventing glaucoma. In yet another exemplary embodiment, the SIRT1-activating or inhibiting compound may be administered in combination with therapy for treating and/or preventing optic neuritis. In a particular exemplary embodiment, the SIRT1-activating or inhibiting compound may be administered in combination with therapy for treating and/or preventing CMV retinopathy. In yet another exemplary embodiment, the SIRT1-activating or inhibiting compound may be administered in combination with therapy for treating and/or preventing multiple sclerosis.

Mitochondria-associated disorder and disease

In a particular exemplary embodiment, the present application may provide a method of treating a disorder or disease that benefits from increased mitochondrial activity using the compound or pharmaceutically acceptable salt thereof according to the present application, or a use of the compound or pharmaceutically acceptable salt thereof for treating the disorder or disease. The method or use involves administering, to a subject in need thereof, a therapeutically effective amount of the compound or pharmaceutically acceptable salt thereof according to the present application. The increased mitochondrial activity refers to an increase in activity of the mitochondria while maintaining the overall numbers of mitochondria (e.g., mitochondrial mass), an increase in the numbers of mitochondria thereby increasing mitochondrial activity (e.g., by stimulating mitochondrial biogenesis), or a combination thereof. In a particular exemplary embodiment, the disorder or disease that benefits from the increased mitochondrial activity includes a disorder or disease associated with mitochondrial dysfunction.

In a particular exemplary embodiment, the method of treating a disorder or disease that benefits from increased mitochondrial activity or the use therefor may include identifying a subject suffering from mitochondrial dysfunction. A method or use for diagnosing mitochondrial dysfunction may involve molecular genetic, pathological and/or biochemical analyses. Diseases and disorders associated with mitochondrial dysfunction include diseases and disorders in which deficits in mitochondrial respiratory chain activity contribute to the development of pathophysiology of such diseases or disorders in a mammal. Diseases or disorders that would benefit from increased mitochondrial activity generally include for example, diseases in which free radical mediated oxidative injury leads to tissue degeneration, diseases in which cells inappropriately undergo apoptosis, and diseases in which cells fail to undergo apoptosis.

In a particular exemplary embodiment, the present application provides a method or use for treating a disorder or disease that benefits from increased mitochondrial activity, which includes administering one or more types of the compounds or pharmaceutically acceptable salts thereof according to the present application to a subject in need of treatment of the disorder or disease in combination with another therapeutic agent, for example, an activator useful for treating mitochondrial dysfunction or an activator useful for reducing symptoms associated with the disorder or disease involving mitochondrial dysfunction.

In an exemplary embodiment, the present application provides a method or use for treating a disorder or disease that benefits from increased mitochondrial activity by administering, to a subject, a therapeutically effective amount of

the compound or pharmaceutically acceptable salt thereof according to the present application. Examples of the disorders or diseases include, for example, neuromuscular disorders (e.g., Friedreich's Ataxia, muscular dystrophy, multiple sclerosis, etc.), disorders of neuronal instability (e.g., seizure disorders, migraine, etc.), developmental delay, neurodegenerative disorders (e.g., Alzheimer's Disease, Parkinson's Disease, amyotrophic lateral sclerosis, etc.), ischemia, renal tubular acidosis, age-related neurodegeneration and cognitive decline, chemotherapy fatigue, age-related or chemotherapy-induced menopause or irregularities of menstrual cycling or ovulation, mitochondrial myopathies, mitochondrial damage (e.g., calcium accumulation, excitotoxicity, nitric oxide exposure, hypoxia, etc.), and mitochondrial deregulation.

Muscular dystrophy frequently refers to a family of diseases involving deterioration of neuromuscular structure and function, often resulting in atrophy of skeletal muscle and myocardial dysfunction, such as Duchenne muscular dystrophy. In a particular exemplary embodiment, the compound or pharmaceutically acceptable salt thereof according to the present application may be used to reduce the rate of decline in muscular function capacity and improving the muscular function status in patients with muscular dystrophy.

In a particular exemplary embodiment, the compound or pharmaceutically acceptable salt thereof according to the present application may be useful for treatment of mitochondrial myopathy. Mitochondrial myopathies range from mild, slowly progressive weakness of the extraocular muscles to severe, fatal infantile myopathies and multisystem encephalomyopathies. Some syndromes have been defined, and some overlap among them. Established syndromes affecting muscle include progressive external ophthalmoplegia, the Kearns-Sayre syndrome (with ophthalmoplegia, pigmentary retinopathy, cardiac conduction defects, cerebellar ataxia, and sensorineural deafness), the MELAS syndrome (mitochondrial encephalomyopathy, lactic acidosis, and stroke-like episodes), the MERFF syndrome (myoclonic epilepsy and ragged red fibers), limb-girdle distribution weakness, and infantile myopathy (benign or severe and fatal).

In a particular exemplary embodiment, the compound or pharmaceutically acceptable salt thereof according to the present application may be useful for treating patients suffering from toxic damage to mitochondria, such as, toxic damage due to calcium accumulation, excitotoxicity, nitric oxide exposure, drug induced toxic damage, or hypoxia.

In a particular exemplary embodiment, the compound or pharmaceutically acceptable salt thereof according to the present application may be useful for treating diseases or disorders associated with mitochondrial deregulation.

The compound or pharmaceutically acceptable salt thereof according to the present application may be used as a CK2-inhibiting compound.

The compound or pharmaceutically acceptable salt thereof according to the present application may be used to prevent or treat a CK2-mediated disease.

In some exemplary embodiments of the present application, CK2-mediated diseases are proliferative diseases and/or inflammatory diseases. In some exemplary embodiments, the proliferative diseases include cancer. The cancer may be breast cancer, prostate cancer, colon cancer, rectal cancer, pancreatic cancer, liver cancer, brain cancer, head and neck cancer, lung cancer (SCLC or NSCLC) or skin cancer (e.g., melanoma). In a specific exemplary embodiment, the cancer is prostate cancer or breast cancer. In a particular exemplary embodiment, the cancer is inflammatory breast cancer. In another exemplary embodiment, the disease is acute or chronic myelogenous leukemia, acute lymphoblastic leukemia, chronic lymphocytic leukemia, Bcr/Abl-positive leukemia, lymphoma or multiple myeloma. In still another exemplary embodiment, the disease is a solid tumor such as an advanced solid tumor. In yet another exemplary embodiment, the disease is Castleman's disease.

In some exemplary embodiments, the disease disclosed herein is an inflammatory disease. Sometimes, the inflammatory disease is glomerulonephritis, multiple sclerosis, systemic lupus erythematosus, rheumatoid arthritis or inflammatory arthritis. In some exemplary embodiments, the compound is used to alleviate inflammatory pain, which is because, in the murine model, CK2 regulates intracellular signal transduction and reduces pain response in mice when injected into the spinal cord.

In some exemplary embodiments, the CK2-mediated disease is selected from the group consisting of neurodegenerative diseases, pain, vascular diseases, pathophysiological diseases of skeletal muscles or bone tissue, protozoasis, parasitosis, and viral diseases.

In some exemplary embodiments, the CK2-mediated disease is a neurodegenerative disease. In some exemplary embodiments, the neurodegenerative diseases include Alzheimer's disease, Parkinson's diseases, memory impairment, brain ischemia, Guam-Parkinson's dementia, chromosome 18 deficiency syndrome, progressive supranuclear palsy, cuff disease or pickle disease.

In some exemplary embodiments, the CK2-mediated disease is a vascular disease. In some exemplary embodiments, the vascular disease is atherosclerosis, layer shear stress or hypoxia.

In some exemplary embodiments, the CK2-mediated disease is a pathophysiological disease of skeletal muscle or bone tissue. The symptoms of the disease include atherosclerosis, layer shear stress, hypoxia and related symptoms. In some exemplary embodiments, the disease includes myocardial cell hypertrophy, insulin signaling injury and bone tissue mineralization.

In some exemplary embodiments, the disease is protozoasis or parasitosis. Particularly, the compound or pharmaceutically acceptable salt thereof according to the present application is useful for treating parasitosis resulting

from Theileria parva, Toxoplasma gondii, Trypanosoma cruzi (Chagas' disease), Leishmania donovani, Herpetomonas muscarum muscarum, Plasmodium falciparum, Trypanosoma brucei and Schistosoma mansoni.

In some exemplary embodiments, the disease is a viral disease. In some exemplary embodiments, the viral disease is human immunodeficiency virus type 1(HIV-1), human papillomavirus, Epstein-Barr virus or herpes simplex virus. In another exemplary embodiment, the viral disease is human papillomavirus, human cytomegalovirus, hepatitis C or B, Borna disease virus, an adenovirus, Coxackie virus, a coronavirus or barisella zoster virus.

A composition of the present application may further include a pharmaceutically acceptable carrier, an excipient or a diluent.

The term "pharmaceutically acceptable carrier" used herein includes a pharmaceutically acceptable material, composition or vehicle, which is associated with delivery or transport of a target chemical such as a liquid or solid filler, a diluent, an additive, a solvent or an encapsulation material from one organ or part of the body to another organ or part of the body. The pharmaceutically acceptable carrier is conventionally used in formulation, includes, but not limited to, saline, sterile water, a Ringer's solution, buffered saline, cyclodextrin, a dextrose solution, a maltodextrin solution, glycerol, ethanol, and liposomes, and if needed, may further include other conventional additives, including an antioxidant, a buffer, etc.. Each carrier should be compatible with other components of the formulation and harmless to patients.

In addition, the composition may be formulated in the form of an injectable form such as a solution, a suspension or an emulsion, a pill, a capsule a granule or a pill, by further adding an excipient, a diluent, a dispersant, a surfactant, a binder or a lubricant. For a pharmaceutically acceptable carrier and formulation, the composition is preferably formulated depending on components using the method disclosed in the Remington's Pharmaceutical Science (Mack Publishing Company, Easton PA).

In the pharmaceutical composition according to the present application, the compound represented by Formula 1 or pharmaceutically acceptable salt thereof may be administered in various oral or parenteral forms in clinical administration, and in formulation, the composition may be prepared using generally used diluents or excipients such as a filler, a bulking agent, a binder, a wetting agent, a disintegrating agent, a surfactant, etc.

Oral formulations include, for example, a tablet, a pill, a hard/soft capsule, a liquid, a suspension, an emulsion, a syrup, a granule, an elixir and a troche, and these formulations contain diluents (e.g., lactose, dextrose, sucrose, mannitol, sorbitol, cellulose and/or glycine), lubricants (e.g, silica, talc, stearic acid and a magnesium or calcium salt thereof, and/or polyethylene glycol) as well as the active ingredient. A tablet may contain binders such as magnesium aluminum silicate, starch paste, gelatin, methylcellulose, sodium carboxymethylcellulose and /or polyvinylpyrrolidone, and in some cases, may contain a dispersant or a boiling mixture, such as starch, agar, alginic acid or a sodium salt thereof, and/or an absorbent, a coloring agent, a flavoring agent and a sweetening agent.

The pharmaceutical composition containing the compound represented by Formula 1 as an active ingredient may be parenterally administered, and parenteral administration is performed by subcutaneous injection, intravenous injection, intramuscular injection or intrathoracic injection.

Here, for parenteral formulations, the compound represented by Formula 1 or a pharmaceutically acceptable salt thereof may be mixed with water in combination with a stabilizer or buffer, thereby preparing a solution or suspension, and may be prepared for administration in the form of an ampoule or vial unit. The composition may be sterilized or contain an adjuvant such as a preservative, a stabilizer, a hydrogenating agent or an emulsifier, a salt for controlling an osmotic pressure and/or a buffer, and other therapeutically acceptable materials, and may be formulated by a conventional method such as mixing, granulation or coating.

Further, a dose of the compound represented by Formula 1 or pharmaceutically acceptable salt thereof of the present application to a human may vary according to a patient's age, body or weight, sex, an administration type, a health condition and the severity of a disease, and on the basis of an adult patient with a body weight of 70 kg, the dose may be generally 0.1 to 1000 mg/day, and preferably, 1 to 500 mg/day. In addition, the compound may be administered once to several times a day at regular intervals according to the judgment of a doctor or pharmacist.

In addition, the present application provides a health functional food for preventing or improving a metabolic disease, which contains the compound represented by Formula 1 or a pharmaceutically acceptable salt thereof as an active ingredient.

Here, the metabolic disease may be any one selected from the group consisting of fatty liver, obesity, diabetes and dyslipidemia.

The health functional food of the present application may be prepared, unlimitedly, in the form of various beverages, gums, teas, confectioneries, vitamin complexes, and health supplements.

In addition, the health functional food of the present application may also be added to health food to improve a metabolic disease such as fatty liver, obesity, diabetes and dyslipidemia, and there is no particular limit to the type of food. Examples of food to which the material is added include meat, sausages, bread, chocolates, candies, snacks, confectioneries, pizza, ramen, other noodles, gums, dairy products including ice cream, all types of soup, beverages, tea, drinks, alcoholic drinks and vitamin complexes, and include all types of health food in the accepted meaning.

A preferable intake of the health functional food of the present application may be suitably selected according

to the condition and body weight of a food consumer, the severity of symptoms, the type of food, and a consumption period. It is preferable for the optimal effect that the health functional food of the present application is preferably provided such that an active ingredient is absorbed at 0.2 to 200 mg/kg per day.

The following Preparation Examples and Experimental Examples are illustrative, and are not intended to limit the scope of the present application in any way.

Preparation Examples

[Preparation Example 1] Synthesis of pentadienoyl compound including para-methylthio group

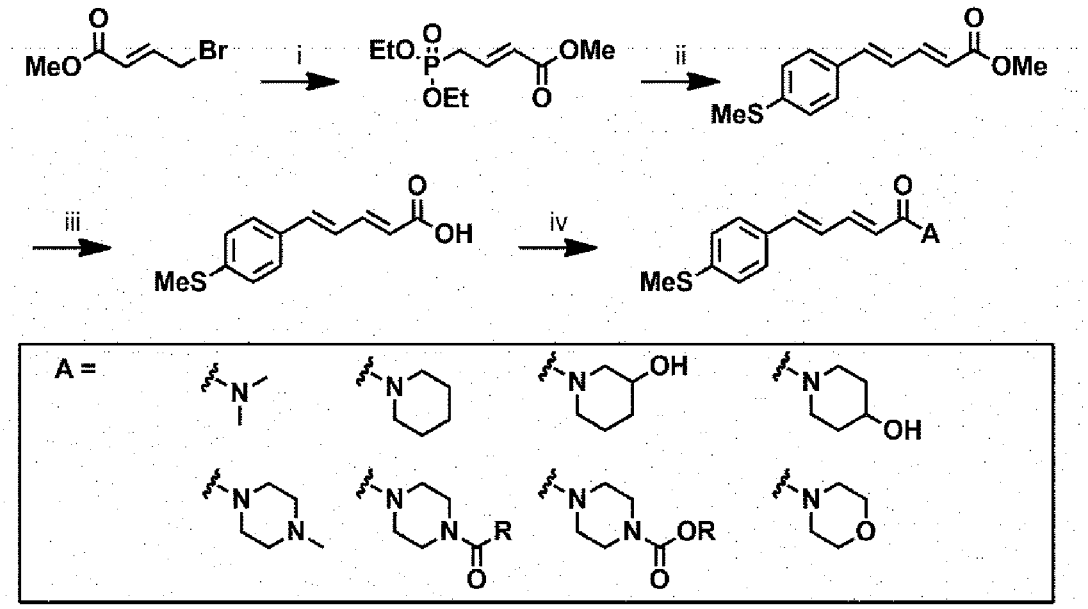


**R = $C_1$ - $C_6$ alkyl (for example, methyl, ethyl, n-propyl, isopropyl, n-butyl, tert-butyl, etc.)**

(1) Synthesis of (2E,4E)-5-(4-(methylthio)phenyl)-1-(piperidine-1-yl)penta-2,4-dien-1-one (YU2014)

Step 1: Synthesis of (E)-methyl 4-(diethoxyphosphoryl)but-2-enoate

Methyl-4-bromocrotonate (85%, 1.40 mL, 12.03 mmol) and triethyl phosphite (2 g, 12.03 mmol) were mixed, and then heated at 120 □ for 3 hours. After the reaction was completed, ethyl bromide as a by-product was removed while heated at 38 □, thereby obtaining (E)-methyl 4-(diethoxyphosphoryl)but-2-enoate (2.6 g, 92%).
$^1H$ NMR (400 MHz, $CDCl_3$) $\delta$ 6.88 (ddd, 1H, J = 15.4, 15.4, 7.8 Hz), 5.96 (dd, 1H, J = 15.6, 4.9 Hz), 4.1 (m, 4H), 3.73 (s, 3H), 2.74 (dd, 2H, 22.9, 7.8 Hz), 1.31 (t, 6H, J = 7.1 Hz).

Step 2: Synthesis of (2E,4E)-methyl 5-(4-(methylthio)phenyl)penta-2,4-dienoate

(E)-methyl 4-(diethoxyphosphoryl)but-2-enoate (173 mg, 0.732 mmol), 4-(methylthio)benzaldehyde (0.097 mL, 0.732 mmol), a molecular sieve (4□, 1.09 g) and anhydrous lithium hydroxide (26.3 mg) were mixed, and moisture was removed in a vacuum. The vacuum was charged with anhydrous argon, and then anhydrous THF (5 mL) was added, followed by heating (refluxing) for 24 hours. After the completion of the reaction, a filtrate obtained by stirring with water (10 mL) and ethyl acetate (10 mL) and filtering through a Celite filter was divided into ethyl acetate and a water layer, and the water layer was extracted with ethyl acetate (15 mL x 2). A combined organic layer was washed with a saturated sodium chloride aqueous solution, dehydrated with anhydrous sodium sulfate and filtrated, followed by vacuum distillation. The residue obtained thereby was purified by silica gel column chromatography (hexane/ethyl acetate = 10/1), thereby obtaining (2E,4E)-methyl 5-(4-(methylthio)phenyl)penta-2,4-dienoate (109 mg, 64%).
$^1H$ NMR (400 MHz, $CDCl_3$) $\delta$ 7.43 (ddd, 1H, J = 15.4, 7.6, 2.7 Hz), 7.37 (d, 2H, J = 8.5 Hz), 7.2 (d, 2H, J = 8.5 Hz), 6.86-6.81 (m, 2H), 5.97 (d, 1H, J = 15.4 Hz), 3.76 (s, 3H), 2.49 (s,3H).

Step 3: Synthesis of (2E,4E)-5-(4-(methylthio)phenyl)penta-2,4-dienoic acid

(2E,4E)-methyl 5-(4-(methylthio)phenyl)penta-2,4-dienoate (109 mg, 0.465 mmol) and 2M LiOH (in water, 34 μL, 0.812 mmol) were mixed, and a mixed solvent (8 mL) of methanol/water (10/1) was added to make the mixture homogeneous, followed by stirring at room temperature for 20 hours. After the reaction was completed, methanol was removed by vacuum distillation, water (3 mL) was added, and the resulting solution was acidified with 6N hydrochloric acid until pH 2 and extracted with ethyl acetate (10 mL x 3). A combined organic layer was dehydrated with anhydrous sodium sulfate, filtered and vacuum distilled, thereby obtaining unpurified (2E,4E)-5-(4-(methylthio)phenyl)penta-2,4-dienoic acid (80.4 mg, 78%).
$^1$H NMR (400 MHz, $CDCl_3$) δ 7.51 (ddd, 1H, J = 15.4, 8.8, 1.7 Hz), 7.38 (d, 2H, J = 8.5 Hz), 7.21 (d, 2H, J = 8.5 Hz), 6.92-6.82 (m, 2H), 5.96 (d, 1H, J = 15.4 Hz), 2.49 (s, 3H).

Step 4: Synthesis of (2E,4E)-5-(4-(methylthio)phenyl)-1-(piperidine-1-yl)penta-2,4-dien-1-one

(2E,4E)-5-(4-(methylthio)phenyl)penta-2,4-dienoic acid (80 mg, 0.363 mmol) and piperidine (92.7 mg, 1.088 mmol) were dissolved in anhydrous tetrahydrofuran (3.6 mL), and solid carbonyl diimidazole (177 mg, 1.088 mmol) was added, followed by stirring at room temperature for 20 hours. After the reaction was completed, tetrahydrofuran was removed by vacuum distillation, a resulting residue was partitioned between ethyl acetate (10 mL) and water (10 mL), followed by separating the organic layer. The water layer was extracted with ethyl acetate (10 mL), combined with the organic layer and washed with brine (10 mL), and a residue obtained by dehydration with anhydrous sodium sulfate, filtration and vacuum distillation was purified by silica gel column chromatography (hexane : ethyl acetate = 1 3), thereby obtaining (2E,4E)-5-(4-(methylthio)phenyl)-1-(piperidine-1-yl)penta-2,4-dien-1-one (48 mg, 46%).
$^1$H NMR (400 MHz, $CDCl_3$) δ 7.41 (dd, 1H, J = 14.6, 10.2 Hz), 7.35 (d, 2H, J = 8.3 Hz), 7.2 (d, 2H, J = 8.3 Hz), 6.9-6.75 (m, 2H), 6.45 (d, 1H, J = 14.6 Hz), 3.68-3.48 (m, 4H), 2.48 (s, 3H), 1.72-1.5 (m, 6H).

(2) Synthesis of (2E,4E)-1-(4-hydroxypiperidine-1-yl)-5-(4-(methylthio)phenyl)penta-2,4-dien-1-one (R-8)

(2E,4E)-1-(4-hydroxypiperidine-1-yl)-5-(4-(methylthio)phenyl)penta-2,4-dien-1-one (28.1 mg, 99%) was synthesized by the same method as described above, except that piperidine-4-ol, instead of piperidine of Step 4, was used in the synthesis process of (2E,4E)-5-(4-(methylthio)phenyl)-1-(piperidine-1-yl)penta-2,4-dien-1-one.
$^1$H NMR (400 MHz, $CDCl_3$) δ 7.41 (dd, 1H, J = 14.6, 10.0 Hz), 7.35 (d, 2H, J = 8.5 Hz), 7.19 (d, 2H, J = 8.5 Hz), 6.89-6.75 (m, 2H), 6.45 (d, 1H, J = 14.6 Hz), 4.24-3.78 (m, 2H), 3.95 (m, 1H), 3.38-3.23 (m, 2H), 2.48 (s, 3H), 1.97-1.86 (m, 2H), 1.62-1.48 (m, 2H).

(3) Synthesis of (2E,4E)-1-(3-hydroxypiperidine-1-yl)-5-(4-(methylthio)phenyl)penta-2,4-dien-1-one (R-7)

(2E,4E)-1-(3-hydroxypiperidine-1-yl)-5-(4-(methylthio)phenyl)penta-2,4-dien-1-one (32.4 mg, 76%) was synthesized by the same method as described above, except that piperidine-3-ol, instead of piperidine of Step 4 was used in the synthesis process of (2E,4E)-5-(4-(methylthio)phenyl)-1-(piperidine-1-yl)penta-2,4-dien-1-one.
$^1$H NMR (400 MHz, $CDCl_3$) δ 7.43 (dd, 1H, J = 15.4, 10.0 Hz), 7.36 (d, 2H, J = 8.5 Hz), 7.2 (d, 2H, J = 8.5 Hz), 6.9-6.76 (m, 2H), 6.45 (d, 1H, J = 15.4 Hz), 3.96-3.38 (m, 3H), 2.48 (s, 3H), 2.2-1.42 (m, 6H).

(4) Synthesis of (2E,4E)-1-(4-methylpiperazine-1-yl)-5-(4-(methylthio)phenyl)penta-2,4-dien-1-one (R-16)

(2E,4E)-1-(4-methylpiperazine-1-yl)-5-(4-(methylthio)phenyl)penta-2,4-dien-1-one (20.1 mg, 49%) was synthesized by the same method as described above, except that N-methylpiperazine, instead of piperidine of Step 4, was used in the synthesis process of (2E,4E)-5-(4-(methylthio)phenyl)-1-(piperidine-1-yl)penta-2,4-dien-1-one.
$^1$H NMR (400 MHz, $CDCl_3$) δ 7.44 (dd, 1H, J = 14.6, 9.2 Hz), 7.35 (d, 2H, J = 8.3 Hz), 7.2 (d, 2H, J = 8.3 Hz), 6.9-6.77 (m, 2H), 6.42 (d, 1H, J = 14.6 Hz), 3.8-3.55 (m, 4H), 2.48 (s, 3H), 2.45-2.4 (m, 4H), 2.31(s, 3H).

(5) Synthesis of tert-butyl 4-((2E,4E)-5-(4-(methylthio)phenyl)penta-2,4-dienoyl)piperazine-1-carboxylate (R-28)

Tert-butyl 4-((2E,4E)-5-(4-(methylthio)phenyl)penta-2,4-dienoyl)piperazine-1-carboxylate (63.9 mg, 47%) was synthesized by the same method as described above, except that tert-butyl piperazine-1-carboxylate, instead of piperidine of Step 4, was used in the synthesis process of (2E,4E)-5-(4-(methylthio)phenyl)-1-(piperidine-1-yl)penta-2,4-dien-1-one.
$^1$H NMR (400 MHz, $CDCl_3$) δ 7.83 (dd, 1H, J = 14.6, 10.5 Hz), 7.43 (d, 2H, J = 8.5 Hz), 7.23 (d, 2H, J = 8.5 Hz), 7.05-6.94 (m, 2H), 6.6 (d, 1H, J = 14.6 Hz), 3.62-3.48 (m, 8H), 2.51 (s, 3H), 1.47 (s, 9H).

(6) Synthesis of (2E,4E)-1-(4-acetylpiperazine-1-yl)-5-(4-(methylthio)phenyl)penta-2,4-dien-1-one (R-17)

(2E,4E)-5-(4-(methylthio)phenyl)penta-2,4-dienoic acid (14.5 mg, 0.066 mmol) and (piperazine-1-yl)ethanone (7 mg, 0.055 mmol) were dissolved in anhydrous methylene chloride (1 mL), and then EDAC (15.2 mg, 0.066 mmol) was added thereto at one time. After stirring at room temperature for 12 hours, methylene chloride was removed by vacuum distillation, and a residue was partitioned between ethyl acetate (10 mL) and water (10 mL), followed by separating the organic layer. The water layer was extracted with ethyl acetate (10 mL), combined with the organic layer and washed with brine (10 mL), and a residue obtained by dehydration with anhydrous sodium sulfate, filtration and vacuum distillation was purified by silica gel column chromatography (hexane : ethyl acetate = 1 3), thereby obtaining (2E,4E)-1-(4-acetylpiperazine-1-yl)-5-(4-(methylthio)phenyl)penta-2,4-dien-1-one (5.8 mg, 28%).
$^1$H NMR (400 MHz, $CDCl_3$) $\delta$ 7.48 (ddd, 1H, J = 2.4, 8.0, 14.6 Hz), 7.36 (d, 2H, J = 8.4 Hz), 7.2 (d, 2H, J = 8.4 Hz), 6.92-6.8 (m, 2H), 6.4 (d, 1H, J = 14.6 Hz), 3.81-3.46 (m, 8H), 2.49 (s, 3H), 2.13 (s,3H).

(7) Synthesis of (2E,4E)-5-(4-(methylthio)phenyl)-1-morpholinopenta-2,4-dien-1-one (R-30)

(2E,4E)-5-(4-(methylthio)phenyl)-1-morpholinopenta-2,4-dien-1-one (67 mg, 70%) was synthesized by the same method as described above, except that morpholine was used, instead of (piperazine-1-yl)ethanone used in the synthesis process of (2E,4E)-1-(4-acetylpiperazine-1-yl)-5-(4-(methylthio)phenyl)penta-2,4-dien-1-one.
$^1$H NMR (400 MHz, $CDCl_3$) $\delta$ 7.46 (ddd, 1H, J = 14.6, 8.0, 2.2 Hz), 7.36 (d, 2H, J = 8.3 Hz), 7.2 (d, 2H, J = 8.3 Hz), 6.9-6.77 (m, 2H), 6.39 (d, 1H, J = 14.6 Hz), 3.8-3.5 (m, 8H), 2.48 (s, 3H).

(8) Synthesis of (2E,4E)-N,N-dimethyl-5-(4-(methylthio)phenyl)penta-2,4-diene amide (R-4)

(2E,4E)-N,N-dimethyl-5-(4-(methylthio)phenyl)penta-2,4-diene amide (38 mg, 99%) was synthesized by the same method as described above, except that dimethylamine (in THF) was used, instead of (piperazine-1-yl)ethanone used in the synthesis process of (2E,4E)-1-(4-acetylpiperazine-1-yl)-5-(4-(methylthio)phenyl)penta-2,4-dien-1-one.
$^1$H NMR (400 MHz, $CDCl_3$) $\delta$ 7.43 (dd, 1H, J = 14.6, 10 Hz), 7.36 (d, 2H, J = 8.4 Hz), 7.2 (d, 2H, J = 8.4 Hz), 6.91-6.77 (m, 2H), 6.44 (d, 1H, J = 14.6 Hz), 3.11 (s, 3H), 3.03 (s, 3H), 2.48 (s, 3H).

**[Preparation Example 2] Synthesis of pentadienoyl compound including para-methylthio group**

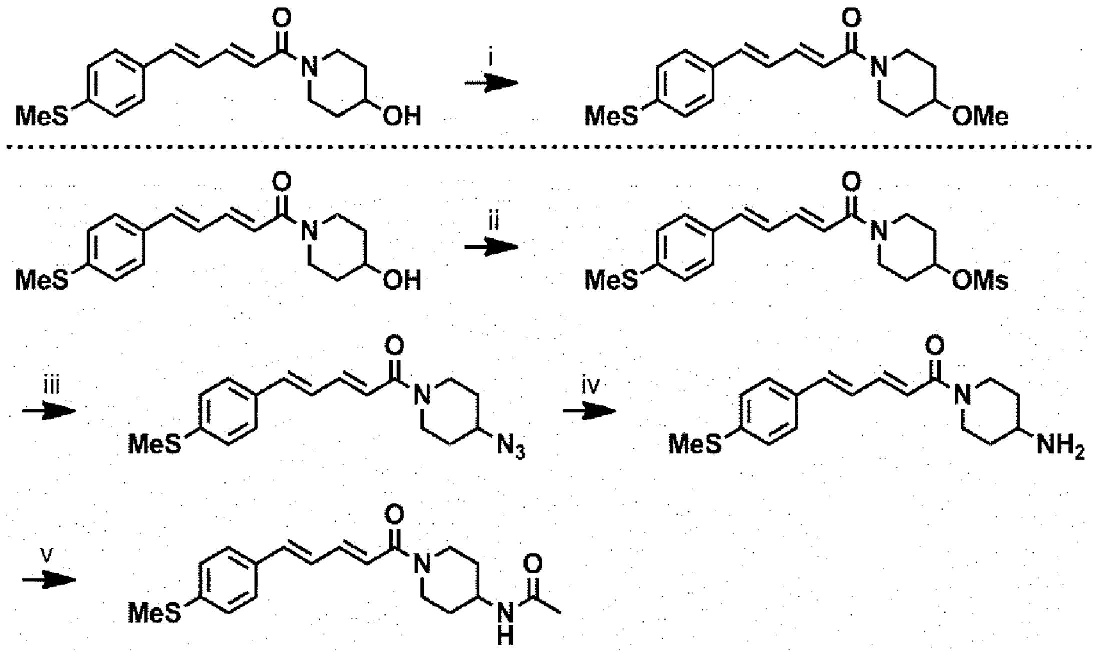


(1) Synthesis of (2E,4E)-1-(4-methoxypiperidine-1-yl)-5-(4-(methylthio)phenyl)penta-2,4-dien-1-one (R-9)

After NaH (60% in mineral oil, 18 mg, 0.45 mmol) was suspended in anhydrous THF (2 mL), (2E,4E)-1-(4-hydroxypiperidine-1-yl)-5-(4-(methylthio)phenyl)penta-2,4-dien-1-one (72.3 mg, 0.24 mmol) dissolved in anhydrous THF (1 mL) was slowly added thereto at -10 □. Following stirring for 10 minutes, excessive methyl iodide (0.1 mL) was slowly added, and then the reaction product was raised to room temperature and stirred for 5 hours. After the reaction was completed, excessive NaH was disrupted by adding water at 0 □. The reaction product was partitioned between ethyl acetate (20 mL) and water (10 mL), followed by separating the organic layer. The water layer was extracted with ethyl acetate (10 mL), combined with the organic layer and washed with brine (10 mL), and a residue obtained by dehydration with anhydrous sodium sulfate, filtration and vacuum distillation was purified by silica gel column chromatography (hexane : ethyl acetate = 1 3), thereby obtaining (2E,4E)-1-(4-methoxypiperidine-1-yl)-5-(4-(methylthio)phenyl)penta-2,4-dien-1-one (26.4 mg, 35%).
$^1$H NMR (400 MHz, $CDCl_3$) δ 7.42 (dd, 1H, J = 14.6, 10 Hz), 7.35 (d, 2H, J = 8.3 Hz), 7.2 (d, 2H, J = 8.3 Hz), 6.9-6.76 (m, 2H), 6.45 (d, 1H, J = 14.6 Hz), 4.1-3.38 (m, 5H), 3.36 (s, 3H), 2.48 (s, 3H), 1.92-1.82 (m, 2H), 1.66-1.57 (m, 2H).

(2) Synthesis of (2E,4E)-1-(4-azidopiperidine-1-yl)-5-(4-(methylthio)phenyl)penta-2,4-dien-1-one (R-12)

Step 1: Synthesis of 1-((2E,4E)-5-(4-(methylthio)phenyl)penta-2,4-dienoyl)piperidine-4-yl methane sulfonate

(2E,4E)-1-(4-hydroxypiperidine-1-yl)-5-(4-(methylthio)phenyl)penta-2,4-dien-1-one (53 mg, 0.175 mmol) and triethylamine (21.25 mg, 0.21 mmol) were dissolved in anhydrous methylene chloride (2 mL) and cooled to -20 □, followed by adding methyl chloride (20 mg, 0.175 mmol). At the same temperature, the resulting mixture was stirred for 1 hour, and raised to room temperature. After confirming that the reaction was completed, the methylene chloride was removed by vacuum distillation, and a residue was partitioned between ethyl acetate (10 mL) and water (10 mL), followed by separating the organic layer. The water layer was extracted with ethyl acetate (10 mL), combined with the organic layer and washed with brine (10 mL), and a residue obtained by dehydration with anhydrous sodium sulfate, filtration and vacuum distillation was purified by silica gel column chromatography (hexane : ethyl acetate = 1 : 3), thereby obtaining 1-((2E,4E)-5-(4-(methylthio)phenyl)penta-2,4-dienoyl)piperidine-4-yl methane sulfonate (41.7 mg, 63%).
$^1$H NMR (400 MHz, $CDCl_3$) δ 7.44 (dd, 1H, J = 14.6, 8.8 Hz), 7.36 (d, 2H, J = 8.4 Hz), 7.2 (d, 2H, J = 8.4 Hz), 6.9-6.78 (m, 2H), 6.43 (d, 1H, J = 14.6 Hz), 4.96 (m, 1H), 4-3.4 (m, 4H), 3.05 (s, 3H), 2.48 (s, 3H), 2.08-1.8 (m, 4H).

Step 2: Synthesis of (2E,4E)-1-(4-azidopiperidine-1-yl)-5-(4-(methylthio)phenyl)penta-2,4-dien-1-one

1-((2E,4E)-5-(4-(methylthio)phenyl)penta-2,4-dienoyl)piperidine-4-yl methane sulfonate (99 mg, 0.26 mmol) and sodium azide (20.3 mg, 0.312 mmol) were dissolved in anhydrous dimethyl formamide (3 mL), and thermally stirred at 90 □ for 20 hours. After the reaction was completed, the cooled reaction product was diluted with ethyl acetate (30 mL), and continuously treated with saturated sodium chloride (5 mL x 5) to remove DMF. A residue obtained by dehydration of the organic layer with anhydrous sodium sulfate, filtration and vacuum distillation was purified by silica gel column chromatography (hexane : ethyl acetate = 1 : 3), thereby obtaining (2E,4E)-1-(4-azidopiperidine-1-yl)-5-(4-(methylthio)phenyl)penta-2,4-dien-1-one (85 mg, 100%).
$^1$H NMR (400 MHz, $CDCl_3$) δ 7.43 (dd, 1H, J = 14.6, 10.0 Hz), 7.36 (d, 2H, J = 8.4 Hz), 7.2 (d, 2H, J = 8.4 Hz), 6.89-6.77 (m, 2H), 6.43 (d, 1H, J = 14.6 Hz), 4.2-3.74 (m, 2H), 3.7 (m, 1H), 3.34-3.1 (m, 2H), 2.48 (s, 3H), 1.99-1.88 (m, 2H), 1.69-1.57 (m, 2H).

(3) Synthesis of (2E,4E)-1-(4-aminopiperidine-1-yl)-5-(4-(methylthio)phenyl)penta-2,4-dien-1-one (R-10)

(2E,4E)-1-(4-azidopiperidine-1-yl)-5-(4-(methylthio)phenyl)penta-2,4-dien-1-one (73.2 mg, 0.223 mmol), triphenylphosphine (117 mg, 0.446 mmol) and water (0.020 mL) were dissolved in THF (3 mL), and refluxed for 16 hours while heated. After the reaction was completed, THF was removed by vacuum distillation, diethyl ether (10 mL) and a 0.1 N hydrochloric acid aqueous solution (5 mL) were added to the resulting product to separate a water layer, followed by removing excessive triphenylphosphine and triphenylphosphine oxide.
The water layer was treated with solid $NaHCO_3$ for neutralization, and extracted with methylene chloride (10 mL x 2). The combined organic layer was dehydrated with anhydrous sodium sulfate, filtered, and subjected to vacuum distillation, thereby obtaining (2E,4E)-1-(4-aminopiperidine-1-yl)-5-(4-(methylthio)phenyl)penta-2,4-dien-1-one (43.8 mg, 67%).
$^1$H NMR (400 MHz, $CDCl_3$) δ 7.42 (dd, 1H, J = 14.6, 10.0 Hz), 7.35 (d, 2H, J = 8.5 Hz), 7.2 (d, 2H, J = 8.5 Hz), 6.9-6.75 (m, 2H), 6.45 (d, 1H, J = 14.6 Hz), 4.55 (m, 1H), 4.0 (m, 1H), 3.22-2.72 (m, 3H), 2.48 (s, 3H), 1.94-1.82 (m, 2H), 1.54-1.38 (m, 2H).

(4) Synthesis of N-(1-((2E,4E)-5-(4-(methylthio)phenyl)penta-2,4-dienoyl)piperidine-4-yl)acetamide (R-11)

(2E,4E)-1-(4-aminopiperidine-1-yl)-5-(4-(methylthio)phenyl)penta-2,4-diene-1-one (27 mg, 0.089 mmol) and triethylamine (22.5 mg, 0.223 mmol) were dissolved in anhydrous methylene chloride (2 mL) and cooled to 0 □, followed by adding anhydrous citric acid (11 mg, 0.107 mmol). At the same temperature, the resulting mixture was stirred for 30 minutes, and raised to room temperature. After the reaction was completed, a saturated $NaHCO_3$ aqueous solution (5 mL) was added, and then extraction was performed with methylene chloride (5 mL x 2). A residue obtained by dehydrating the combined organic layer with anhydrous sodium sulfate, filtering and vacuum distilling the same was purified by silica gel column chromatography (chloroform : methanol = 8 : 1), thereby obtaining N-(1-((2E,4E)-5-(4-(methylthio)phenyl)penta-2,4-dienoyl)piperidine-4-yl)acetamide (27 mg, 88%).
$^1H$ NMR (400 MHz, $CDCl_3$) δ 7.42 (dd, 1H, J = 14.6, 10 Hz), 7.35 (d, 2H, J = 8.5 Hz), 7.2 (d, 2H, J = 8.5 Hz), 6.89-6.76 (m, 2H), 6.43 (d, 1H, J = 14.6 Hz), 5.43 (m, 1H), 4.63 (m, 1H), 4.03 (m, 2H), 3.26-3.1 (m, 1H), 2.9-2.73 (m, 1H), 2.48 (s, 3H), 2.14-1.9 (m, 6H).

**[Preparation Example 3] Synthesis of pentadienoyl compound including para-methylthio group**

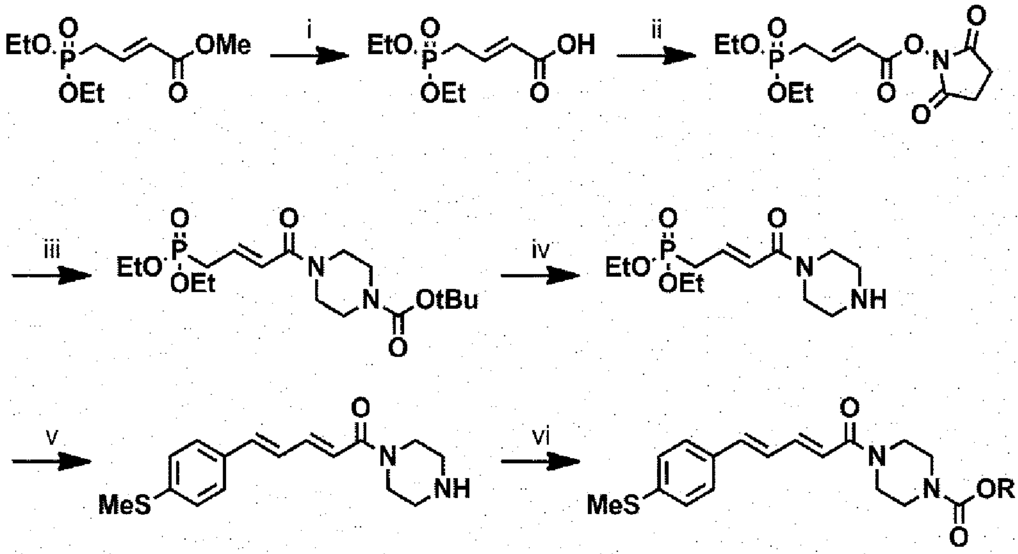


**R = $C_1$ - $C_6$ alkyl (for example, methyl, ethyl, n-propyl, isopropyl, n-butyl, tert-butyl, etc)**

(1) Synthesis of (2E,4E)-5-(4-(methylthio)phenyl)-1-(piperazine-1-yl)penta-2,4-dien-1-one (R-14)

Step 1: Synthesis of (E)-4-(diethoxyphosphoryl)but-2-enoic acid

Methanol/water (10/1, 3.3 mL) was slowly added to a mixture of (E)-methyl 4-(diethoxyphosphoryl)but-2-enoate (2.16 g, 9.14 mmol) and a LiOH aqueous solution (2M in water, 8 mL, 16 mmol) and made homogeneous. The resulting mixture was stirred at room temperature for 40 minutes, cooled to 0 □, and acidified to pH 3 by adding a 6N HCl aqueous solution. After the reaction mixture was vacuum distilled to remove methanol, the resulting product was treated with ethyl acetate (50 mL) and subjected to salting out with a solid salt to separate the organic layer, followed by extracting the water layer with ethyl acetate (25 mL x 2).

The combined organic layer was dehydrated with anhydrous sodium sulfate, filtered, and vacuum-distilled, thereby obtaining (E)-4-(diethoxyphosphoryl)but-2-enoic acid (1.8 g, 89%).
$^1H$ NMR (400 MHz, $CDCl_3$) δ 6.93 (ddd, 1H, J = 15.4, 15.4, 7.8 Hz), 5.95 (dd, 1H, J = 15.4, 4.9 Hz), 4.12 (m,4H), 2.78 (dd, 2H, J = 22.9, 7.8 Hz), 1.31 (t, 6H, J = 7.1 Hz).

Step 2: Synthesis of (E)-2,5-dioxopyrrolidine-1-yl 4-(diethoxyphosphoryl)but-2-enoate

(E)-4-(diethoxyphosphoryl)but-2-enoic acid (718 mg, 3.23 mmol) and N-hydroxysuccinimide (372 mg, 3.23 mmol) were dissolved in anhydrous THF (10 mL) and cooled to 0 □, and then dicyclohexyldiimide (in THF (3 mL), 666

mg, 3.23 mmol) was slowly added. The resulting mixture was stirred at room temperature for 24 hours, and after completion of the reaction, filtered. A filter cake was rinsed with THF (5 mL), and ethyl acetate (15 mL) was added to the filter solution. After cooling to 4 □ and being allowed to stand for about 20 minutes, the resulting mixture was filtered again to obtain (E)-2,5-dioxopyrrolidine-1-yl 4-(diethoxyphosphoryl)but-2-enoate (1.03 g, 100%).
$^1$H NMR (400 MHz, $CDCl_3$) δ 7.15 (ddd, 1H, J = 15.4, 15.4, 7.8 Hz), 6.15 (dd, 1H, J = 15.6, 4.9 Hz), 4.12 (m, 4H), 2.9-2.78 (m, 6H), 1.33 (t, 6H, J = 7.1 Hz).

Step 3: Synthesis of (E)-tert-butyl 4-(4-(diethoxyphosphoryl)but-2-enoyl)piperazine-1-carboxylate

After (E)-2,5-dioxopyrrolidine-1-yl 4-(diethoxyphosphoryl)but-2-enoate (687 mg, 2.15 mmol) was dissolved in anhydrous THF/DCM (1/1, 6 mL), a mono-Boc-piperazine (400 mg, 2.15 mmol) solution in THF/DCM (1/1, 4 mL) was slowly added and stirred at room temperature for 24 hours. After the completion of the reaction, the solvent was completely removed by vacuum distillation, the residue was partitioned between ethyl acetate (50 mL) and water (15 mL), the organic layer was separated by salting out using a salt, and the water layer was extracted with ethyl acetate (10 mL x 2). The combined organic layer was dehydrated with anhydrous sodium sulfate, filtered and vacuum-distilled, and then the residue was purified by silica gel column chromatography (hexane : ethyl acetate = 1 :3) to obtain (E)-tert-butyl 4-(4-(diethoxyphosphoryl)but-2-enoyl)piperazine-1-carboxylate (909 mg, 100%).
$^1$H NMR (400 MHz, $CDCl_3$) δ 6.75 (ddd, 1H, J = 15.4, 15.4, 7.8 Hz), 6.4 (dd, 1H, J = 15.4, 4.8 Hz), 4.1 (m, 4H), 3.7-3.34 (m, 8H), 2.74 (dd, 2H, J = 22.0, 7.8 Hz), 1.44 (s, 9H), 1.30 (t, 6H, J = 7.1 Hz).

Step 4: Synthesis of (E)-diethyl (4-oxo-4-(piperazine-1-yl)but-2-en-1-yl)phosphonate

4-(4-(diethoxyphosphoryl)but-2-enoyl)piperazine-1-carboxylate (908 mg, 2.325 mmol) was dissolved in 5% trifluoroacetic acid (in methylene chloride, 20 mL), and stirred at room temperature for 16 hours. After the reaction, all of the volatile materials were removed by vacuum distillation. The residue was dissolved in methylene chloride (5 mL), a sufficient amount of a basic resin (ammonium hydroxide form) was added thereto, and the resulting mixture was stirred for 1 hour or more, filtered and vacuum-distilled, thereby obtaining (E)-diethyl (4-oxo-4-(piperazine-1-yl)but-2-en-1-yl)phosphonate (472 mg, 70%).
$^1$H NMR (400 MHz, $CDCl_3$) δ 6.73 (ddd, 1H, J = 15.6, 15.6, 7.8 Hz), 6.4 (dd, 1H, J = 15.6, 5.2 Hz), 4.1 (m, 4H), 3.7-3.4 (m, 4H), 2.87-2.81 (m, 4H), 2.72 (ddd, 2H, J = 22.0, 7.8, 1.2 Hz), 1.30 (t, 6H, J = 7.1 Hz).

Step 5: Synthesis of (2E,4E)-5-(4-(methylthio)phenyl)-1-(piperazine-1-yl)penta-2,4-dien-1-one

After (E)-diethyl (4-oxo-4-(piperazine-1-yl)but-2-en-1-yl)phosphonate (100 mg, 0.344 mmol) was dissolved in anhydrous methylene chloride/dimethylformamide (3/1, 3 mL), p-methylthiobenzaldehyde (47 mg, 0.31 mmol) was added and then cooled to 0 □. Potassium tert-butoxide (1M in THF, 0.34 mL) was slowly added. The resulting mixture was stirred for 6 hours at the same temperature, and water (2 mL) was added to stop the reaction. The volatile solvent was completely removed by vacuum distillation, the residue was partitioned between ethyl acetate (20 mL) and water (5 mL), followed by separating the organic layer. The water layer was extracted with ethyl acetate (10 mL x 2), and the combined organic layer was dehydrated with anhydrous sodium sulfate, filtered and vacuum-distilled, and the residue was purified by silica gel column chromatography (chloroform : methanol = 8 : 1) to obtain (2E,4E)-5-(4-(methylthio)phenyl)-1-(piperazine-1-yl)penta-2,4-dien-1-one (99 mg, 47%).
$^1$H NMR (400 MHz, $CDCl_3$) δ7.44 (ddd, 1H, J = 14.6, 9.2, 1.0 Hz), 7.35 (d, 2H, J = 8.5 Hz), 7.2 (d, 2H, J = 8.5 Hz), 6.91-6.77 (m, 2H), 6.43 (d, 1H, J = 14.6 Hz), 3.8-3.5 (m, 4H), 2.94-2.85 (m, 4H), 2.48 (s, 3H).

(2) Synthesis of methyl 4-((2E,4E)-5-(4-(methylthio)phenyl)penta-2,4-dienoyl)piperazine-1-carboxylate (R-21)

After methyl chloroformate obtained using triphosgene and methanol was dissolved in hexane (1 mL), anhydrous sodium carbonate was added and a catalytic amount of triethylamine was added, and then a hexane solution (0.5 mL) of (2E,4E)-5-(4-(methylthio)phenyl)-1-(piperazine-1-yl)penta-2,4-dien-1-one (5.80 mg, 0.020 mmol) was added. After completion of the reaction, water (1 mL) was added, the volatile material was removed by vacuum distillation, and the residue was partitioned between ethyl acetate (5 mL) and water (1 mL), followed by separating the organic layer. The water layer was extracted with ethyl acetate (5 mL x 2), the combined organic layer was dehydrated with anhydrous sodium sulfate, filtered and vacuum-distilled, and the residue was purified by silica gel column chromatography (hexane : ethyl acetate = 1 1), thereby obtaining methyl 4-((2E,4E)-5-(4-(methylthio)phenyl)penta-2,4-dienoyl)piperazine-1-carboxylate (4 mg, 57%).
$^1$H NMR (400 MHz, $CDCl_3$) δ 7.47 (ddd, 1H, J = 14.6, 8.3, 2.0 Hz), 7.36 (d, 2H, J = 8.5 Hz), 7.2 (d, 2H, J = 8.5 Hz), 6.9-6.79 (m, 2H), 6.4 (d, 1H, J = 14.6 Hz), 3.72 (s, 3H), 3.71-3.44 (m, 8H), 2.48 (s, 3H).

(3) Synthesis of ethyl 4-((2E,4E)-5-(4-(methylthio)phenyl)penta-2,4-dienoyl)piperazine-1-carboxylate (R-24)

Ethyl 4-((2E,4E)-5-(4-(methylthio)phenyl)penta-2,4-dienoyl)piperazine-1-carboxylate (2.0 mg, 32%) was synthesized by the same method as described above, except that ethanol, instead of methanol, was used in the synthesis process of methyl 4-((2E,4E)-5-(4-(methylthio)phenyl)penta-2,4-dienoyl)piperazine-1-carboxylate.
$^1$H NMR (400 MHz, $CDCl_3$) δ 7.46 (ddd, 1H, J = 14.6, 8.8, 2.0 Hz), 7.36 (d, 2H, J = 8.4 Hz), 7.2 (d, 2H, J = 8.4 Hz), 6.91-6.78 (m, 2H), 6.4 (d, 1H, J = 14.6 Hz), 4.16 (q, 2H), 3.8-3.42 (m, 8H), 2.49 (s, 3H), 1.27 (t, 3H, J = 7.1 Hz).

(4) Synthesis of isopropyl 4-((2E,4E)-5-(4-(methylthio)phenyl)penta-2,4-dienoyl)piperazine-1-carboxylate (R-27)

Isopropyl 4-((2E,4E)-5-(4-(methylthio)phenyl)penta-2,4-dienoyl)piperazine-1-carboxylate (2.5 mg, 35%) was synthesized by the same method as described above, except that isopropanol, instead of methanol, was used in the synthesis process of methyl 4-((2E,4E)-5-(4-(methylthio)phenyl)penta-2,4-dienoyl)piperazine-1-carboxylate.
$^1$H NMR (400 MHz, $CDCl_3$) δ 7.46 (ddd, 1H, J = 14.6, 8.5, 1.7 Hz), 7.36 (d, 2H, J = 8.3 Hz), 7.2 (d, 2H, J = 8.3 Hz), 6.9-6.78 (m, 2H), 6.41 (d, 1H, J = 14.6 Hz), 4.94 (m, 1H), 3.81-3.4 (m, 8H), 2.48 (s, 3H), 1.25 (d, 6H, J = 6.4 Hz).

**[Preparation Example 4] Synthesis of pentadienoyl compound including para-methoxy group**

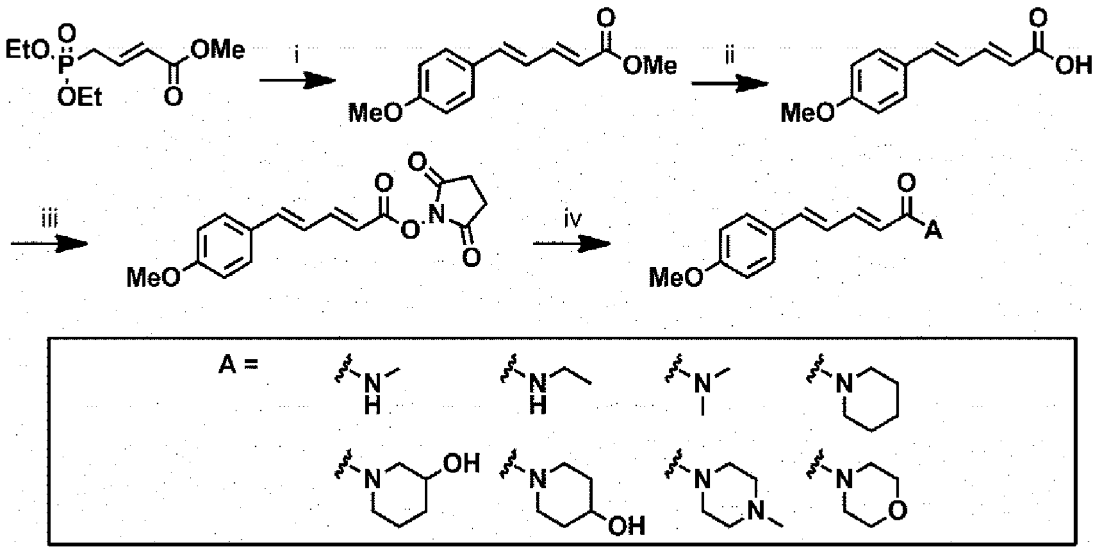


(1) Synthesis of (2E,4E)-5-(4-methoxyphenyl)-1-(piperidine-1-yl)penta-2,4-dien-1-one

Step 1: Synthesis of (2E,4E)-methyl 5-(4-methoxyphenyl)penta-2,4-dienoate

(2E,4E)-methyl 5-(4-methoxyphenyl)penta-2,4-dienoate (675 mg, 73%) was synthesized by the same method as described above, except that p-methoxybenzaldehyde, instead of 4-(methylthio)benzaldehyde, was used in the synthesis process of the (2E,4E)-methyl 5-(4-(methylthio)phenyl)penta-2,4-dienoate of Step 2 included in the synthesis process of (1) (2E,4E)-5-(4-(methylthio)phenyl)-1-(piperidine-1-yl)penta-2,4-dien-1-one described in Preparation Example 1.
$^1$H NMR (400 MHz, DMSO-$d_6$) δ 7.51 (d, 2H, J = 8.8 Hz), 7.4 (dd, 1H, J = 15.1, 10.2 Hz), 7.08-6.97 (m, 2H), 6.96 (d, 2H, J = 8.8 Hz), 6.03 (d, 1H, J = 15.1 Hz), 3.78 (s, 3H), 3.67 (s,3H).

Step 2: Synthesis of (2E,4E)-5-(4-methoxyphenyl)penta-2,4-dienoic acid

(2E,4E)-5-(4-methoxyphenyl)penta-2,4-dienoic acid (622 mg, 51%) was synthesized by the same method as described in Step 3 included in the synthesis process of (1) (2E,4E)-5-(4-(methylthio)phenyl)-1-(piperidine-1-yl)penta-2,4-dien-1-one in Preparation Example 1.
$^1$H NMR (400 MHz, $CDCl_3$) δ 7.55 (dd, 1H, J = 16.4, 8.8 Hz), 7.41 (d, 2H, J = 8.8 Hz), 6.88 (d, 2H, J = 8.8 Hz), 6.82-6.72 (m, 2H), 6.61 (d, 1H, J = 16.4 Hz), 3.82 (s, 3H).

Step 3: Synthesis of (2E,4E)-2,5-dioxopyrrolidine-1-yl 5-(4-methoxyphenyl)penta-2,4-dienoate (17)

(2E,4E)-5-(4-methoxyphenyl)penta-2,4-dienoic acid (622 mg, 3.05 mmol) and N-hydroxysuccinimide (421 mg, 3.66 mmol) were dissolved in anhydrous methylene chloride (30 mL), and EDAC (877 mg, 4.58 mmol) was added once at room temperature. The reaction product was stirred at room temperature for 20 hours, the volatile material was removed by vacuum distillation, the residue was partitioned between ethyl acetate (20 mL) and water (10 mL), and then the organic layer was separated. The water layer was extracted with ethyl acetate (10 mL x 2), the combined organic layer was rinsed with water (5 mL) and saturated brine (10 mL), dehydrated with anhydrous sodium sulfate, filtered, and subjected to vacuum distillation, thereby obtaining (2E,4E)-2,5-dioxopyrrolidine-1-yl 5-(4-methoxyphenyl)penta-2,4-dienoate(737 mg, 80%). The synthesized NHS ester was directly used in a subsequent reaction without a separate purification process.
$^1$H NMR (400 MHz, DMSO-$d_6$) δ 7.65 (dd, 1H, J = 15.4, 11.0 Hz), 7.43 (d, 2H, J = 8.8 Hz), 6.89 (d, 2H, J = 8.8 Hz), 6.82-6.7 (m, 2H), 6.06 (d, 1H, J = 15.4 Hz), 3.88 (s, 3H), 2.95-2.77 (m, 4H).

Step 4: Synthesis of (2E,4E)-5-(4-methoxyphenyl)-1-(piperidine-1-yl)penta-2,4-dien-1-one

(2E,4E)-2,5-dioxopyrrolidine-1-yl 5-(4-methoxyphenyl)penta-2,4-dienoate (123 mg, 0.409 mmol) was dissolved in anhydrous methylene chloride (5 mL), and then piperidine (104 mg, 1.23 mmol) was added. After completion of the reaction, the volatile material was removed by vacuum distillation, and then the residue was purified by silica gel column chromatography(hexane : ethyl acetate = 3 : 1), thereby obtaining (2E,4E)-5-(4-methoxyphenyl)-1-(piperidine-1-yl)penta-2,4-dien-1-one(54.3 mg, 49%).
$^1$H NMR (400 MHz, $CDCl_3$) δ 7.43 (dd, 1H, J = 14.6, 10 Hz), 7.38 (d, 2H, J = 8.8 Hz), 6.86 (d, 2H, J = 8.8 Hz), 6.79-6.76 (m, 2H), 6.42 (d, 1H, J = 14.6 Hz), 3.81 (s, 3H), 3.66-3.48 (m, 4H), 1.7-1.48 (m, 6H).

(2) Synthesis of (2E,4E)-1-(4-hydroxypiperidine-1-yl)-5-(4-methoxyphenyl)penta-2,4-dien-1-one (R-6)

(2E,4E)-1-(4-hydroxypiperidine-1-yl)-5-(4-methoxyphenyl)penta-2,4-dien-1-one (21.6 mg, 35%) was synthesized by the same method as described above, except that 4-hydroxypiperidine, instead of piperidine, was used in the synthesis process of (2E,4E)-5-(4-methoxyphenyl)-1-(piperidine-1-yl)penta-2,4-dien-1-one.
$^1$H NMR (400 MHz, $CDCl_3$) δ 7.46 (dd, 1H, J = 14.6, 10 Hz), 7.39 (d, 2H, J = 8.8 Hz), 6.87 (d, 2H, J = 8.8 Hz), 6.84-6.72 (m, 2H), 6.43 (d, 1H, J = 14.6 Hz), 4.25-3.33 (m, 5H), 3.81 (s, 3H), 1.98-1.87 (m, 2H), 1.68-1.46 (m, 2H).

(3) Synthesis of (2E,4E)-1-(3-hydroxypiperidine-1-yl)-5-(4-methoxyphenyl)penta-2,4-dien-1-one (R-5)

(2E,4E)-1-(3 -hydroxypiperidine-1-yl)-5-(4-methoxyphenyl)penta-2,4-dien-1-one (15.7 mg, 24%) was synthesized by the same method as described above, except that 3-hydroxypiperidine, instead of piperidine, was used in the synthesis process of (2E,4E)-5-(4-methoxyphenyl)-1-(piperidine-1-yl)penta-2,4-dien-1-one
$^1$H NMR (400 MHz, $CDCl_3$) δ 7.43 (dd, 1H, J = 14.4, 10.0 Hz), 7.38 (d, 2H, J = 8.8 Hz), 6.87 (d, 2H, J = 8.8 Hz), 6.82-6.72 (m, 2H), 6.43 (d, 1H, J = 14.4 Hz), 4.02-3.29 (m, 5H), 3.81 (s, 3H), 2-1.44 (m, 4H).

(4) Synthesis of (2E,4E)-5-(4-methoxyphenyl)-1-(4-methylpiperazine-1-yl)penta-2,4-dien-1-one (R-15)

(2E,4E)-5-(4-methoxyphenyl)-1-(4-methylpiperazine-1-yl)penta-2,4-dien-1-one (23.3 mg, 33%) was synthesized by the same method as described above, except that N-methylpiperazine, instead of piperidine, was used in the synthesis process of (2E,4E)-5-(4-methoxyphenyl)-1-(piperidine-1-yl)penta-2,4-dien-1-one.
$^1$H NMR (400 MHz, $CDCl_3$) δ 7.45 (dd, 1H, J = 14.6, 9.8 Hz), 7.38 (d, 2H, J = 8.8 Hz), 6.86 (d, 2H, J = 8.8 Hz), 6.81-6.72 (m, 2H), 6.38 (d, 1H, J = 14.6 Hz), 3.81 (s, 3H), 3.8-3.55 (m, 4H), 2.47-2.38 (m, 4H), 2.31 (s, 3H).

(5) Synthesis of (2E,4E)-5-(4-methoxyphenyl)-1-morpholinopenta-2,4-dien-1-one (R-29)

(2E,4E)-5-(4-methoxyphenyl)-1-morpholinopenta-2,4-dien-1-one (8.4 mg, 27%) was synthesized by the same method as described above, except that morpholine, instead of piperidine, was used in the synthesis process of (2E,4E)-5-(4-methoxyphenyl)-1-(piperidine-1-yl)penta-2,4-dien-1-one.
$^1$H NMR (400 MHz, $CDCl_3$) δ 7.47 (dd, 1H, J = 14.6, 10.2 Hz), 7.39 (d, 2H, J = 8.8 Hz), 6.87 (d, 2H, J = 8.8 Hz), 6.83-6.72 (m, 2H), 6.35 (d, 1H, J = 14.6 Hz), 3.81 (s, 3H), 3.8-3.5 (m, 8H).

(6) Synthesis of (2E,4E)-5-(4-methoxyphenyl)-N-methylpenta-2,4-dieneamide (R-1)

(2E,4E)-5-(4-methoxyphenyl)-N-methylpenta-2,4-dieneamide (12.4 mg, 33%) was synthesized by the same method as described above, except that methylamine, instead of piperidine, was used in the synthesis process of (2E,4E)-5-(4-methoxyphenyl)-1-(piperidine-1-yl)penta-2,4-dien-1-one.
$^1$H NMR (400 MHz, $CDCl_3$) δ 7.42-7.33 (m, 3H), 6.91- 6.64 (m, 4H), 5.89 (d, 1H, J = 14.9 Hz), 5.48 (bs, 1H), 3.81 (s, 3H), 2.9 (d, 3H, J = 5.13).

(7) Synthesis of (2E,4E)-N-ethyl-5-(4-methoxyphenyl)penta-2,4-dieneamide (R-2)

(2E,4E)-N-ethyl-5-(4-methoxyphenyl)penta-2,4-dieneamide (11.2 mg, 41%) was synthesized by the same method as described above, except that ethylamine, instead of piperidine, was used in the synthesis process of (2E,4E)-5-(4-methoxyphenyl)-1-(piperidine-1-yl)penta-2,4-dien-1-one.
$^1$H NMR (400 MHz, $CDCl_3$) δ 7.38 (d, 2H, J = 8.8 Hz), 7.34 (d, 1H, J = 10.8 Hz), 6.86 (d, 2H, J = 8.8 Hz), 6.84-6.66 (m, 2H), 5.87 (d, 1H, J = 15.2 Hz), 5.43 (bs, 1H), 3.81 (s,3H), 3.4 (m, 2H), 1.18 (t, 3H, J = 7.3 Hz).

(8) Synthesis of (2E,4E)-5-(4-methoxyphenyl)-N,N-dimethylpenta-2,4-dieneamide (R-3)

(2E,4E)-5-(4-methoxyphenyl)-N,N-dimethylpenta-2,4-dieneamide (16.8 mg, 43%) was synthesized by the same method as described above, except that dimethylamine, instead of piperidine, was used in the synthesis process of (2E,4E)-5-(4-methoxyphenyl)-1-(piperidine-1-yl)penta-2,4-dien-1-one.
$^1$H NMR (400 MHz, $CDCl_3$) δ 7.44 (ddd, 1H, J = 14.6, 8.8, 1.2 Hz), 7.39 (d, 2H, J = 8.8 Hz), 6.86 (d, 2H, J = 8.8 Hz), 6.81-6.73 (m, 2H), 6.4 (d, 1H, J = 14.6 Hz), 3.81 (s, 3H), 3.1 (s, 3H), 3.03 (s, 3H).

**[Preparation Example 5] Synthesis of pentadienoyl compound having para-methoxy** group

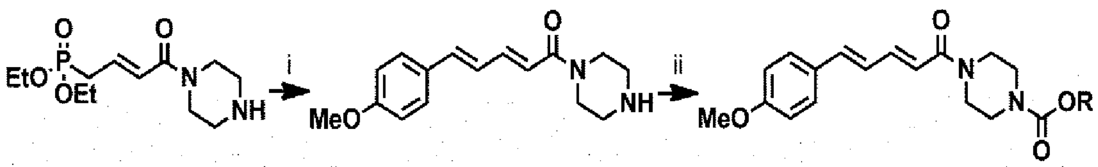


**R = $C_1$- $C_6$ alkyl**
**(for example, methyl, ethyl, n-propyl, isopropyl, n-butyl, tert-butyl, etc)**

(1) Synthesis of (2E,4E)-5-(4-methoxyphenyl)-1-(piperazine-1-yl)penta-2,4-dien-1-one (R-13)

(2E,4E)-5-(4-methoxyphenyl)-1-(piperazine-1-yl)penta-2,4-dien-1-one (93 mg, 45%) was synthesized by the same method as described above, except that p-methoxybenzaldehyde, instead of p-methylthiobenzaldehyde, was used in the synthesis process of (1) (2E,4E)-5-(4-(methylthio)phenyl)-1-(piperazine-1-yl)penta-2,4-dien-1-one in Preparation Example 3.
$^1$H NMR (400 MHz, $CDCl_3$) δ 7.44 (dd, 1H, J = 10.6, 9.8 Hz), 7.38 (d, 2H, J = 8.8 Hz), 6.87 (d, 2H, J = 8.8 Hz), 6.82-6.72 (m, 2H), 6.38 (d, 1H, J = 14.6 Hz), 3.81 (s, 3H), 3.8-3.5 (m, 4H), 2.93-2.85 (m, 4H).

(2) Synthesis of methyl 4-((2E,4E)-5-(4-methoxyphenyl)penta-2,4-dienoyl)piperazine-1-carboxylate (R-20, BWL-215)

After methyl chloroformate obtained using triphosgene and methanol was dissolved in hexane (1 mL), anhydrous sodium carbonate was added and a catalytic amount of triethylamine was added, and then a hexane solution (0.5 mL) of (2E,4E)-5-(4-(methoxyphenyl)-1-(piperazine-1-yl)penta-2,4-dien-1-one (5.80 mg, 0.020 mmol) was added. After completion of the reaction, water (1 mL) was added, the volatile material was removed by vacuum distillation, and the residue was partitioned between ethyl acetate (5 mL) and water (1 mL), and then the organic layer was separated. The water layer was extracted with ethyl acetate (5 mL x 2), the combined organic layer was dehydrated with anhydrous sodium sulfate, filtered and vacuum-distilled, and the residue was purified by silica gel column chromatography (hexane ethyl acetate = 1 1), thereby obtaining methyl 4-((2E,4E)-5-(4-(methoxyphenyl)penta-2,4-dienoyl)piperazine-1-carboxylate (3.7 mg, 51%).
$^1$H NMR (400 MHz, $CDCl_3$) δ 7.47 (dd, 1H, J = 14.6, 10.2 Hz), 7.39 (d, 2H, J = 8.8 Hz), 6.87 (d, 2H, J = 8.8 Hz), 6.83-6.72

(m, 2H), 6.36 (d, 1H, J = 14.6 Hz), 3.83 (s, 3H), 3.72 (s, 3H), 3.71-3.44 (m, 8H).

(3) Synthesis of ethyl 4-((2E,4E)-5-(4-methoxyphenyl)penta-2,4-dienoyl)piperazine-1-carboxylate (R-23, BWL-211)

Ethyl 4-((2E,4E)-5-(4-methoxyphenyl)penta-2,4-dienoyl)piperazine-1-carboxylate (3.4 mg, 42%) was synthesized by the same method as described above, except that ethanol, instead of methanol, was used in the synthesis process of methyl 4-((2E,4E)-5-(4-methoxyphenyl)penta-2,4-dienoyl)piperazine-1-carboxylate.
$^1$H NMR (400 MHz, $CDCl_3$) δ 7.46 (dd, 1H, J = 14.6, 10.2 Hz), 7.38 (d, 2H, J = 8.8 Hz), 8.87 (d, 2H, J = 8.8 Hz), 6.83-6.72 (m, 2H), 6.37 (d, 1H, J = 14.6 Hz), 4.16 (q, 2H), 3.82 (s, 3H), 3.8-3.42 (m, 8H), 1.27 (t, 3H, J = 7.1 Hz).

(4) Synthesis of isopropyl 4-((2E,4E)-5-(4-methoxyphenyl)penta-2,4-dienoyl)piperazine-1-carboxylate (R-26, BWL-213)

Isopropyl 4-((2E,4E)-5-(4-methoxyphenyl)penta-2,4-dienoyl)piperazine-1-carboxylate (3.2 mg, 49%) was synthesized by the same method as described above, except that isopropanol, instead of methanol, was used in the synthesis process of methyl 4-((2E,4E)-5-(4-methoxyphenyl)penta-2,4-dienoyl)piperazine-1-carboxylate.
$^1$H NMR (400 MHz, $CDCl_3$) δ 7.46 (dd, 1H, J = 14.6, 10.0 Hz), 7.39 (d, 2H, J = 8.8 Hz), 6.87 (d, 2H, J = 8.8 Hz), 6.83-6.72 (m, 2H), 6.36 (d, 1H, J = 14.6 Hz), 4.94 (m, 1H), 3.81 (s, 3H), 3.8-3.42 (m, 8H), 1.26 (d, 6H, J = 6.4 Hz).

(5) Synthesis of (2E,4E)-5-(4-methoxyphenyl)-1-(4-propionylpiperazine-1-yl)penta-2,4-dien-1-one (R-18, BWL-233)

After (2E,4E)-5-(4-methoxyphenyl)-1-(piperazine-1-yl)penta-2,4-dien-1-one (5.9 mg, 0.0216 mmol) and triethylamine (20 uL) were dissolved in anhydrous methylene chloride (2 mL), anhydrous propionic acid (2.8 uL) was added at room temperature, followed by stirring for 1 hour. After completion of the reaction, the volatile solvent was removed by vacuum distillation, ethyl acetate (10 mL) was added, distilled water (2 mL) and a saturated sodium bicarbonate solution (2 mL) were added for partitioning, thereby separating the organic layer. The organic layer was washed with brine, dehydrated with anhydrous sodium sulfate, filtered and vacuum-distilled, and then the residue was purified by silica gel column chromatography (chloroform : methanol = 8 : 1) to obtain (2E,4E)-5-(4-(methylthio)phenyl)-1-(4-propionylpiperazine-1-yl)penta-2,4-dien-1-one (4.2 mg, 60%).
$^1$H NMR (400 MHz, $CDCl_3$) δ 7.47 (dd, 1H, J= 10.1, 14.6 Hz), 7.39 (d, J = 8.5 Hz), 6.73-6.91 (m, 4H), 6.38 (d, J = 14.6 Hz). 3.81 (s, 3H), 3.43-3.79 (m, 8H), 2.37 (q, 1H, J = 7.3 Hz), 1.16 (t, 3H, J = 7.3 Hz).

**[Preparation Example 6] Synthesis of pentadienoyl compound including meta-methoxy group**

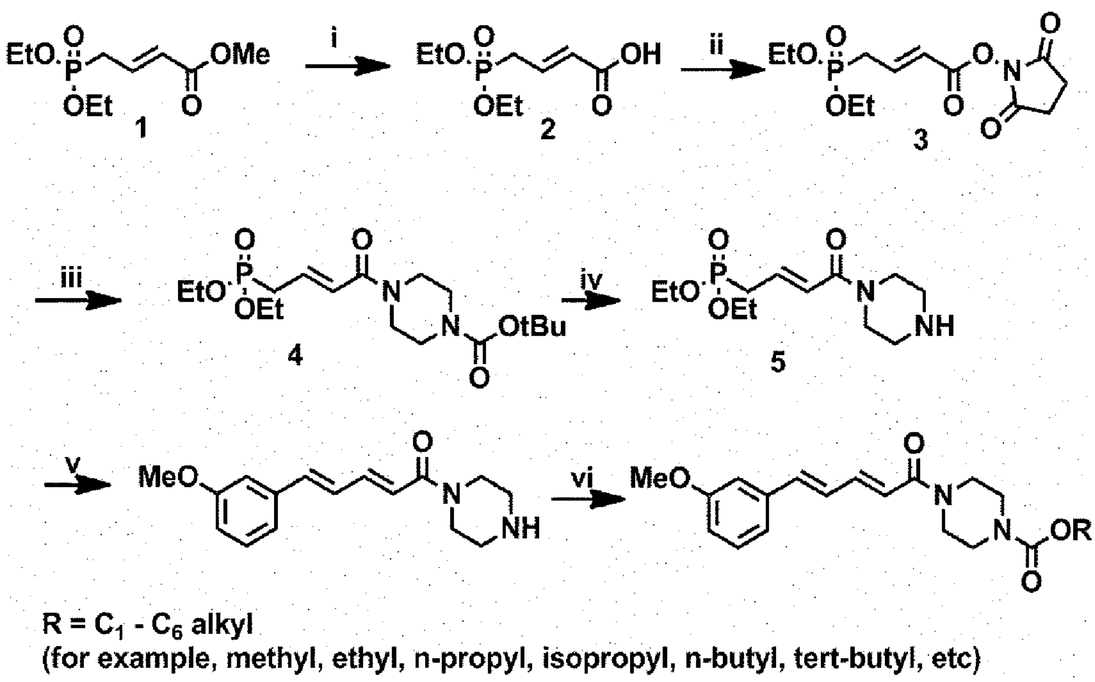


**R = $C_1$ - $C_6$ alkyl**
**(for example, methyl, ethyl, n-propyl, isopropyl, n-butyl, tert-butyl, etc)**

(1) Synthesis of (2E,4E)-5-(3-methoxyphenyl)-1-(piperazine-1-yl)penta-2,4-dien-1-one

(E)-diethyl (4-oxo-4-(piperazine-1-yl)but-2-en-1-yl)phosphonate (85 mg, 0.294 mmol) was dissolved in anhydrous methylene chloride/dimethylformamide (4/1, 3 mL), and then p-methoxybenzaldehyde (40 mg, 0.31 mmol) was added to the resulting mixture, and then cooled to 0 □. Potassium tert-butoxide (1M in THF, 0.30 mL) was slowly added. The resulting mixture was stirred for 6 hours at the same temperature, and water (2 mL) was added to stop the reaction. The volatile solvent was completely removed by vacuum distillation, the residue was partitioned between ethyl acetate (20 mL) and water (5 mL), followed by separating the organic layer. The water layer was extracted with ethyl acetate (10 mL x 2), and the combined organic layer was dehydrated with anhydrous sodium sulfate, filtered and vacuum-distilled, and the residue was purified by silica gel column chromatography (chloroform : methanol = 8 : 1) to obtain (2E,4E)-5-(4-(methylthio)phenyl)-1-(piperazine-1-yl)penta-2,4-dien-1-one (14.6 mg, 8%).
$^1$H NMR (400 MHz, $CDCl_3$) $\delta$ 7.44 (ddd, 1H, J= 1.0, 9.3, 14.6 Hz), 7.22-7.29 (m, 1H), 7.04 (bd, 7.6 Hz), 6.79-6.98 (m, 4H), 6.43 (d, J = 14.9 Hz). 3.82 (s, 3H), 2.79-2.97 (m, 8H).

(2) Synthesis of ethyl ((2E,4E)-5-(3-methoxyphenyl)penta-2,4-dienoyl)piperazine-1-carboxylate (R-22, BWL-231)

After (2E,4E)-5-(3-methoxyphenyl)-1-(piperazine-1-yl)penta-2,4-dien-1-one (4.0 mg, 0.0147 mmol) and excessive sodium carbonate were added to anhydrous THF, ethyl chloroformate (4 uL) was added and stirred for 2 hours. After completion of the reaction, the volatile solvent was removed by vacuum distillation, ethyl acetate (10 mL) was added, and distilled water (2 mL) and a saturated sodium bicarbonate solution (2 mL) were added for partitioning to separate the organic layer. The organic layer was washed with brine, dehydrated with anhydrous sodium sulfate, filtered and vacuum-distilled, and the residue was purified by silica gel column chromatography (chloroform : methanol = 8 : 1), thereby obtaining ethyl ((2E,4E)-5-(3-methoxyphenyl)penta-2,4-dienoyl)piperazine-1-carboxylate (3.1 mg, 61%).
$^1$H NMR (400 MHz, $CDCl_3$) $\delta$ 7.46 (ddd, 1H, J= 1.2, 9.0, 14.7 Hz), 7.26 (t, 1H, J = 7.8 Hz), 7.04 (bd, 1H, J = 7.6 Hz), 6.97 (m, 1H), 6.81-6.93 (m, 3H), 6.42 (d, 1H, J = 14.6 Hz). 4.16 (q, 2H, J = 7.1 Hz), 3.82 (s, 3H), 3.43-3.79 (m, 8H), 1.27 (t, 3H, J = 7.1 Hz).

(3) Synthesis of isopropyl ((2E,4E)-5-(3-methoxyphenyl)penta-2,4-dienoyl)piperazine-1-carboxylate (R-25, BWL-232)

Isopropyl ((2E,4E)-5-(3-methoxyphenyl)penta-2,4-dienoyl)piperazine-1-carboxylate (3.5 mg, 67%) was obtained by the same method as the synthesis method of ethyl ((2E,4E)-5-(3-methoxyphenyl)penta-2,4-dienoyl)piperazine-1-carboxylate by using (2E,4E)-5-(3-methoxyphenyl)-1-(piperazine-1-yl)penta-2,4-dien-1-one (4.0 mg, 0.0147 mmol) and isopropyl chloroformate (4 uL) as starting materials.
$^1$H NMR (400 MHz, $CDCl_3$) $\delta$ 7.46 (ddd, 1H, J= 0.8, 9.3, 14.7 Hz), 7.26 (t, 1H, J = 8.1 Hz), 7.04 (bd, 1H, J = 7.8 Hz), 6.97 (m, 1H), 6.81-6.93 (m, 3H), 6.43 (d, 1H, J = 14.6 Hz). 4.94 (m, 1H), 3.82 (s, 3H), 3.43-3.79 (m, 8H), 1.26 (d, 6H, J = 6.3 Hz).

**Experimental Examples**

**[Chemicals]**

As long chain fatty acids (long chain FAs), oleic acid (OA, 18 : 1) and palmitic acid (PA, 16 : 0) were purchased from Sigma-Aldrich (St. Louis, MO, USA).
OA was dissolved in dimethyl sulfoxide (DMSO, Sigma-Aldrich), and aliquoted into Eppendorf tubes. The final concentration of a stock solution was 100 mM.
PA was dissolved in DMSO, and incubated at 56 °C for 10 minutes. A PA stock solution was inverted and vortexed during incubation, and then aliquoted into Eppendorf tubes. The final concentration of a stock solution was 100 mM.
A fresh OA/PA mixture was prepared from 1% FA-free bovine serum albumin (FA-free BSA, GenDEPOT, Katy, TX, USA) containing 0.66 mM OA and 0.33 mM PA (molar ratio: 2:1). The final concentration of the OA/PA mixture was 1 mM.
All stock solutions were stored at -20 °C before the experiment.
Piperine and resveratrol were purchased from Sigma-Aldrich. Both compounds were dissolved in DMSO.
YU2014 and all test compounds were synthesized at Wonkwang University (Iksan, Jeollabuk-do). All compounds, excluding R-25 (BWL-232) dissolved in ethanol (EtOH), were dissolved in DMSO.

**[Preparation of 3T3-L1 cells]**

3T3-L1 mouse preadipocytes (American Type Culture Collection) were incubated in an incubator set to 37 □, humidity of 95 % and 5% $CO_2$. The 3T3-L1 mouse preadipocytes were sufficiently proliferated using a proliferation medium (Dulbecco's modified Eagle's medium (DMEM) supplemented with 5% fetal bovine serum (FBS), 100 U/mL of penicillin and 100 μg/mL of streptomycin), and treated with a differentiation medium (DMEM containing 5% FBS, 0.5 mM 3-isobutyl-1-methylxanthine, 1 mM dexamethasone and 10 μg/mL insulin) for 4 days. Afterward, the 3T3-L1 mouse preadipocytes were treated with an induction medium (DMEM containing 5 % FBS and 10 μg/mL insulin, DMEM containing 5 % FBS) for 2 days, and to maintain differentiated adipocytes until the end of the experiment, the induction medium was replaced every day. The 3T3-L1 cells were used in Experimental Examples 1, 2, and 3.

**[Preparation of Huh-7 cells]**

Huh-7 cells were maintained in a RPMI1640 medium containing 10% FBS, 100U/mL of penicillin, and 100 μg/mL of streptomycin. All reagents were purchased from Life Technologies Corporation (Grand Island, NY, USA).
The Huh-7 cells were incubated under an atmosphere containing 5% $CO_2$ at 37 °C.
In Experimental Example 4, the Huh-7 cells were plated in 24 wells at $3X10^5$ cells/mL and incubated under an atmosphere containing 5% $CO_2$ at 37 °C.
In Experimental Example 5, the Huh-7 cells were plated in 6 wells at $4X10^5$ cells/mL and incubated under an atmosphere containing 5% $CO_2$ at 37 °C.
In Experimental Example 6, the Huh-7 cells were plated in 24 wells at $4X10^5$ cells/mL and incubated under an atmosphere containing 5% $CO_2$ at 37 °C.
One day later, the cell density approached about 80 to 90%. The medium was changed to an FBS-free RPMI1640 medium, and incubated under an atmosphere containing 5% $CO_2$ at 37 °C for 24 hours.
The Huh-7 cells were used in Experimental Examples 4, 5, and 6.

**[Experimental Example 1] Confirmation of lipid droplets through Oil-Red-O staining and detection of stained lipid droplets in cells after induction of 3T3-L1 preadipocyte differentiation**

Experimental method

To confirm capacity of inhibiting the formation of lipid droplets of a test material, 3T3-L1 cells, which are preadipocytes, were induced to differentiate into adipocytes, and then the lipid droplets were stained through the Oil-Red-O staining.
First, after preadipocytes, **3T3-L1** cells, were incubated in a dish having a diameter of 10 $cm^2$, excluding G1 and G2, the cells were treated with 50 μM of a test material previously diluted with a culture medium (refer to Table 2).
Next, for staining, first, the cell supernatant present in a well was removed, and fixed with 4% paraformaldehyde. Afterward, after adding a 100% 1,2-propanediol dehydration solution, the resulting sample was incubated for 5 minutes, and then an Oil-Red-O stain solution was added for staining of lipid droplets. After the Oil-Red-O staining, an 85% 1,2-propanediol stain differential solution was added for washing.
Finally, after filling a well with D.W. without drying the stained well, the formation of lipid droplets was confirmed by observation using a microscope.
After confirming the formation of lipid droplets using Oil-Red-O staining, a part stained with isopropanol was dissolved, and the degree of staining lipid droplets was confirmed by absorbance at 540 nm. The increase/decrease rate, compared to the normal control, was evaluated using an optical density (OD) value.

[Table 2]

| Name | Adipocyte differentiation | Cell count |
|---|---|---|
| G1 | - | $5X10^5$ |
| G2 | + | $5X10^5$ |
| R-8 | + | $5X10^5$ |
| R-7 | + | $5X10^5$ |
| R-28 | + | $5X10^5$ |
| R-15 | + | $5X10^5$ |

(continued)

| Name | Adipocyte differentiation | Cell count |
|---|---|---|
| R-3 | + | $5X10^5$ |
| R-29 | + | $5X10^5$ |
| R-9 | + | $5X10^5$ |
| R-11 | + | $5X10^5$ |
| R-17 | + | $5X10^5$ |
| R-30 | + | $5X10^5$ |
| R-4 | + | $5X10^5$ |

Experimental results

The experimental results are shown in FIGS. 1 and 2.

As shown in FIG. 1, it can be confirmed that lipid droplet staining was less shown in all test groups, compared to the induced control G2.

For more accurate analysis, the stained cells were dissolved in isopropanol, and the degree of lipid droplet staining was confirmed through absorbance measurement, and as shown in FIG. 2, in all test groups, it can be confirmed that an amount of the stained lipid droplets was less than G2. In addition, as shown in FIG. 2, the lipid droplet staining in the test groups treated with R-8, 7, 28, 15, 29, 9, 11, 17, and 30 was less than that of the test groups treated with R-3 and 4. Accordingly, it was seen that an effect of inhibiting the formation of lipid droplets when a ring structure was included at the first position of the pentadienoyl compound was higher than that when a ring structure was not included.

**[Experimental Example 2] Measurement of triglyceride content after induction of 3T3-L1 preadipocyte differentiation**

Experimental method

Preadipocytes, 3T3-L1 cells, were incubated in a dish having a diameter of 10 $cm^2$ by the same method as described in Experimental Example 1, and then treated with a predetermined concentration of a test material diluted with a culture medium. After treatment, a triglyceride content was confirmed using an assay kit.

Experimental results

The experimental results are shown in FIG. 3.

In all test groups treated with all test materials, it can be confirmed that a triglyceride content was lower than that of G2. Accordingly, the fact that a test material inhibits the formation of lipid droplets, and lowers the content of a triglyceride, which is a product of lipid metabolism, can be confirmed. In addition, as shown in FIG. 3, triglyceride contents of the test groups treated with R-8, 7, 28, 15, 29, 9, 11, 17, and 30 were lower than that of the test group treated with R-3. Therefore, it was seen that the effect of inhibiting the formation of lipid droplets and the effect of reducing a content of the product of lipid metabolism when a ring structure is included at the first position of the pentadienoyl compound were higher than that when a ring structure was not included.

**[Experimental Example 3] Measurement of glycerol content after induction of 3T3-L1 preadipocyte differentiation**

Experimental method

Preadipocytes, 3T3-L1 cells, were incubated in a dish having a diameter of 10 $cm^2$ by the same method as described in Experimental Example 1, and then treated with a predetermined concentration of a test material diluted with a culture medium. After treatment, a glycerol content was confirmed using an assay kit.

Experimental results

The experimental results are shown in FIG. 4.

In all test groups treated with all test materials, it can be confirmed that a glycerol content was lower than that of G2. Accordingly, the fact that a test material inhibits the formation of lipid droplets, and lowers the content of a glycerol, which is a product of lipid metabolism, can be confirmed. In addition, as shown in FIG. 4, glycerol contents of the test groups treated with R-8, 7, 28, 15, 29, 9, 11, 17, and 30 were lower than that of the test group treated with R-3. Therefore, it was seen that the effect of inhibiting the formation of lipid droplets and the effect of reducing a content of the product of lipid metabolism when a ring structure is included at the first position of the pentadienoyl compound were higher than that when a ring structure was not included.

**[Experimental Example 4] Analyses of Oil-Red O (ORO) staining and lipid accumulation**

Experimental method

Huh-7 cells of each well, excluding control cells, were pretreated with an OA/PA mixture (however, R-28 is OA) for 1 hour to induce steatosis. Control cells were pretreated with 1% FA-free BSA.
Each well was treated with 10 μM, 20 μM, or 40 μM of a test compound (however, for R-28, 2 μM, 10 μM and 50 μM), and cultured for 24 hours. The control cells and the OA/PA-treated cells were treated with DMSO. Cells of a positive control were treated with 20 μM of resveratrol, 10 μM of piperine, and 10 μM, 20 μM, or 40 μM of YU2014.
The medium was entirely aspirated, and the cells were washed with PBS three times. The cells were fixed with 4% formaldehyde (Sigma-Aldrich), and cultured at room temperature for 20 minutes. The cells were washed with PBS twice, and then with distilled water twice.
An ORO solution was prepared by mixing 6 mL of an ORO stock solution (Sigma-Aldrich) and 4 mL of distilled water, and filtering the mixed solution through a 0.45 μm filter. 200 μL of the ORO solution was added to each well, and cultured at room temperature for 30 minutes. The cells were washed with distilled water for 10 or more times until the background was clean.
Images were obtained from a Cytation 5 Cell Imaging Multi-Mode Reader (BioTek, Winooski, VT, USA). After the ORO-stained samples were imaged, distilled water was thoroughly removed from each well, and then the well was completely dried.
200 μL of isopropyl alcohol was added to each well, and the cells were collected in a 96-well plate. Absorbance was measured at 540 nm, and data was analyzed.

Experimental results

The experimental results are shown in FIGS.5 to 9.
As shown in FIGS. 5 and 6, the degree of lipid staining when treated with a test compound was less than the induced control (OA/PA or oleic acid).
As shown in FIGS. 7 and 8, the degree of lipid staining for a test compound was decreased as a treatment concentration increased. Although the treatment concentration of YU2014 increased, the degree of lipid staining was not greatly changed (FIG. 5).
As shown in FIG. 9, when the degree of lipid staining for a test compound was compared to each of the degrees of lipid staining for resveratrol (RSV) and YU2014 at the same concentration (20 μM), the degree of lipid staining for the test compound was much lower.
Therefore, it was seen that the test compound has a higher effect of inhibiting lipid accumulation in cells than resveratrol and piperine, which are conventionally known to inhibit lipid accumulation in cells. In addition, it was seen that the effect of inhibiting lipid accumulation in cells when the pentadienoyl compound includes a ring structure containing a substituent at the first position (e.g., the test compound) is much higher than that when a ring structure not including a substituent (YU2014) is included.

**[Experimental Example 5] Quantitative analysis of mRNA**

Total RNA extraction and cDNA synthesis

Huh-7 cells of each well, excluding control cells, were pretreated with an OA/PA mixture for one hour to induce steatosis. The control cells were pretreated with 1% FA-free BSA.
Each well was treated with 10 μM of a test compound, and then the cells were incubated for 24 hours. The control cells and OA/PA-treated cells were treated with DMSO. Cells of a positive control were treated with 10 μM of resveratrol, 10 μM of piperine, and 10 μM of YU2014. The medium was entirely aspirated, and the cells were washed with PBS (phosphate buffered saline, Corning, Manassas, VA, USA) two times.
Total RNA was extracted using a RNeasy RNA isolation kit (Qiagen, Hilden, Germany).

10 μL of β-mercaptoethanol (Sigma-Aldrich) per 1 mL of lysis buffer was added, thereby preparing an RNA lysis buffer. The cells were washed twice with cold PBS, and disrupted by adding 600 μL of the RNA lysis buffer. A RNase-free syringe was equipped with a blunt 20-gauge needle (diameter: 0.99 mm), and the lysate was homogenized 15 times. To the homogenized lysate, 70% EtOH prepared by mixing diethylpyrocarbonate (DEPC)-treated water and EtOH was added with the same volume, and the resulting mixture was well mixed by pipetting and vigorously stirred for 10 seconds.

Each sample was transferred to a spin column, and centrifuged at 8,000 x g for 20 seconds. The solution passing through the column was removed, and 700 μL of a washing buffer was added to the spin column. Centrifugation was performed at 8,000 x g, and the solution passing through the column was removed. 500 μL of a washing buffer including EtOH was added to the spin column, and centrifuged at 8,000 x g for 2 minutes. All of the solution passing through the column was discarded and centrifuged once, and then the spin column membrane was completely dried. The spin column was put into a new Eppendorf tube, 35 μL of RNase-free water was added directly to the spin column membrane, and then centrifugation was performed at 8,000 x g for one minute to dissolve and isolate RNA.

An RNA concentration was measured at O.D. 260 nm using a nanophotometer N60 (Implen, Munich, Germany). The concentration and purity of RNA were measured at O.D. 260/280 nm and calculated as a ratio.

3 μg of total RNA was reverse transcribed in a first strand synthesis buffer including 6 μg/mL of oligo (dT) primer, 3 μg/mL of a random primer, 50 U of reverse transcriptase, 4 mM dNTP, 5 mM dNTP, 5 mM magnesium chloride ($MgCl_2$), and 40 U RNase inhibitor. cDNA was synthesized using a polymerase chain reaction (PCR) apparatus which was designed to undergo denaturation at 95 °C for 5 minutes, cDNA synthesis at 42 °C for 50 minutes, and denaturation at 95 °C for 5 minutes.

After cDNA synthesis, DEPC-treated water (Invitrogen, Carlsbad, CA, USA) was used to dilute cDNA to 1/400.

Quantitative Real-Time PCR (qRT-PCR)

qRT-PCR was performed on genes in Table 3 using a StepOnePlus Real-Time PCR System (Applied Biosystems, Foster City, CA, USA). All primers for qRT-PCR were synthesized at GenoTech (Daejeon).

A reaction mixture was prepared by mixing 3 μL of cDNA, 8 μL of SYBR green Master Mix (Applied Biosystems), 3 μL of a primer mix (sense primers and antisense primers) and distilled water (DW). A 36b4 gene was used as a control.

qRT-PCR was performed using a StepOnePlus Real-Time PCR System set to denature at 95 °C for 15 seconds, anneal and synthesize at 60 °C for 1 minute. 40 cycles were performed in the total reaction.

Quantitative analyses for the genes in Table 3 were performed.

[Table 3]

| Gene | Species | Sense primer (SEQ ID NO. 1 - 7) | Anti-sense primer (SEQ ID NO. 8 - 14) |
|---|---|---|---|
| *36b4* | Human | AAGGCTGTGGTGCTGATG (SEQ ID NO. 1) | GGTCCTCCTTGGTGAACA (SEQ ID NO. 8) |
| *Pparα* | Human | ACGCTTTCACCAGCTTCGAG (SEQ ID NO. 2) | GAAAGAAGCCCTTGCAGCCT (SEQ ID NO. 9) |
| *Fgf21* | Human | ACCAGAGCCCCGAAAGTCT (SEQ ID NO. 3) | CTTGACTCCCAAGATTTGAATAACT C (SEQ ID NO. 10) |
| *Fas* | Human | TGCAGAAGATGTAGATTGTGTGAT GA (SEQ ID NO. 4) | GGGTCCGGGTGCAGTTTATT (SEQ ID NO. 11) |
| *Srebpl c* | Human | GCAAGGCCATCGACTACATT (SEQ ID NO. 5) | GGTCAGTGTGTCCTCCACCT (SEQ ID NO. 12) |
| *Sirtl* | Human | TTGACTGTGAAGCTGTACGAGGA (SEQ ID NO. 6) | CAAGCGGTTCATCAGCTGG (SEQ ID NO. 13) |
| *Ck2α* | Human | TGTCAGGGATTTCTTCAGTGC (SEQ ID NO. 7) | ATCAAGGAGAGGGTGGACTC (SEQ ID NO. 14) |

Experimental results

The experimental results are shown in FIGS. 10, 12, 13, 14, 16 and 19.

As shown in FIG. 10, when steatosis-induced Huh-7 cells were treated with the test compounds, Ppara mRNA levels were significantly increased compared to that of the induced control (OA/PA). In addition, when steatosis-induced Huh-7 cells were treated with piperine or YU2014, Ppara mRNA levels were hardly increased.

Accordingly, it was seen that the test compounds have an excellent effect of increasing Ppara mRNA expression in steatosis-induced liver cells. Thereby, it was confirmed that the test compounds can be used as a Ppara activating material. In addition, the effect of increasing Ppara mRNA expression in steatosis-induced liver cells when the pentadienoyl compound includes a ring structure having a substituent at the first position (e.g., the test compound) was much greater than that when the pentadienoyl compound includes a ring structure not having a substituent (piperine or YU2014).

As shown in FIG. 12, when the steatosis-induced Huh-7 cells are treated with the test compounds, Fgf21 mRNA levels were significantly increased, compared to that of the induced control (OA/PA). In addition, when the steatosis-induced Huh-7 cells are treated with piperine or YU2014, there was almost no increase in Fgf21 mRNA level.

Accordingly, it was seen that the test compounds have an excellent effect of increasing Fgf21 mRNA expression in steatosis-induced liver cells. Therefore, it was confirmed that the test compounds can be used as a Fgf21 activating material. In addition, the effect of increasing Fgf21 mRNA expression in steatosis-induced liver cells exhibited when the pentadienoyl compound includes a ring structure having a substituent at the first position (e.g., the test compound) was much greater than that when the pentadienoyl compound includes a ring structure without a substituent (piperine or YU2014).

As shown in FIG. 13, when the steatosis-induced Huh-7 cells were treated with the test compounds, Fas mRNA levels were greatly lower than that of the induced control (OA/PA). In addition, Fas mRNA levels when the steatosis-induced Huh-7 cells were treated with test compounds (except R-21) were much lower than when the steatosis-induced Huh-7 cells were treated with piperine or YU2014.

Accordingly, it was seen that the test compounds have an excellent effect of reducing Fas mRNA expression in steatosis-induced liver cells. Thereby, it was confirmed that the test compounds can be used as an Fas inhibitor. In addition, it was demonstrated that the effect of reducing Fas mRNA expression in steatosis-induced liver cells when a pentadienoyl compound includes a ring structure having a substituent at the first position (e.g., the test compound) is higher than that when a pentadienoyl compound includes a ring structure without a substituent (piperine or YU2014).

As shown in FIG. 14, when steatosis-induced Huh-7 cells were treated with the test compounds, Srebplc mRNA levels were greatly lower than that of the induced control (OA/PA). In addition, Srebplc mRNA levels when the steatosis-induced Huh-7 cells were treated with the test compounds were further decreased than when the steatosis-induced Huh-7 cells were treated with piperine or YU2014.

Therefore, it was seen that the test compounds have an excellent effect of reducing Srebplc mRNA expression in steatosis-induced liver cells. Thereby, it was confirmed that the test compounds can be used as a Srebplc inhibitor. It was also seen that the effect of reducing Srebplc mRNA expression in steatosis-induced liver cells when a pentadienoyl compound includes a ring structure having a substituent at the first position (e.g., the test compound) is higher than that when the pentadienoyl compound includes a ring structure without a substituent (piperine or YU2014).

In the case of fatty liver or fatty liver-related disease, it has been known that the expression levels of genes (e.g., Ppara and Fgf21) related to lipid metabolism such as β-oxidation in liver cells decrease, and the expression levels of genes (e.g., Fas and Srebplc) related to lipogenesis in liver cells increase.

It was seen that the test compound significantly increases Ppara mRNA and Fgf21 mRNA expression levels, and greatly reduces Fas mRNA and Srebplc mRNA expression levels in steatosis-induced Huh-7 cells. On the contrary, it was seen that piperine and YU2014 reduce Fas mRNA and Srebplc mRNA expression levels, but have no effect on the Ppara mRNA and Fgf21 mRNA expression levels in the steatosis-induced Huh-7 cells.

According to the above-described experiments, it was confirmed that the effect of activating β-oxidation in fatty acids when a pentadienoyl compound includes a ring structure having a substituent at the first position (e.g., the test compound) is higher than that when a pentadienoyl compound includes a ring structure without a substituent (piperine or YU2014). In addition, it was confirmed that when the pentadienoyl compound includes a ring structure having a substituent at the first position (e.g., the test compound), an effect of inhibiting lipogenesis is exhibited. Therefore, it is expected that the function of regulating lipid metabolism in liver cells when the pentadienoyl compound includes a ring structure having a substituent at the first position (e.g., the test compound) is higher than that when the pentadienoyl compound includes a ring structure without a substituent (piperine or YU2014), resulting in an excellent effect of improving steatosis-related symptoms.

For this reason, when the pentadienoyl compound includes a ring structure having a substituent at the first position (e.g., the test compound), the excellent effect of preventing or treating fatty liver and fatty liver-related disease can be expected.

As shown in FIG. 16, when steatosis-induced Huh-7 cells are treated with the test compounds, Sirt1 mRNA

levels are higher than that of the induced control (OA/PA). In addition, some of these test compounds (e.g., R-23, R-18, and R-22) greatly increased Sirt1 mRNA levels, compared to resveratrol (RSV), piperine, or YU2014.

Accordingly, it was seen that the test compounds have an excellent effect of increasing Sirt1 mRNA expression in steatosis-induced liver cells. Thereby, it was confirmed that the test compounds can be used as a SIRT1 activating material. It was also seen that the effect of increasing Sirt1 mRNA expression in steatosis-induced liver cells when the pentadienoyl compound includes a ring structure having a substituent at the first position (e.g., the test compound) is similar to or much higher than that when the pentadienoyl compound includes a ring structure without a substituent (piperine or YU2014).

As shown in FIG. 19, when the steatosis-induced Huh-7 cells are treated with the test compounds, Ck2α mRNA levels were lower than that of the induced control (OA/PA). In addition, some of these test compounds (e.g., R-24, R-18, and R-22) greatly reduced Ck2α mRNA levels, compared to resveratrol (RSV), piperine, or YU2014.

Accordingly, it was seen that the test compounds have an excellent effect of reducing Ck2α mRNA expression in steatosis-induced liver cells. Thereby, it was confirmed that the test compounds can be used as a CK2-inhibiting compound. It was also seen that the effect of reducing Ck2α mRNA expression in steatosis-induced liver cells when the pentadienoyl compound includes a ring structure having a substituent at the first position (e.g., the test compound) is similar to or much higher than that when the pentadienoyl compound includes a ring structure without a substituent (piperine or YU2014).

**[Experimental Example 6] Western blot analysis**

Experimental method

Huh-7 cells in each well, except control cells, were pretreated with an OA/PA mixture for 1 hour to induce steatosis. The control cells were pretreated with 1% FA-free BSA.

Each well was treated with 20 μM of a test compound and then incubated for 24 hours. The control cells and the OA/PA-treated cells were treated with DMSO. Positive control cells were treated with 20 μM resveratrol, 10 μM piperine, or 20 μM YU2014. The medium was entirely aspirated, and the cells were gently washed with cold PBS three times.

A cold lysis buffer including 150 mM sodium chloride (NaCl), 5 mM ethylenediaminetetraacetic acid (EDTA), 10 mM 4-(2-hydroxyethyl)-1-piperazineethanesulfonic acid (HEPES, pH 8.0), 1% (nonylphenoxypolyethoxylethanol (NP-40), 2 μg/mL of aprotinin, 1 μM pepstatin A, 10 μM leupeptin, 1 mM sodium fluoride (NaF), 0.2 mM sodium orthovanadate ($Na_2VO_4$), and 1 μM phenylmethylsulfonyl fluoride (PMSF) was added. All of the chemicals were purchased from Sigma-Aldrich.

The entire cell lysate was collected, and then gently mixed by pipetting 10 or more times to completely dissolve. The sample was incubated on ice for 20 minutes, and centrifuged at 13,000 rpm and 4 °C for 20 minutes. The supernatant was collected, and then the sample was mixed with a 5X sodium dodecyl sulfate (SDS) sample buffer including 50 mM TrisCl (pH 6.8), 2% SDS, 0.1% bromophenol blue and 10% glycerol at 95 °C for 5 minutes.

Equal amounts of proteins were respectively loaded in each well for 10% SDS-polyacrylamide gel electrophoresis (SDS-PAGE), together with a molecular weight marker. The gel was subjected to electrophoresis at 100 V for 1 hour in an electrophoresis chamber (Bio-Rad Laboratories, Hercules, CA, USA).

A polyvinylidene fluoride (PVDF) membrane (MilliporeSigma, Burlington, MA USA) was activated with methanol (MeOH) for 1 minute, and washed with a transfer buffer including 25 mM Tris, 190 mM glycine and 20% MeOH. The gel was put into the transfer buffer for 10 minutes.

A transfer sandwich (in the order of paper, gel, membrane, and paper) was assembled and the transfer sandwich was checked for bubbles. The transfer sandwich was put into a transfer tank (Bio-Rad Laboratories), and then a lump of ice was put into the transfer tank to prevent the transfer buffer from being heating up. The protein was transferred from the gel to the PVDF membrane at 100 V for 1 hour. The PVDF membrane was washed with distilled water, and then blocked with a blocking buffer prepared of 5% non-fat milk (Difco Laboratories, Franklin Lakes, NJ, USA) in TBST containing 0.1% Tween 20 (TBST, Sigma-Aldrich) at room temperature for 1 hour.

The membrane was incubated in buffer in which primary antibodies were suitably diluted. Sirtuin 1 (SIRT1), Casein Kinase 2 alpha (CK2α), Peroxisome proliferator-activated receptor alpha (PPARα) and Sterol regulatory element-binding protein 1 (SREBP1) antibodies were purchased from Santa Cruz Biotechnology (Dallas, Texas, U.S.A.). The membrane was washed with TBST three times for 5 minutes each.

The membrane was incubated in a blocking buffer together with a recommended diluent for horseradish peroxidase (HRP)-conjugated secondary mouse antibodies at room temperature for 1 hour. The secondary antibodies were purchased from Santa Cruz Biotechnology.

The membrane was washed with TBST five times for 5 minutes each. An excessive reagent was removed, and the membrane was covered with a transparent plastic wrap.

An ECL primer western blotting detection reagent (GE Healthcare, Chicago, IL, USA) was applied according to the manufacturer's instructions.

Images were obtained using ImageQuant LAS 3000 (Fujifilm, Tokyo, Japan). Data was analyzed.

Experimental Results

The experimental results are shown in FIGS. 11, 15, 17, and 20.

As shown in FIG. 11, PPARα protein levels when steatosis-induced Huh-7 cells were treated with the test compounds were higher than that of the induced control (OA/PA). Therefore, it was seen that the test compounds have an effect of increasing PPARα protein expression in steatosis-induced liver cells.

Considering the results of Experimental Examples 5 and 6, it was seen that test compounds have an effect of increasing a Ppara mRNA level and a PPARα protein level. Accordingly, it was confirmed that the test compounds can be used as a PPARα activating material.

As shown in FIG. 15, SREBP1 protein levels when steatosis-induced Huh-7 cells were treated with the test compounds were lower than that of the induced control (OA/PA). Accordingly, it was seen that the test compounds have an effect of reducing SREBP1 protein expression in steatosis-induced liver cells.

Considering the results of Experimental Examples 5 and 6, it was seen that the test compounds have an effect of reducing a Srebp1c mRNA level and a SREBP1 protein level. Accordingly, it was confirmed that the test compounds can be used as a SREBP1 inhibitor.

As shown in FIG. 17, SIRT1 protein levels when steatosis-induced Huh-7 cells were treated with the test compound were higher than that of the induced control (OA/PA). Accordingly, it was seen that the test compounds have an effect of increasing SIRT1 protein expression in steatosis-induced liver cells.

Considering the results of Experimental Examples 5 and 6, it was seen that the test compounds have an effect of increasing a Sirt1 mRNA level and a SIRT1 protein level. Accordingly, it was confirmed that the test compounds can be used as a SIRT1-activating compound.

As shown in FIG. 20, CK2α protein levels when steatosis-induced Huh-7 cells were treated with the test compounds were lower than that of the induced control (OA/PA). Accordingly, it was seen that the test compounds have an effect of reducing CK2α protein expression in steatosis-induced liver cells.

Considering the results of Experimental Examples 5 and 6, it was seen that the test compounds have an effect of reducing a Ck2α mRNA level and a CK2α protein level. Accordingly, it was confirmed that the test compounds can be used as a CK2-inhibiting compound.

**[Experimental Example 7] SIRT1 direct fluorescent** assay

Experimental method

All materials were provided from a Sirt1 direct fluorescent screening assay kit (Cayman, Ann Arbor, MI, USA).

SIRT1, p53 conjugated aminomethyl coumarin (AMC) protein, and 3 mM $NAD^+$ were added to a diluted analysis buffer (50 mM Tris-HCl, pH 8.0, 137 mM NaCl, 2.7 mM KCl, and 1 mM $MgCl_2$).

25 μL of the analysis buffer, 5 μL of diluted SIRT1, and 5 μL of a solvent were added to each of three 100 % initial activity wells of a 96-well plate.

30 μL of the analysis buffer and 5 μL of a solvent were added to each of three background wells of the 96-well plate.

25 μL of the analysis buffer, 5 μL of diluted SIRT1, and 5 μL of a diluted test compound were added to each of three sample wells of the 96-well plate. The final DMSO concentration was less than 2%.

15 μL of a 3 mM $NAD^+$-containing substrate solution was added to all wells to begin the reaction. The 96-well plate was covered with a plate cover, and then incubated in a shaker at room temperature (25 to 30 °C) for 45 minutes.

A stop/developing solution was prepared. To prepare the final 5 mL solution, 200 μL of nicotinamide (NAM, Sigma-Aldrich) was added to a color developing powder weighing 30 mg, 4.8 mL of a diluted analysis buffer was added thereto, and the resulting mixture was stirred until the powder became a solution.

The plate cover was removed, and 50 μL of the stop/developing solution was added to each well.

The 96-well plate was covered with a plate cover, and incubated at room temperature (25 to 30 °C) for 30 minutes.

The plate cover was removed, and the plate was read using Cytation 5 at an excitation wavelength of 360 nm and an emission wavelength of 450 nm.

The mean fluorescence of each sample was measured.

The fluorescence of the background wells was subtracted from the fluorescence of the 100% initial activity wells and sample wells.

To determine the percent of each sample, each sample value was subtracted from the 100% initial activity

value, and the resulting value was divided by the 100% initial activity value and then multiplied by 100 and added to 100, which was expressed as a percentage.

$$\text{Degree of SIRT1 activation of sample (\%)} = 100 + \frac{Initial\ activity\ value - Sample\ value}{Initial\ activity\ value} \times 100$$

Experimental results

The experimental result is shown in FIG. 18.

As shown in FIG. 18, the 2 μM of test compounds, except R-22, raised SIRT1 activity 10% or more. 2 μM R-22 increased SIRT1 activity about 5%. In addition, the 10 μM of test compounds, except R-26, increased SIRT1 activity 20% or more. 10 μM R-26 increased SIRT1 activity 10% or more. Considering that 20 μM YU2014 increased SIRT1 activity about 20%, it was confirmed that the test compounds have the same or higher SIRT1 activation effect compared to YU2014 at lower concentrations. Accordingly, it was confirmed that the test compounds can be used as a SIRT1 activating material.

Therefore, it was seen that the effect of activating SIRT1 when a pentadienoyl compound includes a ring structure having a substituent at the first position (e.g., the test compound) is higher than that when a pentadienoyl compound includes a ring structure without a substituent (YU2014).

As a result, when the pentadienoyl compound includes a ring structure having a substituent at the first position (e.g., the test compound), an excellent effect of preventing or treating a SIRT1-mediated disease can be expected through SIRT1 activation.

**[Experimental Example 8] CK2 kinase assay**

Experimental method

All materials were provided from a CycLex CK2 kinase assay/inhibitor screening kit (Medical and Biological Laboratories CO., LTD, Nagoya, Japan).

All specimens were prepared in triplicate.

10 μL of a diluted sample was added to a 96-well plate placed on ice.

Three wells having 10 μL of a CK2α positive control (2 unit/μL) were included as a positive control in each analysis method. CK2α was purchased from New England Biolabs (Ipswich, MA, USA).

A phosphorylation reaction was initiated by adding 90 μL of a phosphorylation reaction buffer to each well, and the 96-well plate was covered with a plate cover. The plate was incubated at 30 °C for 30 minutes. Each well was washed with a washing buffer five times, and it was confirmed whether each well was completely filled. With gentle tapping, a residual washing solution was removed.

100 μL of HRP-conjugated detection antibodies were added to each well, and the plate was covered with a plate cover. While the plate was incubated at room temperature (25 to 30 °C) for 30 minutes, the remaining HRP-conjugated detection antibodies after use were removed. Each well was washed five times, and the residual washing buffer was removed with gentle tapping.

100 μL of a substrate reagent was added to each well, and incubated at room temperature (25 to 30 °C) for 15 minutes.

100 μL of a stop solution was added to each well, and incubated at room temperature (25 to 30 °C) for 15 minutes.

Absorbance at dual wavelengths of 450/540 nm was measured for each well using Cytation 5. Data was analyzed.

Experimental results

The experimental result is shown in FIG. 21.

Referring to FIG. 21, it was seen that the test compounds reduced CK2 activity at a concentration of 2 or 10 μM. However, 20 μM YU2014 did not have a CK2 activity reducing effect. Thereby, it was confirmed that the test compounds can be used as a CK2-inhibiting compound.

Accordingly, it was seen that, when a pentadienoyl compound includes a ring structure having a substituent at the first position (e.g., the test compound), a CK2 activity inhibitory effect was exhibited.

Therefore, when the pentadienoyl compound includes a ring structure having a substituent at the first position (e.g., the test compound), through CK2 inhibition, an excellent effect of preventing or treating a CK2-mediated disease

may be expected.

In addition, considering the results of Experimental Examples 7 and 8, when the pentadienoyl compound includes a ring structure having a substituent at the first position (e.g., the test compound), both of the SIRT1 activation effect and the CK2 inhibitory effect are exhibited, and thus an excellent effect of preventing or treating fatty liver and fatty liver-related disease may be expected, compared to that when the pentadienoyl compound includes a ring structure without a substituent at the first position (YU2014).

## Claims

**1.** A compound represented by Formula 2 below or pharmaceutically acceptable salt thereof:

[Formula 2]

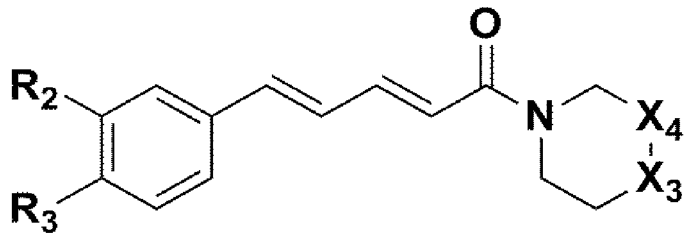

where $R_2$ and $R_3$ are independently selected from H, $-OR_{11}$, and $-SR_{11}$;
each $R_{11}$ is independently unsubstituted or substituted $C_1$-$C_6$ alkyl, wherein the alkyl is linear alkyl, branched alkyl, or cycloalkyl;
$X_3$ and $X_4$ are independently selected from $CR_6R_6'$, O, and $NR_7$, wherein, when $X_3$ is $CH_2$, $X_4$ is not $CH_2$;
$R_6$ and $R_6'$ are independently selected from H, a halogen, $R_{31}$, -OH, $-OR_{31}$, - SH, $-SR_{31}$, -CN, $-N_3$, $-NH_2$, $-NHR_{31}$, $-N(R_{31})_2$, -C(=O)H, $-C(=O)R_{31}$, -C(=O)OH, - $C(=O)OR_{31}$, $-C(=O)NH_2$, $-C(=O)NHR_{31}$, $-C(=O)N(R_{31})_2$, -NHC(=O)H, - NHC(=O)OH, and $-NHC(=O)R_{31}$;
$R_7$ is selected from H, a halogen, $R_{31}$, -OH, $-OR_{31}$, -SH, $-SR_{31}$, -C(=O)H, - $C(=O)R_{31}$, -C(=O)OH, $-C(=O)OR_{31}$, $-C(=O)NH_2$, $-C(=O)NHR_{31}$, and $-C(=O)N(R_{31})_2$; and
each $R_{31}$ is independently unsubstituted or substituted $C_1$-$C_6$ alkyl, wherein the alkyl is linear alkyl, branched alkyl, or cycloalkyl.

**2.** The compound of claim 1 or pharmaceutically acceptable salt thereof,, wherein $R_2$ and $R_3$ are independently selected from H, $-OR_{11}$, and $-SR_{11}$;
each $R_{11}$ isindependently unsubstituted or substituted $C_1$-$C_6$ alkyl, wherein the alkyl is linear alkyl, branched alkyl, or cycloalkyl;
$X_3$ and $X_4$ are $CR_6R_6'$, wherein, when $X_3$ is $CH_2$, $X_4$ is not $CH_2$;
each $R_6$ and $R_6'$ are independently selected from H, a halogen, $R_{31}$, -OH, - $OR_{31}$, -SH, $-SR_{31}$, -CN, $-N_3$, $-NH_2$, $-NHR_{31}$, $-N(R_{31})_2$, -C(=O)H, $-C(=O)R_{31}$, - C(=O)OH, $-C(=O)OR_{31}$, $-C(=O)NH_2$, $-C(=O)NHR_{31}$, $-C(=O)N(R_{31})_2$, -NHC(=O)H, - NHC(=O)OH, and $-NHC(=O)R_{31}$;
both $R_6$ and $R_6'$ are not H at once; and
each $R_{31}$ is independently unsubstituted or substituted $C_1$-$C_6$ alkyl, wherein the alkyl is linear alkyl, branched alkyl, or cycloalkyl.

**3.** The compound of claim 2 or pharmaceutically acceptable salt thereof, wherein $R_6$ is selected from -OH, $-OR_{31}$, $-NH_2$, and $-N_3$, and $R_6'$ is H.

**4.** The compound of claim 1 or pharmaceutically acceptable salt thereof, wherein $R_2$ and $R_3$ are independently selected from H, $-OR_{11}$, and $-SR_{11}$;
each $R_{11}$ is independently unsubstituted or substituted $C_1$-$C_6$ alkyl, wherein the alkyl is linear alkyl, branched alkyl, or cycloalkyl;
$X_3$ and $X_4$ are independently selected from $CH_2$ and $NR_7$, wherein, when $X_3$ is $CH_2$, $X_4$ is not $CH_2$;
$R_7$ is selected from H, a halogen, $R_{31}$, -OH, $-OR_{31}$, -SH, $-SR_{31}$, -C(=O)H, - $C(=O)R_{31}$, -C(=O)OH, $-C(=O)OR_{31}$, $-C(=O)NH_2$, $-C(=O)NHR_{31}$, and $-C(=O)N(R_{31})_2$; and
each $R_{31}$ is independently unsubstituted or substituted $C_1$-$C_6$ alkyl, wherein the alkyl is linear alkyl, branched alkyl, or cycloalkyl.

**5.** The compound of claim 4 or pharmaceutically acceptable salt thereof, wherein $R_7$ is selected from $-C(=O)OR_{31}$ and

$-C(=O)R_{31}$.

**6.** The compound of claim 1 or pharmaceutically acceptable salt thereof, wherein $R_2$ and $R_3$ are independently selected from H, $-OR_{11}$, and $-SR_{11}$;
each $R_{11}$ is independently unsubstituted or substituted $C_1$-$C_6$ alkyl, wherein the alkyl is linear alkyl, branched alkyl, or cycloalkyl;
$X_3$ and $X_4$ are independently selected from $CR_6R_6$' and O, wherein, when $X_3$ is $CH_2$, $X_4$ is not $CH_2$;
each $R_6$ and $R_6$' are independently selected from H, a halogen, $R_{31}$, -OH, - $OR_{31}$, -SH, $-SR_{31}$, -CN, $-N_3$, $-NH_2$, $-NHR_{31}$, $-N(R_{31})_2$, -C(=O)H, $-C(=O)R_{31,}$ - C(=O)OH, $-C(=O)OR_{31}$, $-C(=O)NH_2$, $-C(=O)NHR_{31}$, $-C(=O)N(R_{31})_2$, -NHC(=O)H, - NHC(=O)OH, and $-NHC(=O)R_{31}$; and
each $R_{31}$ is independently unsubstituted or substituted $C_1$-$C_6$ alkyl, wherein the alkyl is linear alkyl, branched alkyl, or cycloalkyl.

**7.** The compound of claim 6 or pharmaceutically acceptable salt thereof, wherein $R_6$ and $R_6$' are H.

**8.** A pharmaceutical composition for preventing or treating at least one selected from fatty liver and fatty liver-related disease, comprising the compound of claim 1 or pharmaceutically acceptable salt thereof as an active ingredient.

**9.** The pharmaceutical composition of claim 8, wherein the fatty liver and the fatty liver-related disease are selected from obesity, steatosis, fatty liver, non-alcoholic fatty liver disease, non-alcoholic steatohepatitis, hepatic fibrosis, cirrhosis, liver cancer, diabetes, dyslipidemia, hyperinsulinemia, insulin resistance, insulin resistance syndrome, and metabolic syndrome.

**10.** The pharmaceutical composition of claim 8, wherein the composition has at least one effect selected from
inhibiting lipogenesis in cells,
inhibiting lipid accumulation in cells,
activating the mechanism of fatty acid β oxidation,
inhibiting a fat biosynthesis mechanism, and
activating a mechanism of regulating lipid metabolism.

Fig. 1

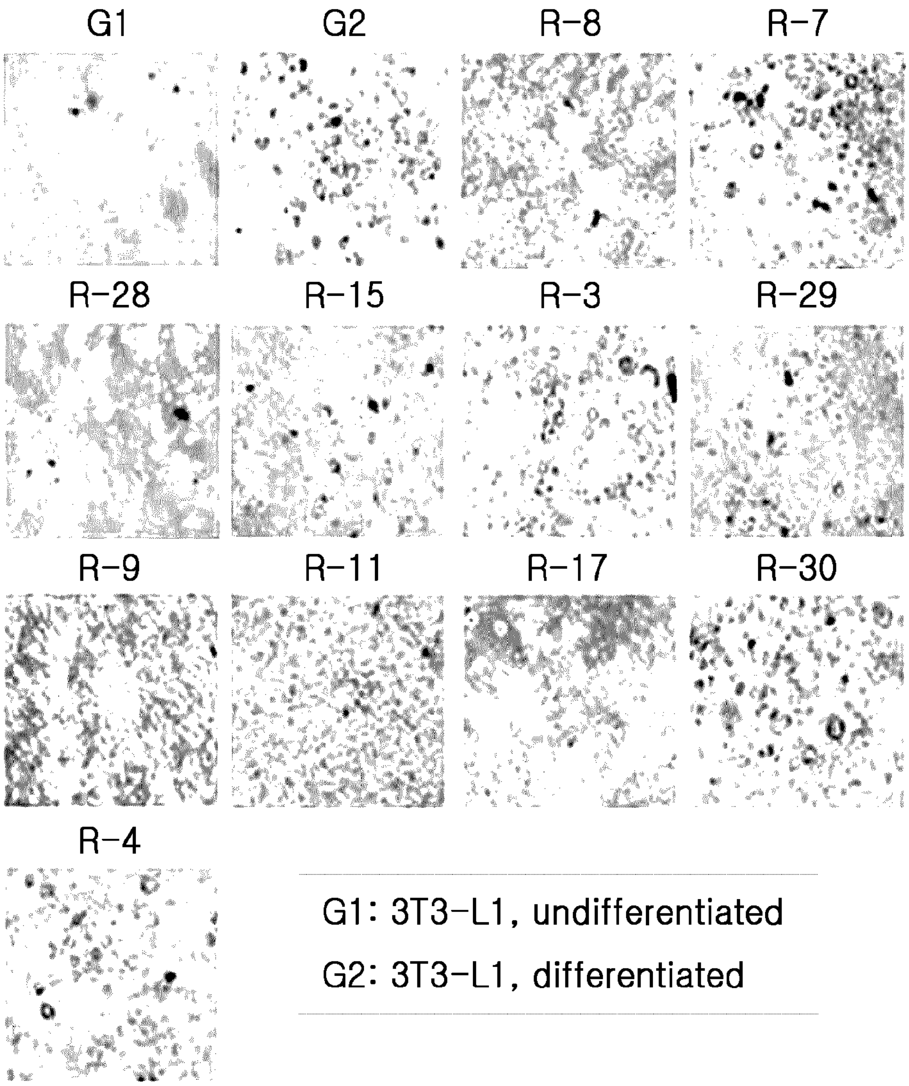
G1
G2
R-8
R-7
R-28
R-15
R-3
R-29
R-9
R-11
R-17
R-30
R-4
G1: 3T3-L1, undifferentiated
G2: 3T3-L1, differentiated

Fig. 2

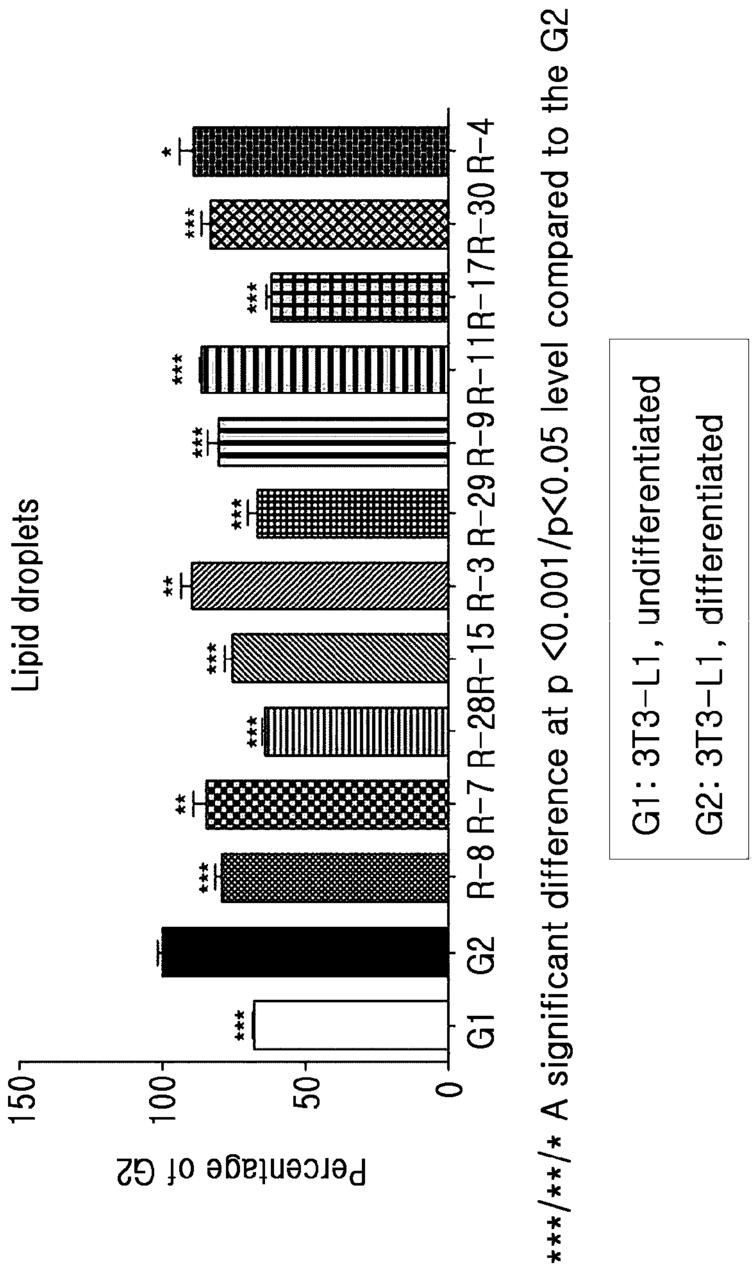
Lipid droplets
Percentage of G2
0
50
100
150
G1
G2
R-8
R-7
R-28
R-15
R-3
R-29
R-9
R-11
R-17
R-30
R-4
***/**/* A significant difference at p <0.001/p<0.05 level compared to the G2
G1 : 3T3-L1, undifferentiated
G2: 3T3-L1, differentiated

Fig. 3

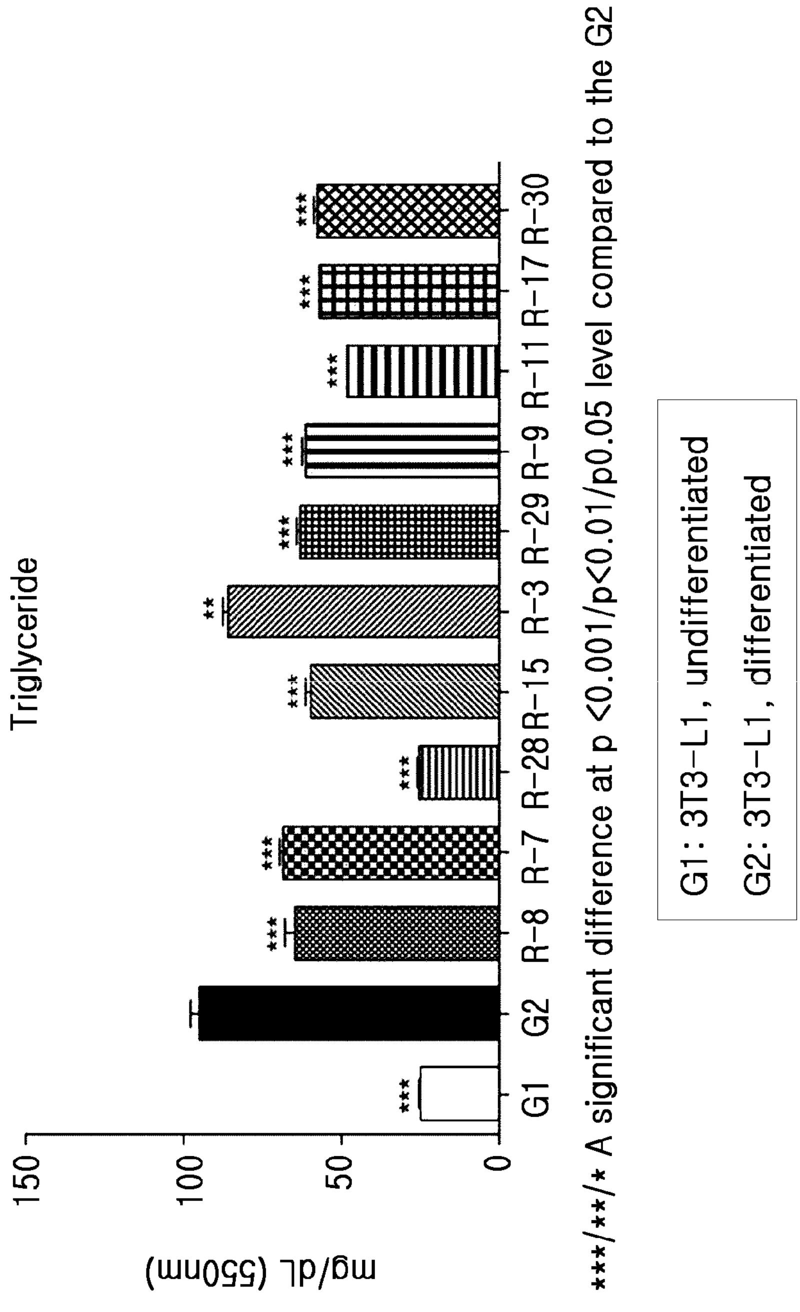
Triglyceride
mg/dL (550nm)
150
100
50
0
G1
G2
R-8
R-7
R-28
R-15
R-3
R-29
R-9
R-11
R-17
R-30
***
**
***/**/* A significant difference at p <0.001/p<0.01/p0.05 level compared to the G2
G1: 3T3-L1, undifferentiated
G2: 3T3-L1, differentiated

Fig. 4

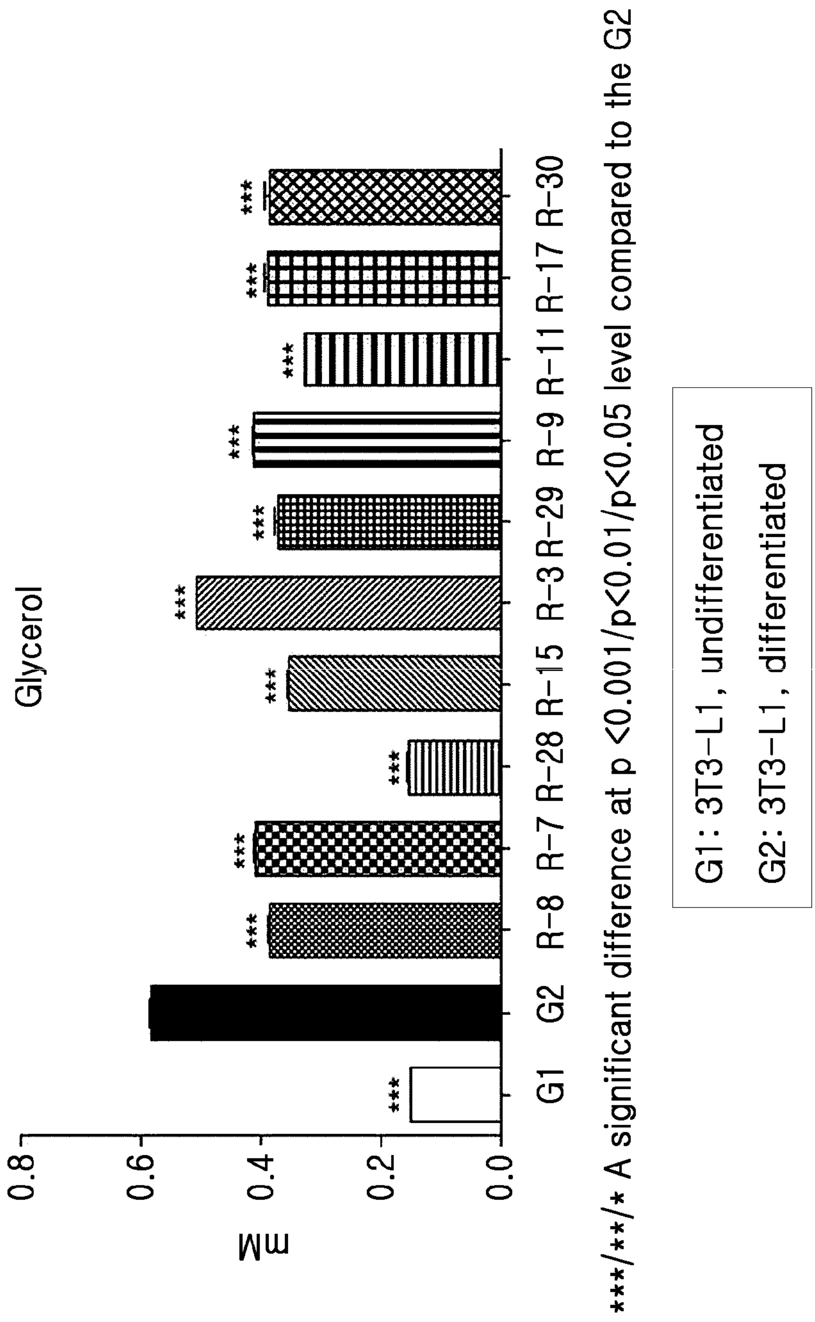
Glycerol
mM
0.0
0.2
0.4
0.6
0.8
***
G1
G2
R-8
R-7
R-28
R-15
R-3
R-29
R-9
R-11
R-17
R-30
***/**/* A significant difference at p <0.001/p<0.01/p<0.05 level compared to the G2
G1: 3T3-L1, undifferentiated
G2: 3T3-L1, differentiated

Fig. 5

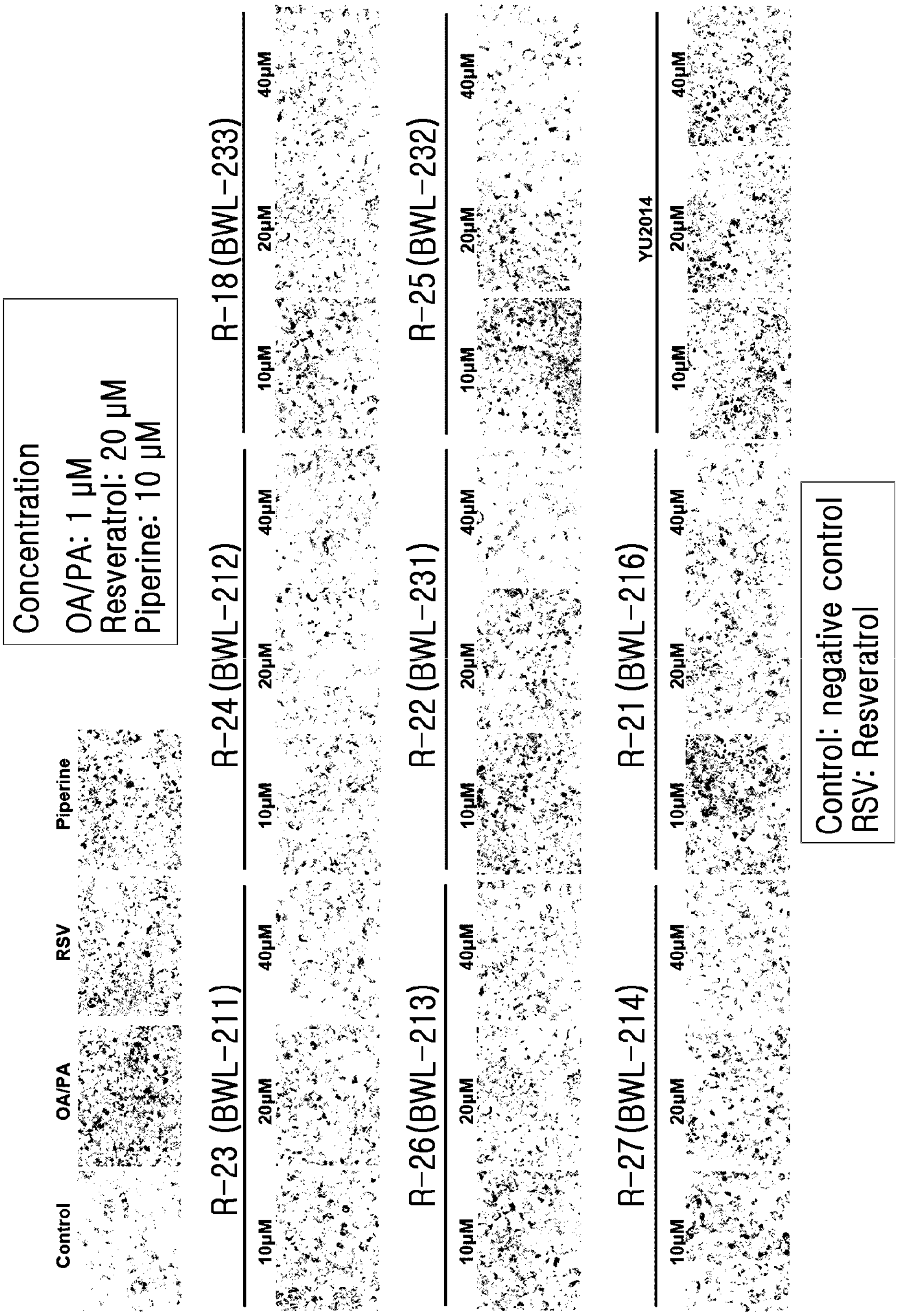
Concentration
OA/PA: 1 μM
Resveratrol: 20 μM
Piperine: 10 μM
Control
OA/PA
RSV
Piperine
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)
YU2014
10μM
20μM
40μM
Control: negative control
RSV: Resveratrol

Fig. 6

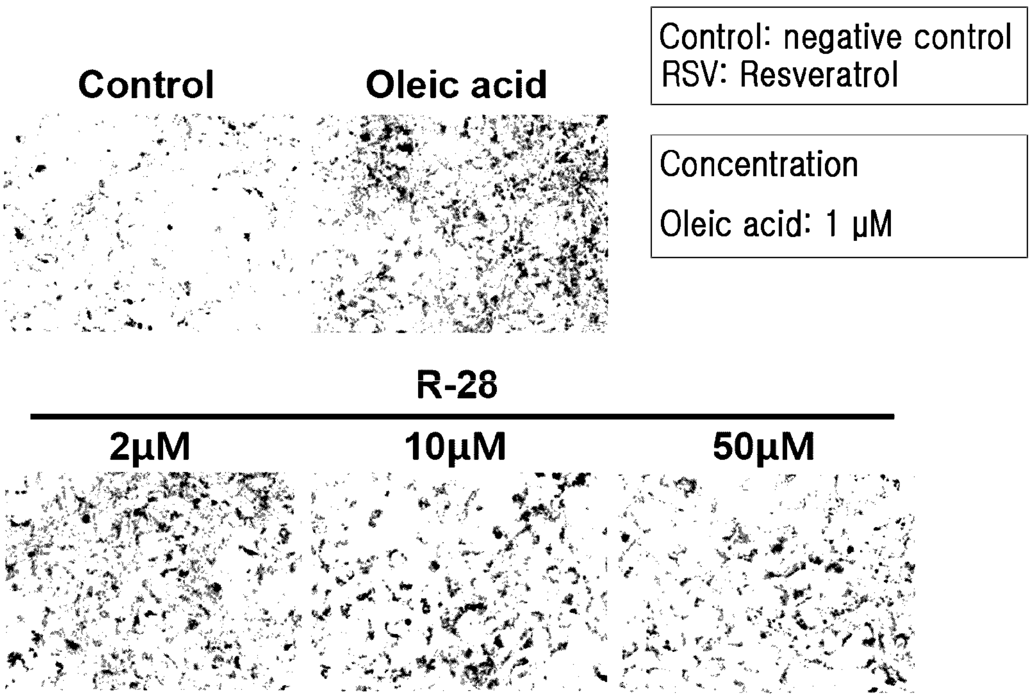
Control
Oleic acid
Control: negative control
RSV: Resveratrol
Concentration
Oleic acid: 1 μM
R-28
2μM
10μM
50μM

Fig. 7

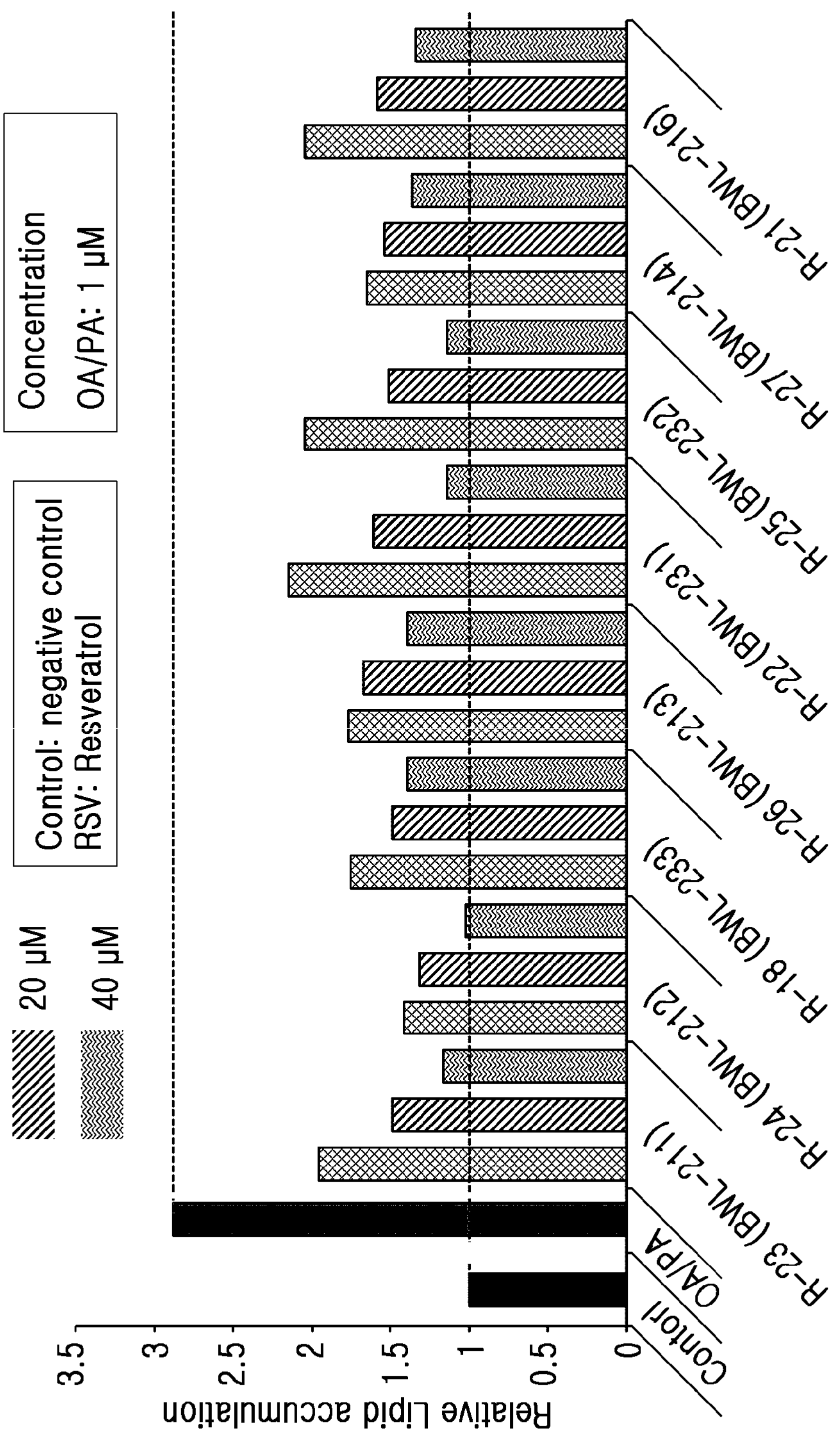
Relative Lipid accumulation
0
0.5
1
1.5
2
2.5
3
3.5
Contorl
OA/PA
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)
10 μM
20 μM
40 μM
Control: negative control
RSV: Resveratrol
Concentration
OA/PA: 1 μM

Fig. 8

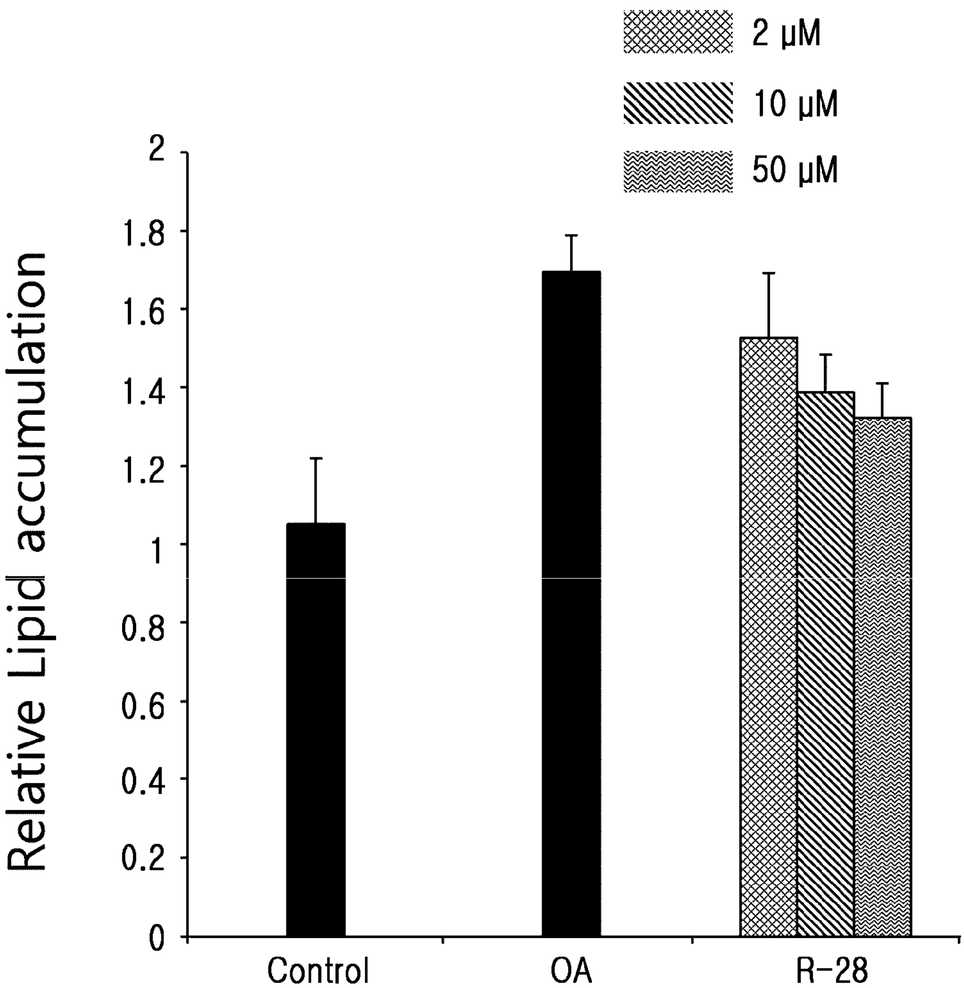
2 μM
10 μM
50 μM
Relative Lipid accumulation
2
1.8
1.6
1.4
1.2
1
0.8
0.6
0.4
0.2
0
Control
OA
R-28

Fig. 9

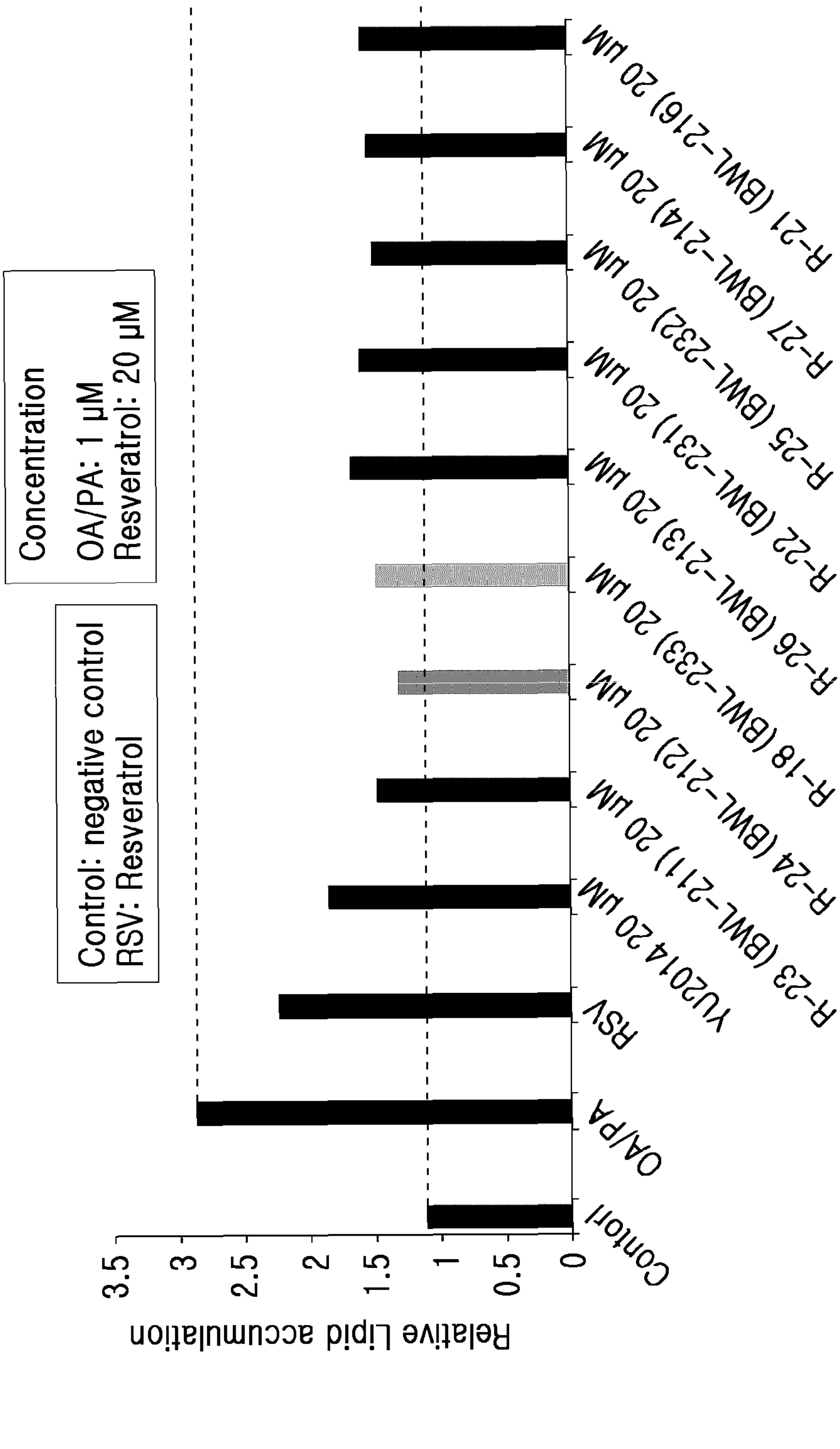
Relative Lipid accumulation
0
0.5
1
1.5
2
2.5
3
3.5
Control: negative control
RSV: Resveratrol
Concentration
OA/PA: 1 µM
Resveratrol: 20 µM
Contorl
OA/PA
RSV
YU2014 20 uM
R-23 (BWL-211) 20 uM
R-24 (BWL-212) 20 uM
R-18 (BWL-233) 20 uM
R-26 (BWL-213) 20 uM
R-22 (BWL-231) 20 uM
R-25 (BWL-232) 20 uM
R-27 (BWL-214) 20 uM
R-21 (BWL-216) 20 uM

Fig. 10

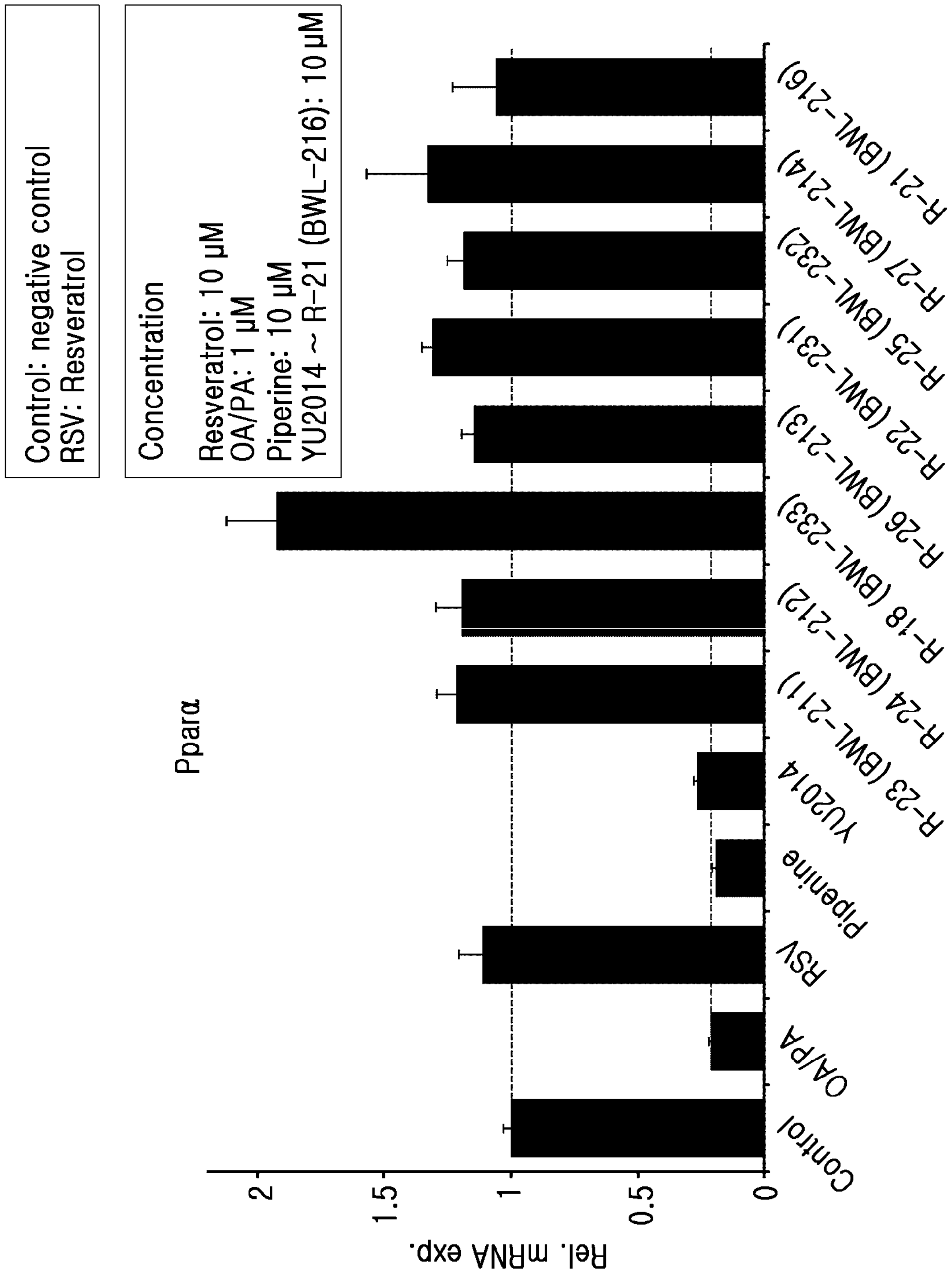
Control: negative control
RSV: Resveratrol
Concentration
Resveratrol: 10 μM
OA/PA: 1 μM
Piperine: 10 μM
YU2014 ~ R-21 (BWL-216): 10 μM
Pparα
Rel. mRNA exp.
0
0.5
1
1.5
2
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

Fig. 11

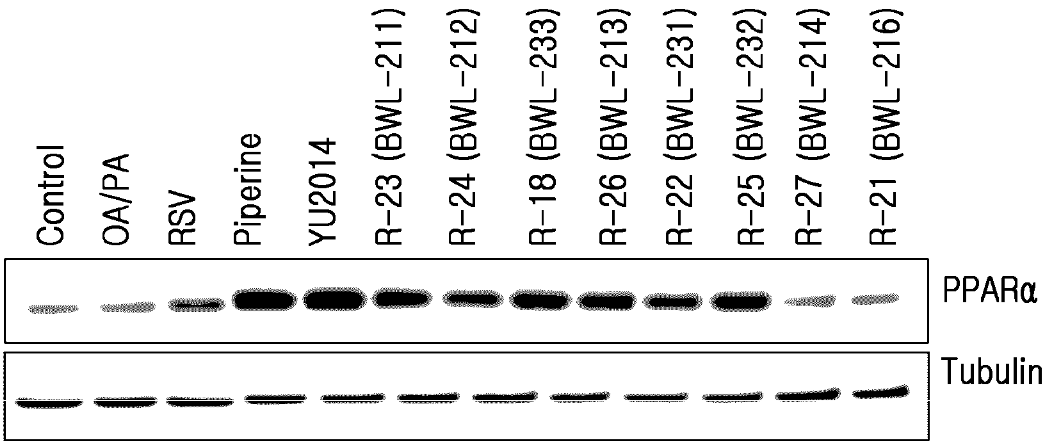


Control: negative control
RSV: Resveratrol

Concentration

RSV: 20 μM
YU2014: 20 μM
Piperine: 10 μM
BWLs: 20 μM

Fig. 12

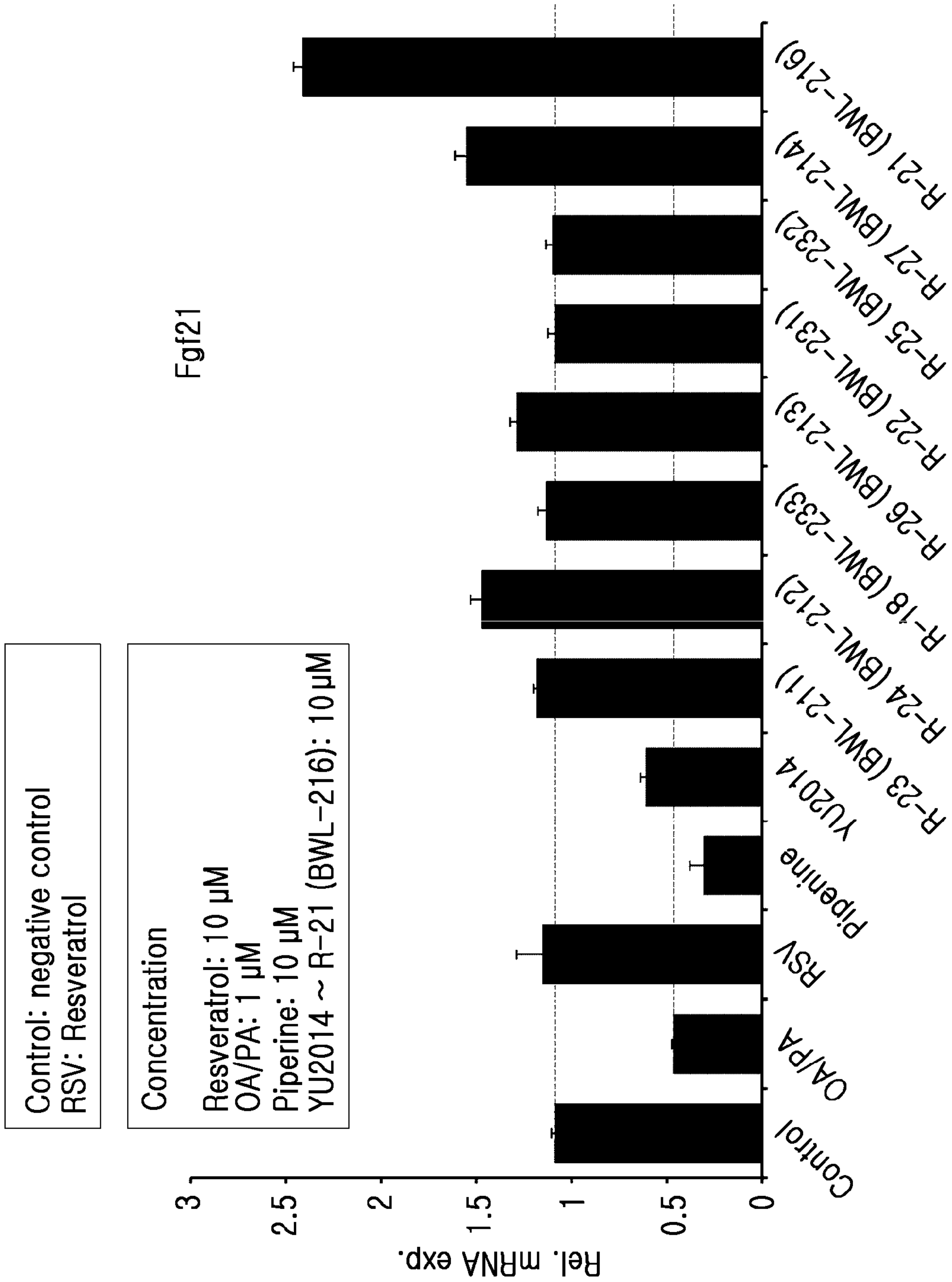
Fgf21
Control: negative control
RSV: Resveratrol
Concentration
Resveratrol: 10 µM
OA/PA: 1 µM
Piperine: 10 µM
YU2014 ~ R-21 (BWL-216): 10µM
Rel. mRNA exp.
0
0.5
1
1.5
2
2.5
3
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

Fig. 13

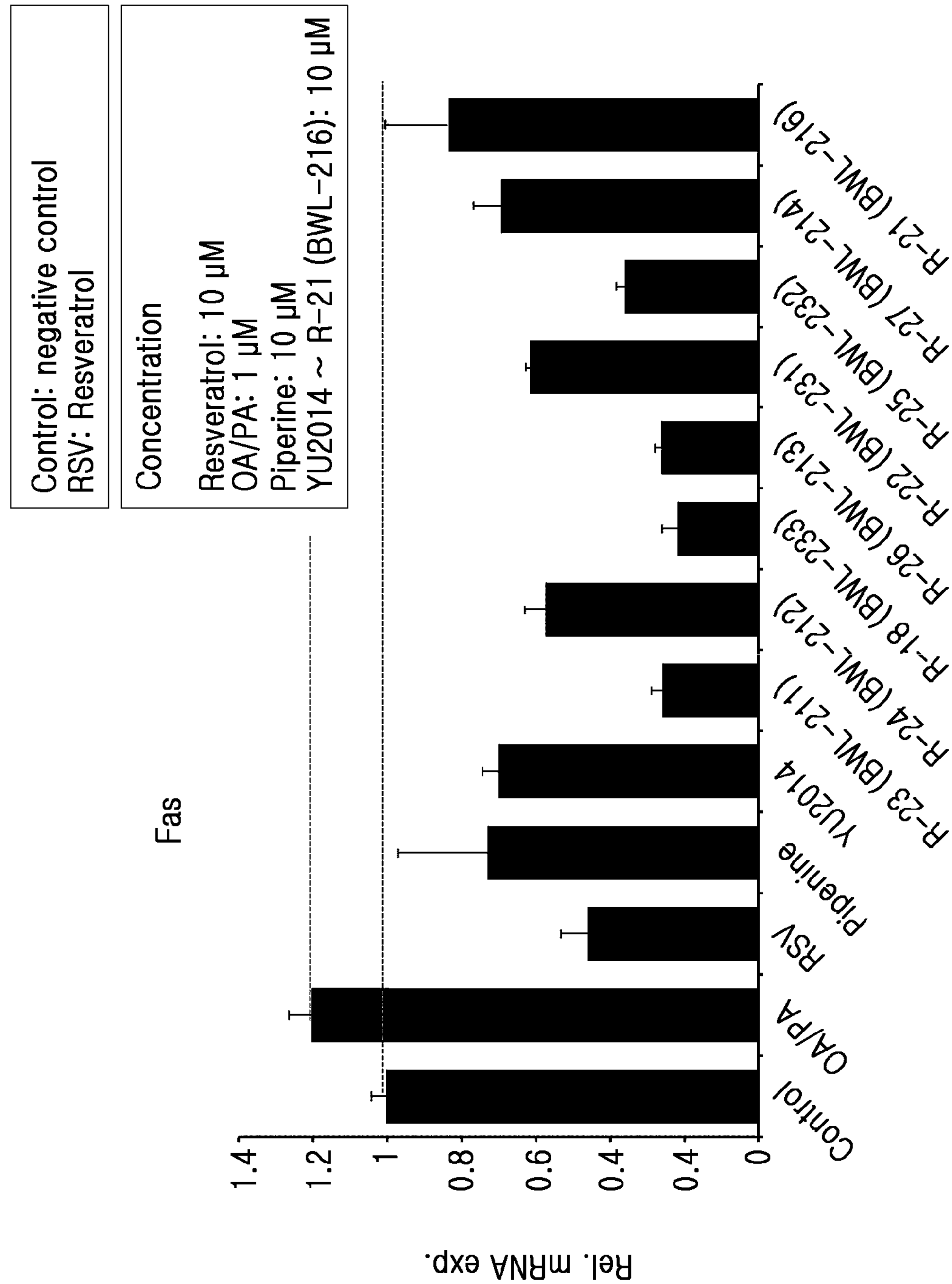
Control: negative control
RSV: Resveratrol
Concentration
Resveratrol: 10 µM
OA/PA: 1 µM
Piperine: 10 µM
YU2014 ~ R-21 (BWL-216): 10 µM
Fas
Rel. mRNA exp.
1.4
1.2
1
0.8
0.6
0.4
0.4
0
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

Fig. 14

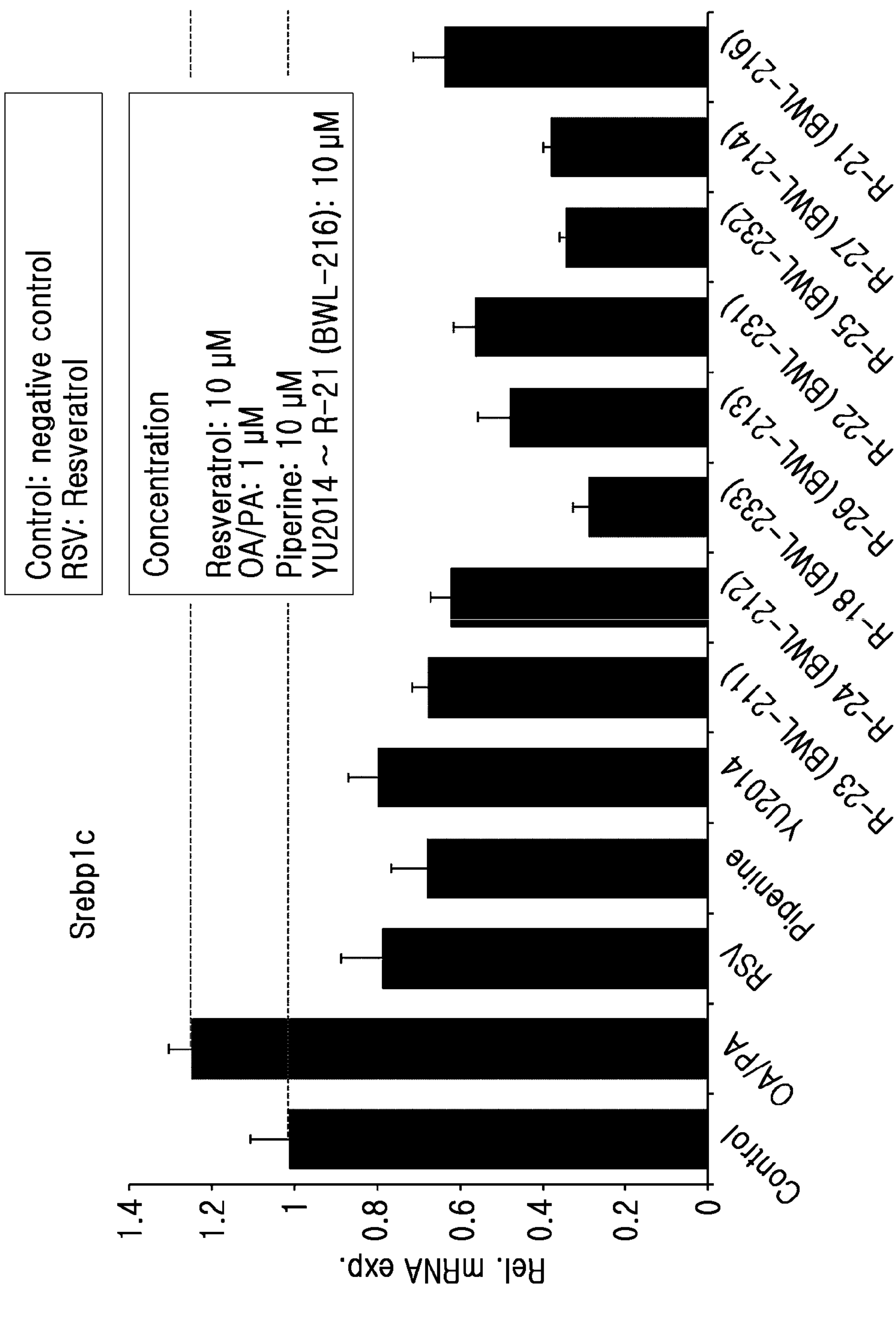
Srebp1c
Control: negative control
RSV: Resveratrol
Concentration
Resveratrol: 10 μM
OA/PA: 1 μM
Piperine: 10 μM
YU2014 ~ R-21 (BWL-216): 10 μM
Rel. mRNA exp.
1.4
1.2
1
0.8
0.6
0.4
0.2
0
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

Fig. 15

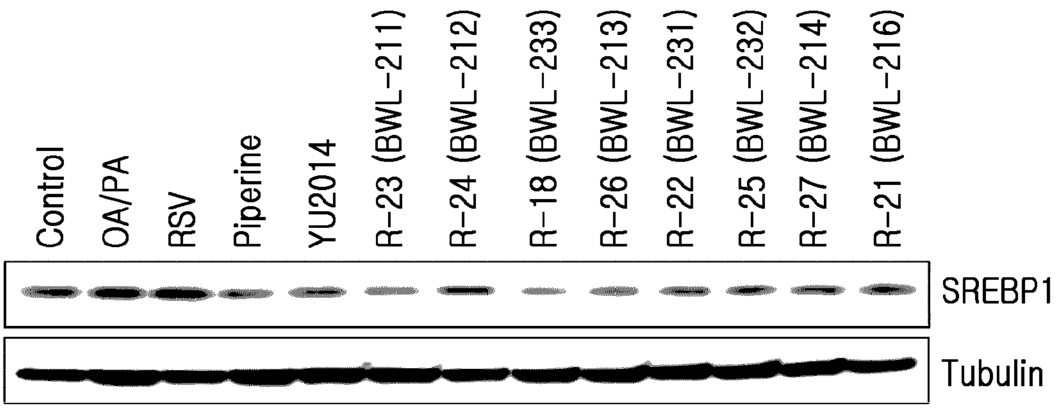


Control: negative control
RSV: Resveratrol

Concentration

Resveratrol: 20 μM
Piperine: 10 μM
YU2014 ~ R-21 (BWL-216): 20 μM

Fig. 16

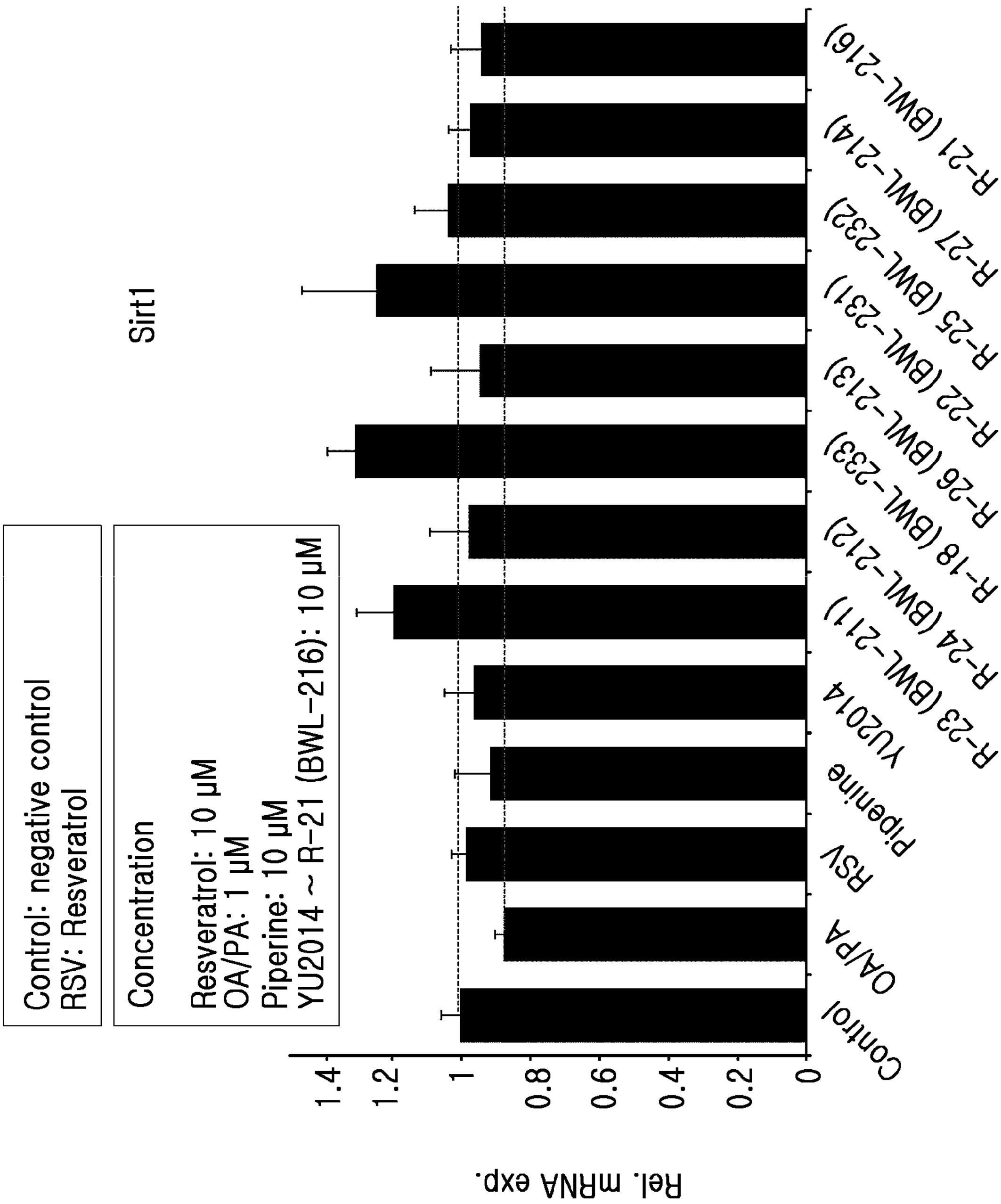
Sirt1
Control: negative control
RSV: Resveratrol
Concentration
Resveratrol: 10 μM
OA/PA: 1 μM
Piperine: 10 μM
YU2014 ~ R-21 (BWL-216): 10 μM
Rel. mRNA exp.
0
0.2
0.4
0.6
0.8
1
1.2
1.4
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

Fig. 17

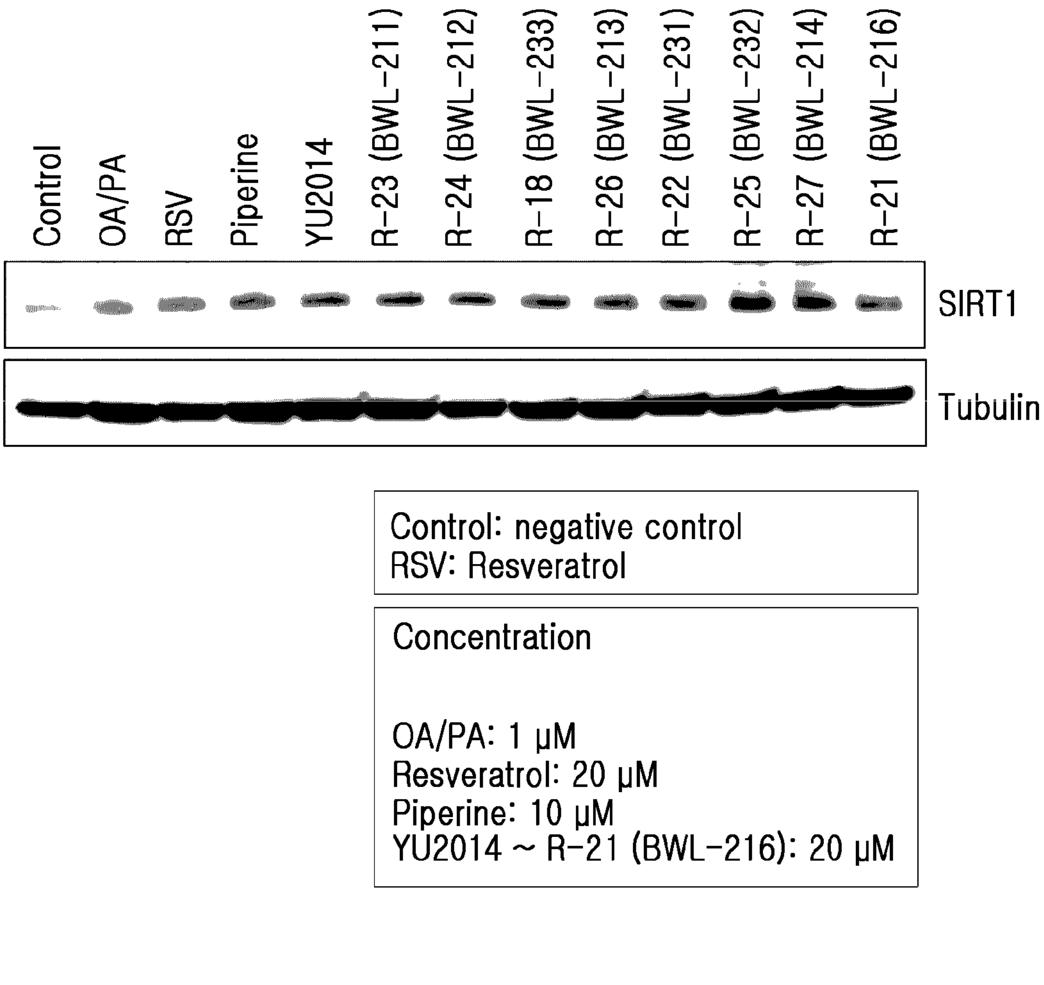
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)
SIRT1
Tubulin
Control: negative control
RSV: Resveratrol
Concentration
OA/PA: 1 μM
Resveratrol: 20 μM
Piperine: 10 μM
YU2014 ~ R-21 (BWL-216): 20 μM

Fig. 18

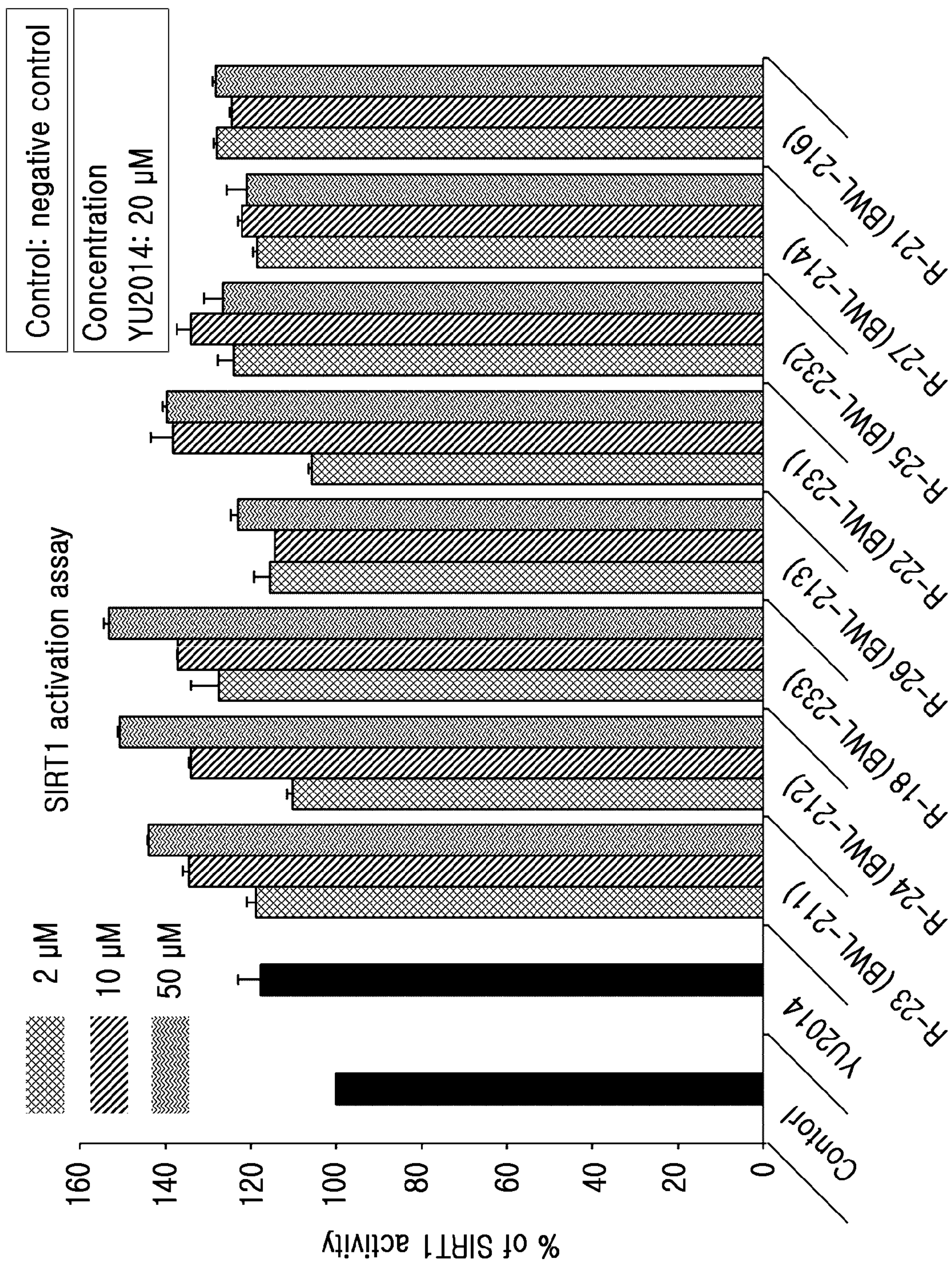
Control: negative control
Concentration
YU2014: 20 μM
SIRT1 activation assay
2 μM
10 μM
50 μM
% of SIRT1 activity
0
20
40
60
80
100
120
140
160
Contorl
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

Fig. 19

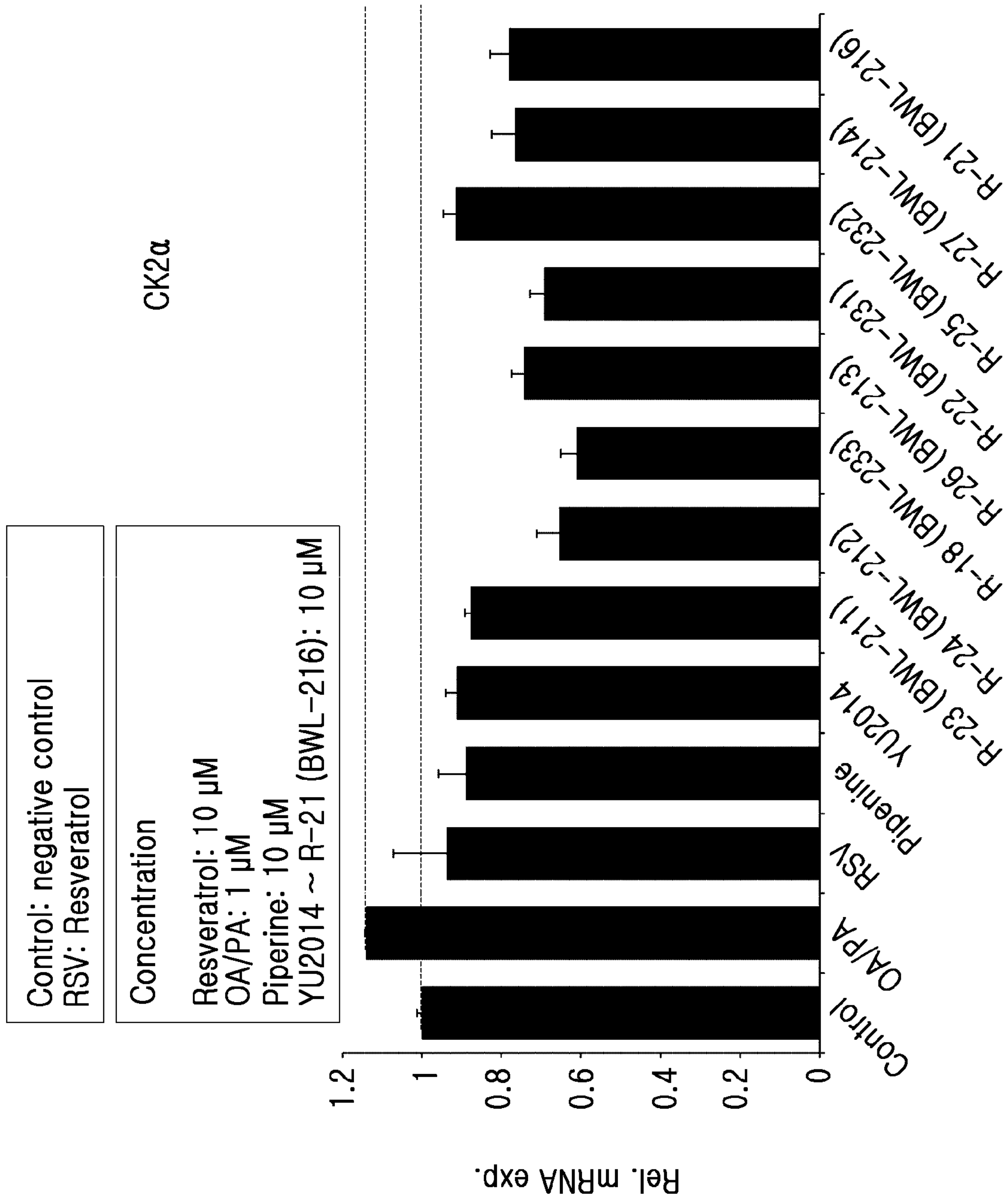
CK2α
Control: negative control
RSV: Resveratrol
Concentration
Resveratrol: 10 μM
OA/PA: 1 μM
Piperine: 10 μM
YU2014 ~ R-21 (BWL-216): 10 μM
Rel. mRNA exp.
0
0.2
0.4
0.6
0.8
1
1.2
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

Fig. 20

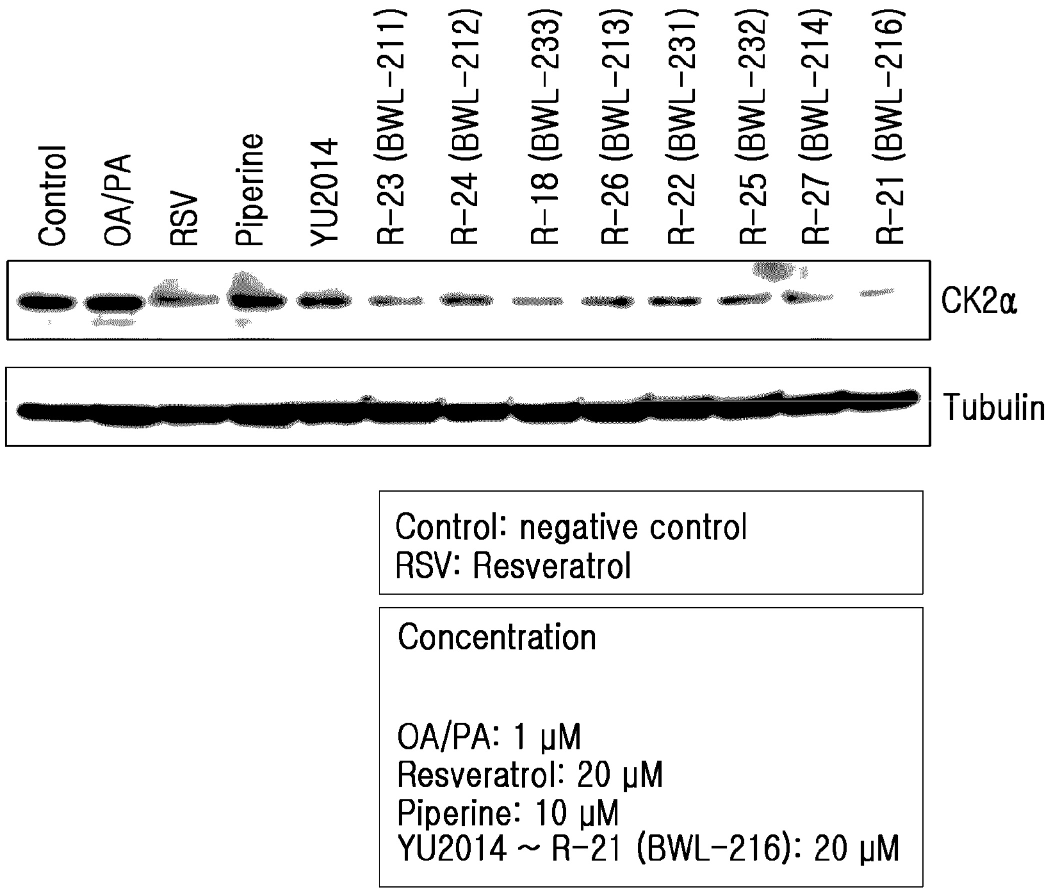
Control
OA/PA
RSV
Piperine
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)
CK2α
Tubulin
Control: negative control
RSV: Resveratrol
Concentration
OA/PA: 1 μM
Resveratrol: 20 μM
Piperine: 10 μM
YU2014 ~ R-21 (BWL-216): 20 μM

Fig. 21

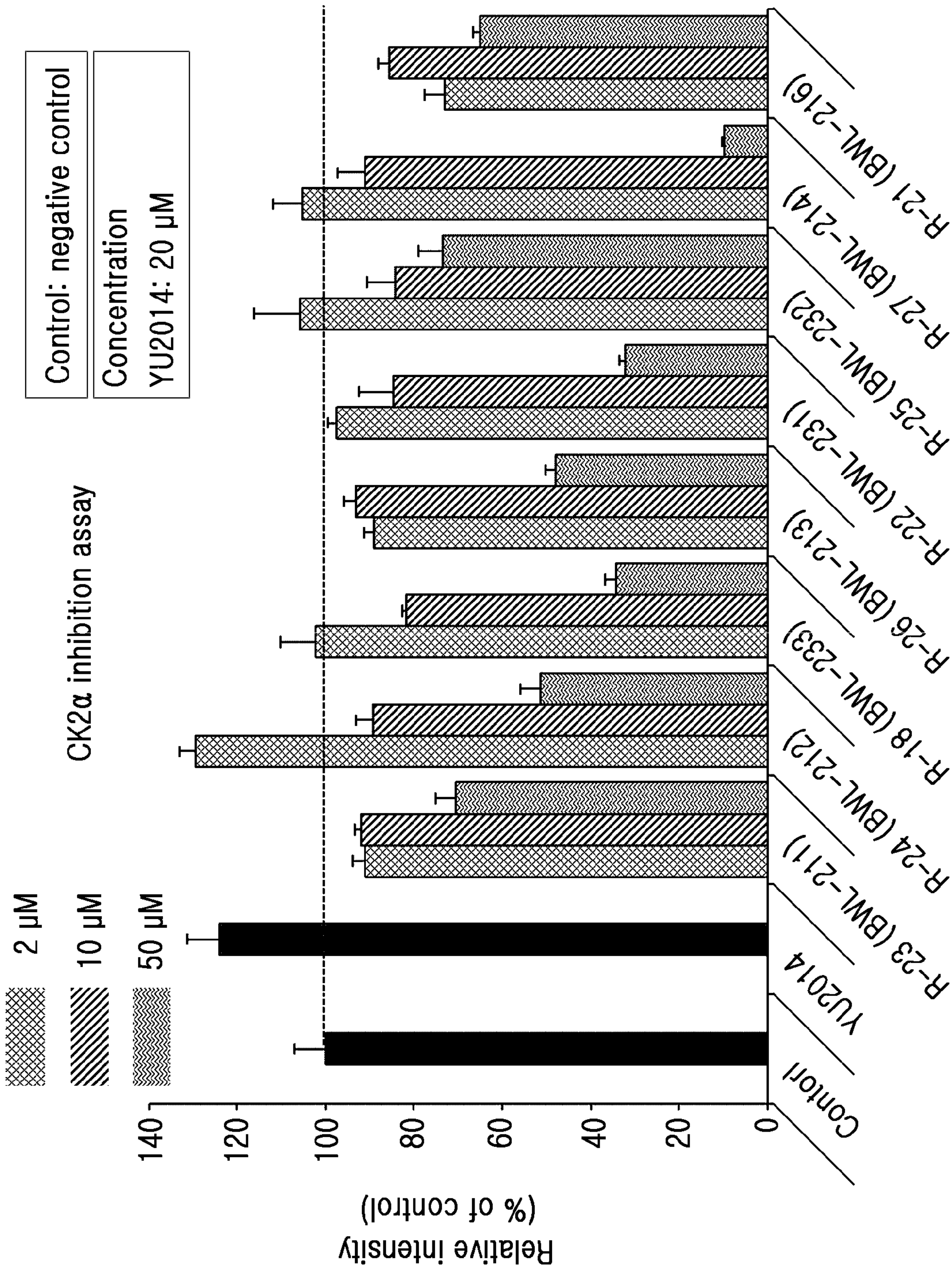
CK2α inhibition assay
Control: negative control
Concentration
YU2014: 20 μM
2 μM
10 μM
50 μM
Relative intensity
(% of control)
0
20
40
60
80
100
120
140
Contorl
YU2014
R-23 (BWL-211)
R-24 (BWL-212)
R-18 (BWL-233)
R-26 (BWL-213)
R-22 (BWL-231)
R-25 (BWL-232)
R-27 (BWL-214)
R-21 (BWL-216)

**INTERNATIONAL SEARCH REPORT**

International application No.
PCT/KR2019/003525

A. CLASSIFICATION OF SUBJECT MATTER

***C07D 211/22(2006.01)i, A61K 31/4453(2006.01)i, A61K 31/496(2006.01)i, A61K 31/5375(2006.01)i, C07D 241/04(2006.01)i, C07D 265/30(2006.01)i, A23L 33/10(2016.01)i, A61P 3/04(2006.01)i, A61P 3/10(2006.01)i***

According to International Patent Classification (IPC) or to both national classification and IPC

B. FIELDS SEARCHED

Minimum documentation searched (classification system followed by classification symbols)

C07D 211/22; A61K 31/385; A61P 7/00; C07D 211/06; C07D 211/32; C07D 241/14; C07D 295/185; C07D 311/58; C07D 317/46; C07D 405/12; C07D 411/06; A61K 31/4453; A61K 31/496; A61K 31/5375; C07D 241/04; C07D 265/30; A23L 33/10; A61P 3/04; A61P 3/10

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & Keywords: phenyl-pentadienone derivative, fatty liver, obesity, diabetes

C. DOCUMENTS CONSIDERED TO BE RELEVANT

| Category* | Citation of document, with indication, where appropriate, of the relevant passages | Relevant to claim No. |
|---|---|---|
| X | Chemical Abstract Compound. STN express. RN 1181623-88-0 (Entered STN: 03 September 2009) See the chemical formulas. | 1-3 |
| A | | 4-10 |
| X | Chemical Abstract Compound. STN express. RN 958823-18-2 (Entered STN: 19 December 2007) See the chemical formulas. | 1,2 |
| X | KOUL, S. et al. Structure-activity relationship of piperine and its synthetic analogues for their inhibitory potentials of rat hepatic microsomal constitutive and inducible cytochrome P450 activities. Bioorganic & Medicinal Chemistry. 2000, vol. 8, pages 251-268 See abstract; table 1. | 1,4 |
| X | WO 03-070713 A1 (COUNCIL OF SCIENTIFIC AND INDUSTRIAL RESEARCH) 28 August 2003 See claim 2. | 1,6,7 |
| X | KR 10-1498218 B1 (INDUSTRY-ACADEMIC COOPERATION FOUNDATION, YONSEI UNIVERSITY et al.) 12 March 2015 See abstract; paragraphs [0098]-[0106]; claims 1, 3. | 1-10 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 JULY 2019 (15.07.2019) | **15 JULY 2019 (15.07.2019)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

INTERNATIONAL SEARCH REPORT

International application No.
PCT/KR2019/003525

C (Continuation). DOCUMENTS CONSIDERED TO BE RELEVANT

| Category* | Citation of document, with indication, where appropriate, of the relevant passages | Relevant to claim No. |
|---|---|---|
| X | KR 10-1186500 B1 (INDUSTRY-ACADEMIC COOPERATION FOUNDATION, YONSEI UNIVERSITY) 27 September 2012<br>See claims 1, 8, 11-15. | 1-10 |
| X | KR 10-2009-0031735 A (PIERRE FABRE MEDICAMENT) 27 March 2009<br>See claims 1, 15; examples 10-15. | 1-10 |
| A | LU, Y. et al. Cytochrome P450 2E1 contributes to ethanol-induced fatty liver in mice. Hepatology. 2008, vol. 47, no. 5, pages 1483-1494<br>See the entire document. | 1-10 |
| PX | KR 10-2018-0053032 A (BIOWAY., INC.) 21 May 2018<br>See abstract; claim 1. | 1-10 |

Form PCT/ISA/210 (continuation of second sheet) (January 2015)

**INTERNATIONAL SEARCH REPORT**
Information on patent family members

International application No.
**PCT/KR2019/003525**

| Patent document cited in search report | Publication date | Patent family member | Publication date |
|---|---|---|---|
| WO 03-070713 A1 | 28/08/2003 | EP 1478632 A1 | 24/11/2004 |
| | | JP 2005-517737 A | 16/06/2005 |
| | | JP 4332431 B2 | 16/09/2009 |
| | | US 2003-0161860 A1 | 28/08/2003 |
| | | US 2006-0094874 A1 | 04/05/2006 |
| | | US 7057040 B2 | 06/06/2006 |
| | | US 7262296 B2 | 28/08/2007 |
| KR 10-1498218 B1 | 12/03/2015 | US 10100011 B2 | 16/10/2018 |
| | | US 2017-0320825 A1 | 09/11/2017 |
| | | WO 2016-048005 A2 | 31/03/2016 |
| | | WO 2016-048005 A3 | 18/08/2016 |
| KR 10-1186500 B1 | 27/09/2012 | EP 2810941 A1 | 10/12/2014 |
| | | EP 2810941 B1 | 15/03/2017 |
| | | US 2014-0371271 A1 | 18/12/2014 |
| | | US 9321751 B2 | 26/04/2016 |
| | | WO 2013-115486 A1 | 08/08/2013 |
| KR 10-2009-0031735 A | 27/03/2009 | CN 101472894 A | 01/07/2009 |
| | | CN 101472894 B | 05/09/2012 |
| | | EP 2046748 A1 | 15/04/2009 |
| | | EP 2046748 B1 | 02/03/2011 |
| | | JP 2009-541258 A | 26/11/2009 |
| | | JP 5268897 B2 | 21/08/2013 |
| | | US 2010-0003260 A1 | 07/01/2010 |
| | | US 8022064 B2 | 20/09/2011 |
| | | WO 2007-147822 A1 | 27/12/2007 |
| KR 10-2018-0053032 A | 21/05/2018 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1078376
- KR 1186500
- KR 1498218


### Non-patent literature cited in the description


- **DING RB ; BAO J ; DENG CX.** *Int J Biol Sci.,* 2017, vol. 13 (7), 852-867
- **PURUSHOTHAM A et al.** *Cell Metab.,* 2009, vol. 9 (4), 327-38
- **CHOI SE ; KWON S ; SEOK S et al.** *Mol Cell Biol.,* 2017, vol. 37 (15), e00006-17
- **VISCARRA JA ; WANG Y ; HONG IH ; SUL HS.** *Sci Signal,* 2017, vol. 10 (467), eaai8596
- **CHOI SE ; KWON S ; SEOK S et al.** *Mol Cell Biol.,* 2017
- Remington's Pharmaceutical Science. Mack Publishing Company